(19) Europäisches Patentamt
European Patent Office
Office européen des brevets

(11) EP 1 608 620 B1

(12) **EUROPEAN PATENT SPECIFICATION**

(45) Date of publication and mention of the grant of the patent:
**28.12.2011 Bulletin 2011/52**

(21) Application number: **04711379.0**

(22) Date of filing: **16.02.2004**

(51) Int Cl.:
*C07D 211/94* (2006.01) *C07D 401/14* (2006.01)
*C08K 5/3435* (2006.01) *C08K 5/3492* (2006.01)

(86) International application number:
**PCT/EP2004/050133**

(87) International publication number:
**WO 2004/076419 (10.09.2004 Gazette 2004/37)**

(54) **WATER COMPATIBLE STERICALLY HINDERED HYDROXY SUBSTITUTED ALKOXYAMINES**

WASSERVERTRÄGLICHE STERISCH GEHINDERTE HYDROXYSUBSTITUTIERTE ALKOXYAMINE

ALCOXYAMINES HYDROXY SUBSTITUEES STERIQUEMENT ENCOMBREES HYDROCOMPATIBLES

(84) Designated Contracting States:
**AT BE BG CH CY CZ DE DK EE ES FI FR GB GR HU IE IT LI LU MC NL PT RO SE SI SK TR**

(30) Priority: **26.02.2003 US 450262 P**

(43) Date of publication of application:
**28.12.2005 Bulletin 2005/52**

(73) Proprietor: **BASF SE**
**67056 Ludwigshafen (DE)**

(72) Inventors:
- **WOOD, Mervin Gale**
  **Maysville, KY 41056 (US)**
- **DETLEFSEN, Robert Edward**
  **Putnam Valley, New York 10579 (US)**
- **GALBO, James Peter**
  **Wingdale, New York 12594 (US)**
- **MARTIN, De Wanda H.**
  **Fruitdale, AL 36539 (US)**
- **KONDRACKI, Paul,**
  **Penthouse B/304**
  **Miramar,**
  **Panaji Goa 403 001 (IN)**
- **DIFAZIO, Michael Peter**
  **Mobile, Alabama 36695 (US)**
- **BABIARZ, Joseph Edmund**
  **Amawalk, New York 10501 (US)**

(56) References cited:

| | |
|---|---|
| **EP-A- 0 222 093** | **EP-A- 0 309 402** |
| **EP-A- 0 389 419** | **EP-A- 0 389 428** |
| **EP-A- 0 413 665** | **EP-A- 0 466 647** |
| **EP-A- 0 882 600** | **WO-A-02/25007** |
| **WO-A-02/055618** | **WO-A-02/100831** |
| **WO-A-2004/000809** | **DE-A- 10 008 367** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 608 620 B1

## Description

The instant invention pertains to sterically hindered hydroxy substituted alkoxyamine stabilizer compounds which are water compatible via certain backbones with affinity towards water. These materials are particularly effective in stabilizing aqueous polymer systems against the deleterious effects of oxidative, thermal and actinic radiation. The compounds are effective for example in stabilizing water borne coatings, aqueous inks, aqueous ink jet media and photocured aqueous systems.

U.S. Pat. Nos. 5,004,770 and 5,096,950 describe hindered amine compounds which are substituted on the N-atom by alkoxy moieties.

U.S. Pat. Nos. 6,271,377, 6,392,041 and 6,376,584 disclose sterically hindered hydroxy substituted alkoxyamines.

U.S. Pat. No. 6,254,724 teaches hindered hydroxylamine salt compounds.

U.S. Pat. No. 6,465,645 discloses long chain hindered amine stabilizers.

U.S. Pat. Nos. 5,286,865 and 5,216,156 disclose non-migrating hindered amine stabilizers.

U.S. published app. No. 2002/0050226 and equivalent EP 1174476 disclose certain hindered amines with certain water soluble groups.

U.S. Pat. No. 6,102,997 discloses certain hindered amine compounds with water solubilizing groups.

JP2000044851 teaches an ink composition that contains certain hindered amine compounds.

JP99170686 teaches hindered amine type additives generically in the ink receiving layer of ink jet recording media.

The present compounds are sterically hindered hydroxy substituted alkoxyamines, which are made water compatible or water soluble with a water compatible or water soluble backbone.

The instant compounds perform extremely well in aqueous polymeric systems and polar high solids systems. The present compounds exhibit excellent compatibility in polar environments such as polyurethane based coating systems, water borne automotive coating systems, polar recording media and aqueous inks.

## Detailed Disclosure

The present water compatible or water soluble sterically hindered hydroxy substituted alkoxyamines are of the formula (1) to (5)

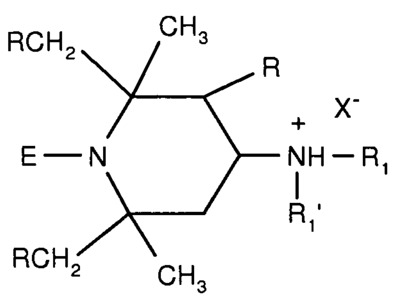

(1)

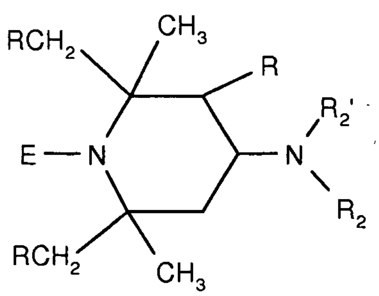

(2)

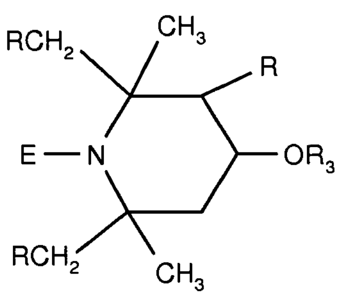

(3)

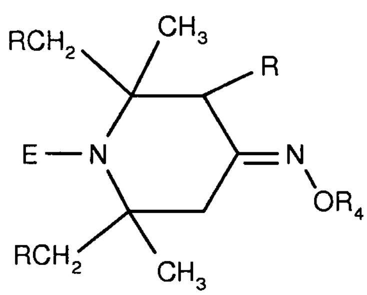

(4)

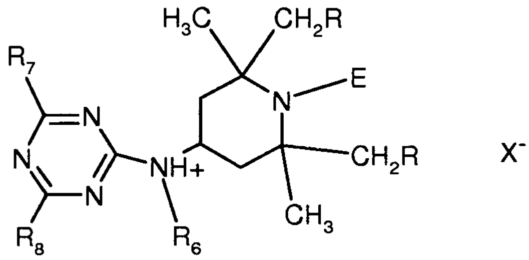

(5)

where
E is $-O-T-(OH)_b$,
T is a straight or branched chain alkylene of 1 to 18 carbon atoms, cycloalkylene of 5 to 18 carbon atoms, cycloalkenylene of 5 to 18 carbon atoms, a straight or branched chain alkylene of 1 to 4 carbon atoms substituted by phenyl or by phenyl substituted by one or two alkyl groups of 1 to 4 carbon atoms;
b is 1, 2 or 3 with the proviso that b cannot exceed the number of carbon atoms in T, and when b is 2 or 3, each hydroxyl group is attached to a different carbon atoms of T;
E' is hydrogen, $C_1$-$C_{18}$alkyl, $C_2$-$C_{18}$alkenyl, $C_7$-$C_{15}$phenylalkyl, $C_2$-$C_{18}$alkanoyl or phenyl, or E' is independently defined as for E,
R is hydrogen or methyl,
$R_1$ is hydrogen, $C_1$-$C_{12}$alkyl, $C_5$-$C_8$cycloalkyl, $C_5$-$C_8$cycloalkyl substituted by one to three $C_1$-$C_4$alkyl, $C_2$-$C_{12}$alkenyl, phenyl, $C_7$-$C_9$phenylalkyl, glycidyl, $C_2$-$C_{12}$alkanoyl, $C_6$-$C_9$cycloalkylcarbonyl, $C_2$-$C_{12}$carbamoyl, $C_2$-$C_{12}$alkenoyl, benzoyl, benzoyl substituted by one to three $C_1$-$C_4$alkyl, $C_2$-$C_{12}$alkanoyl substituted by a di($C_1$-$C_6$alkyl) phosphonate,
or $R_1$ is $C_2$-$C_{12}$alkyl, $C_2$-$C_{12}$alkanoyl or $C_7$-$C_{18}$phenylalkyl, each interrupted by one to six oxygen, sulfur or $-N(R_6)$- groups; $C_1$-$C_{12}$alkyl, $C_2$-$C_{12}$alkanoyl, phenyl or $C_7$-$C_{18}$phenylalkyl, each substituted by one to six hydroxy groups or by one to six $-NHR_6$ groups; $C_2$-$C_{12}$alkyl, $C_2$-$C_{12}$alkanoyl or $C_7$-$C_{18}$phenylalkyl, each interrupted by one to three $-NR_6C(O)$- groups; or $C_1$-$C_{12}$alkyl, $C_2$-$C_{12}$alkanoyl, phenyl or $C_7$-$C_{18}$phenylalkyl, each substituted by one to three $-SO_3H$ groups or by one to three $-COOR_6$ groups; or
$R_1$ is said alkyl substituted by a piperazine or by a morpholine group; or
$R_1$ is said interrupted group further substituted by one to six hydroxy groups or by one to six - $NHR_6$ groups; or
$R_1$ is said interrupted group further substituted by one to three $-SO_3H$ groups or by one to three $-COOR_6$ groups;
or $R_1$ is a mono-valent homo- or co-oligomer consisting of monomer units derived from monomers selected from the group consisting of ethylene oxide, propylene oxide, ethylene glycol, propylene glycol, acrylic acid, methacrylic acid, ethylene imine, acrylamide, vinyl formamide, vinyl alcohol and vinyl acetate; which homo- or co-oligomer consists of between 2 and 24 monomer units;
$R_1'$ is independently defined as for $R_1$,
$R_6$ is hydrogen or $C_1$-$C_6$alkyl,
$R_6'$, $R_6''$ are independently defined as for $R_6$,
$R_7$ is $-N(R_2)(R_2')$ or is chlorine, alkoxy of 1 to 12 carbon atoms, 2-hydroxyethylamino or $-N(R_6)(R_6')$;
or $R_7$ is

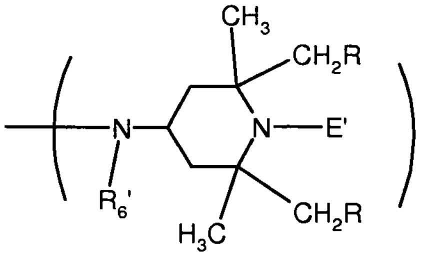

or

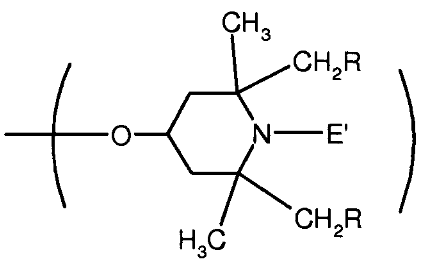

;

$R_8$ is defined as for $R_7$, where one of $R_7$ and $R_8$ is $-N(R_2)(R_2')$;
$X^-$ is an inorganic or organic anion,

$Y^+$ is a mono-, di- or tri-valent cation,
$R_2$ is $C_2$-$C_{12}$alkyl, $C_2$-$C_{12}$alkanoyl or $C_7$-$C_{18}$phenylalkyl, each interrupted by one to six oxygen, sulfur or -N($R_6$)- groups further substituted by 1 to 3 -$COO^-Y^+$ groups; or
$R_2$ is $C_1$-$C_{12}$alkyl, $C_2$-$C_{12}$alkanoyl, phenyl or $C_7$-$C_{18}$phenylalkyl, each substituted by one or two -$COO^-Y^+$, -N($R_6$)($R_6$')($R_6$")$^+X^-$ or -$SO_3^-Y^+$ groups; or
$R_2$ is said $C_1$-$C_{12}$alkyl, $C_2$-$C_{12}$alkanoyl, phenyl or $C_7$-$C_{18}$phenylalkyl, each of which is substituted by one or two -$COO^-Y^+$, -N($R_6$)($R_6$')($R_6$")$^+X^-$ or -$SO_3^-Y^+$ groups, each further substituted by one or two -OH, -$COOR_6$ or -$NHR_6$ groups; or
$R_2$' is $C_2$-$C_{12}$alkyl, $C_2$-$C_{12}$alkanoyl or $C_7$-$C_{18}$phenylalkyl, each interrupted by one to six oxygen, sulfur or -N($R_6$)- groups; $C_1$-$C_{12}$alkyl, $C_2$-$C_{12}$alkanoyl, phenyl or $C_7$-$C_{18}$phenylalkyl, each substituted by one to six hydroxy groups or by one to six -$NHR_6$ groups; $C_2$-$C_{12}$alkyl, $C_2$-$C_{12}$alkanoyl or $C_7$-$C_{18}$phenylalkyl, each interrupted by one to three -$NR_6$C(O)- groups; or $R_2$ is $C_1$-$C_{12}$alkyl, $C_2$-$C_{12}$alkanoyl, phenyl or $C_7$-$C_{18}$phenylalkyl, each substituted by one to three - $SO_3$H groups or by one to three -$COOR_6$ groups; or
$R_2$' is alkyl substituted by a piperazine or by a morpholine group; or
$R_2$' is one of said interrupted groups further substituted by one to six hydroxy groups or by one to six -$NHR_6$ groups; or
$R_2$' is one of said interrupted groups further substituted by one to three -$SO_3$H groups or by one to three -$COOR_6$ groups; or
$R_2$' is $C_2$-$C_{12}$alkyl, $C_2$-$C_{12}$alkanoyl or $C_7$-$C_{18}$phenylalkyl, each interrupted by one to six oxygen, sulfur or -N($R_6$)- groups further substituted by 1 to 3 -$COO^-Y^+$ groups; or
$R_2$' is $C_1$-$C_{12}$alkyl, $C_2$-$C_{12}$alkanoyl, phenyl or $C_7$-$C_{18}$phenylalkyl, each substituted by one or two -$COO^-Y^+$, -N($R_6$)($R_6$')($R_6$")$^+X^-$ or -$SO_3^-Y^+$ groups; or
$R_2$' is $C_1$-$C_{12}$alkyl, $C_2$-$C_{12}$alkanoyl, phenyl or $C_7$-$C_{18}$phenylalkyl, each of which is substituted by one or two -$COO^-Y^+$, -N($R_6$)($R_6$')($R_6$")$^+X^-$ or -$SO_3^-Y^+$ groups, each further substituted by one or two -OH, -$COOR_6$ or -$NHR_6$ groups; or
$R_2$' is a mono-valent homo- or co-oligomer consisting of monomer units derived from monomers selected from the group consisting of ethylene oxide, propylene oxide, ethylene glycol, propylene glycol, acrylic acid, methacrylic acid, ethylene imine, acrylamide, vinyl formamide, vinyl alcohol and vinyl acetate; which homo- or co-oligomer consists of between 2 and 24 monomer units, or
$R_2$' is hydrogen,
$R_3$ is $C_2$-$C_{12}$alkyl, $C_2$-$C_{12}$alkanoyl or $C_7$-$C_{18}$phenylalkyl, each interrupted by one to six oxygen, sulfur or -N($R_6$)- groups further substituted by 1 to 3 -$COO^-Y^+$ groups; or
$R_3$ is $C_1$-$C_{12}$alkyl, $C_2$-$C_{12}$alkanoyl, phenyl or $C_7$-$C_{18}$phenylalkyl, each substituted by one or two -$COO^-Y^+$, -N($R_6$)($R_6$')($R_6$")$^+X^-$ or -$SO_3^-Y^+$ groups; or
$R_3$ is $C_1$-$C_{12}$alkyl, $C_2$-$C_{12}$alkanoyl, phenyl or $C_7$-$C_{18}$phenylalkyl, each of which is substituted by one or two -$COO^-Y^+$, -N($R_6$)($R_6$')($R_6$")$^+X^-$ or -$SO_3^-Y^+$ groups, each further substituted by one or two -OH, -$COOR_6$ or -$NHR_6$ groups; or
$R_3$ is -$SO_3^-Y^+$ or -$PO_3H^-Y^+$, and
$R_4$ is $C_2$-$C_{12}$alkyl, $C_2$-$C_{12}$alkanoyl or $C_7$-$C_{18}$phenylalkyl, each interrupted by one to six oxygen, sulfur or -N($R_6$)- groups further substituted by 1 to 3 -$COO^-Y^+$ groups; or
$R_4$ is $C_1$-$C_{12}$alkyl, $C_2$-$C_{12}$alkanoyl, phenyl or $C_7$-$C_{18}$phenylalkyl, each substituted by one or two -$COO^-Y^+$, -N($R_6$)($R_6$')($R_6$")$^+X^-$ or -$SO_3^-Y^+$ groups; or
$R_4$ is $C_1$-$C_{12}$alkyl, $C_2$-$C_{12}$alkanoyl, phenyl or $C_7$-$C_{18}$phenylalkyl, each of which is substituted by one or two -$COO^-Y^+$, -N($R_6$)($R_6$')($R_6$")$^+X^-$ or -$SO_3^-Y^+$ groups, each further substituted by one or two -OH, -$COOR_6$ or -$NHR_6$ groups.

For example, E is 2-hydroxycyclohexyloxy or 2-hydroxy-2-methylpropoxy.

For example, these water compatible or water soluble sterically hindered hydroxy substituted alkoxyamine compound are of the formula

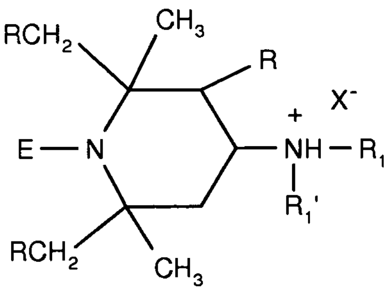

,

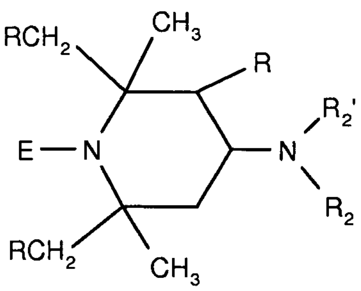

,

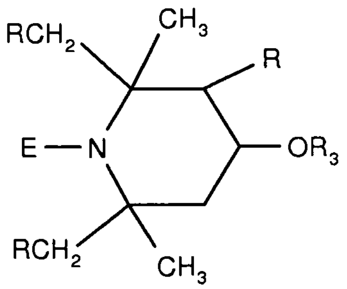 , 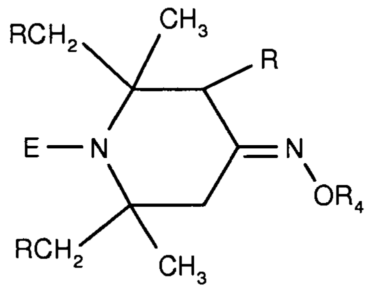 ,

where

E is $-O-T-(OH)_b$,

T is a straight or branched chain alkylene of 1 to 18 carbon atoms, cycloalkylene of 5 to 18 carbon atoms, cycloalkenylene of 5 to 18 carbon atoms, a straight or branched chain alkylene of 1 to 4 carbon atoms substituted by phenyl or by phenyl substituted by one or two alkyl groups of 1 to 4 carbon atoms;

b is 1, 2 or 3 with the proviso that b cannot exceed the number of carbon atoms in T, and when b is 2 or 3, each hydroxyl group is attached to a different carbon atoms of T;

R is hydrogen or methyl,

$R_1$ is hydrogen, $C_1$-$C_{12}$alkyl, $C_5$-$C_8$cycloalkyl, $C_5$-$C_8$cycloalkyl substituted by one to three $C_1$-$C_4$alkyl, $C_2$-$C_{12}$alkenyl, phenyl, phenyl substituted by one to three $C_1$-$C_4$alkyl, $C_7$-$C_9$phenylalkyl, $C_7$-$C_9$phenylalkyl substituted on the phenyl ring by one to three $C_1$-$C_4$alkyl, glycidyl, $C_2$-$C_{12}$alkanoyl, $C_6$-$C_9$cycloalkylcarbonyl, $C_2$-$C_{12}$carbamoyl, $C_2$-$C_{12}$alkenoyl, benzoyl, benzoyl substituted by one to three $C_1$-$C_4$alkyl, $C_2$-$C_{12}$alkanoyl substituted by a di($C_1$-$C_6$alkyl) phosphonate, or $R_1$ is $C_2$-$C_{12}$alkyl, $C_2$-$C_{12}$alkanoyl, $C_7$-$C_{18}$phenylalkyl or $C_7$-$C_{18}$phenylalkyl substituted on the phenyl ring by one to three $C_1$-$C_4$alkyl, each interrupted by one to six oxygen, sulfur or $>NR_6$ groups; $C_1$-$C_{12}$alkyl, $C_2$-$C_{12}$alkanoyl, $C_7$-$C_{18}$phenylalkyl or $C_7$-$C_{18}$phenylalkyl substituted on the phenyl ring by one to three $C_1$-$C_4$alkyl, each substituted by one to six hydroxy groups or by one to six $-NHR_6$ groups; $C_2$-$C_{12}$alkyl, $C_2$-$C_{12}$alkanoyl, $C_7$-$C_{18}$phenylalkyl or $C_7$-$C_{18}$phenylalkyl substituted on the phenyl ring by one to three $C_1$-$C_4$alkyl, each interrupted by one to three $-NR_6C(O)-$ groups; or $C_1$-$C_{12}$alkyl, $C_2$-$C_{12}$alkanoyl, $C_7$-$C_{18}$phenylalkyl or $C_7$-$C_{18}$phenylalkyl substituted on the phenyl ring by one to three $C_1$-$C_4$alkyl, each substituted by one to three $-SO_3H$ groups or by one to three $-COOR_6$ groups; or

$R_1$ is a mono-valent homo- or co-oligomer consisting of monomer units derived from monomers selected from the group consisting of ethylene oxide, propylene oxide, ethylene glycol, propylene glycol, acrylic acid, methacrylic acid, ethylene imine, acrylamide, vinyl formamide, vinyl alcohol and vinyl acetate; which homo- or co-oligomer consists of between 2 and about 24 monomer units;

$R_1$' is independently defined as for $R_1$,

$R_6$ is hydrogen or $C_1$-$C_6$alkyl,

$X^-$ is an inorganic or organic anion,

$Y^+$ is a mono-, di- or tri-valent cation, and

$R_2$ is $C_1$-$C_{12}$alkyl, $C_2$-$C_{12}$alkanoyl, or $C_7$-$C_{18}$phenylalkyl, each of which is substituted by one or two $-COO^-Y^+$, $-N(R_6)(R_6')(R_6'')^+X^-$ or $-SO_3^-Y^+$ groups, each further substituted by one or two - OH, $-COOR_6$ or $-NHR_6$ groups;

$R_2$ is $C_1$-$C_{12}$alkyl, $C_2$-$C_{12}$alkanoyl, or $C_7$-$C_{18}$phenylalkyl, each substituted by one or two $-COO^-Y^+$, $-N(R_6)(R_6')(R_6'')^+X^-$ or $-SO_3^-Y^+$ groups; or

$R_2$' is $C_2$-$C_{12}$alkyl, $C_2$-$C_{12}$alkanoyl, or $C_7$-$C_{18}$phenylalkyl, each interrupted by one to six oxygen, sulfur or $>NR_6$ groups; $C_1$-$C_{12}$alkyl, $C_2$-$C_{12}$alkanoyl, or $C_7$-$C_{18}$phenylalkyl, each substituted by one to six hydroxy groups or by one to six $-NHR_6$ groups;

$C_2$-$C_{12}$alkyl, $C_2$-$C_{12}$alkanoyl, or $C_7$-$C_{18}$phenylalkyl, each interrupted by one to three $-NR_6C(O)$-groups; or $R_2$ is $C_1$-$C_{12}$alkyl, $C_2$-$C_{12}$alkanoyl, or $C_7$-$C_{18}$phenylalkyl, each substituted by one to three $-SO_3H$ groups or by one to three $-COOR_6$ groups; or

$R_2$' is $C_1$-$C_{12}$alkyl, $C_2$-$C_{12}$alkanoyl, or $C_7$-$C_{18}$phenylalkyl, each of which is substituted by one or two $-COO^-Y^+$, $-N(R_6)(R_6')(R_6'')^+X^-$ or $-SO_3^-Y^+$ groups, each further substituted by one or two -OH, $-COOR_6$ or $-NHR_6$ groups;

$R_2$' is $C_1$-$C_{12}$alkyl, $C_2$-$C_{12}$alkanoyl, or $C_7$-$C_{18}$phenylalkyl, each substituted by one or two $-COO^-Y^+$, $-N(R_6)(R_6')(R_6'')^+X^-$ or $-SO_3^-Y^+$ groups; or

$R_2$' is a mono-valent homo- or co-oligomer consisting of monomer units derived from monomers selected from the group consisting of ethylene oxide, propylene oxide, ethylene glycol, propylene glycol, acrylic acid, methacrylic acid, ethylene imine, acrylamide, vinyl formamide, vinyl alcohol and vinyl acetate; which homo- or co-oligomer consists of between 2 and about 24 monomer units, or

$R_2$' is hydrogen,

$R_3$ is $C_1$-$C_{12}$alkyl, $C_2$-$C_{12}$alkanoyl, or $C_7$-$C_{18}$phenylalkyl, each of which is substituted by one or two -$COO^-Y^+$, -$N(R_6)(R_6')(R_6'')^+X^-$ or -$SO_3^-Y^+$ groups, each further substituted by one or two -OH, -$COOR_6$ or -$NHR_6$ groups;
$R_3$ is $C_1$-$C_{12}$alkyl, $C_2$-$C_{12}$alkanoyl, or $C_7$-$C_{18}$phenylalkyl, each substituted by one or two -$COO^-Y^+$, -$N(R_6)(R_6')(R_6'')^+X^-$ or -$SO_3^-Y^+$ groups; or
$R_3$ is -$SO_3^-Y^+$ or -$PO_3H^-Y^+$, and
$R_4$ is $C_1$-$C_{12}$alkyl, $C_2$-$C_{12}$alkanoyl, or $C_7$-$C_{18}$phenylalkyl, each of which is substituted by one or two -$COO^-Y^+$, -$N(R_6)(R_6')(R_6'')^+X^-$ or -$SO_3^-Y^+$ groups, each further substituted by one or two -OH, -$COOR_6$ or -$NHR_6$ groups;
$R_4$ is $C_1$-$C_{12}$alkyl, $C_2$-$C_{12}$alkanoyl, or $C_7$-$C_{18}$phenylalkyl, each substituted by one or two -$COO^-Y^+$, -$N(R_6)(R_6')(R_6'')^+X^-$ or -$SO_3^-Y^+$ groups.

For example, the present compounds are of the formula (1).

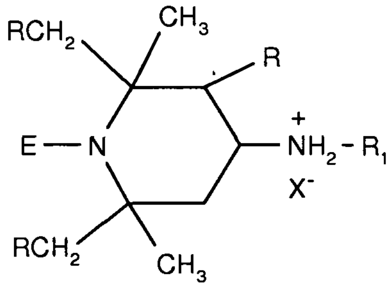

(1)

Preferably, $R_1$ is hydrogen, $C_1$-$C_6$alkyl, $C_2$-$C_6$alkanoyl, $C_2$-$C_6$alkyl or $C_2$-$C_6$alkanoyl interrupted by one or two oxygen, sulfur or -$N(R_6)$- groups; $C_1$-$C_6$alkyl or $C_2$-$C_6$alkanoyl substituted by one to three hydroxy groups or by one to three -$NHR_6$ groups, $C_2$-$C_6$alkyl or $C_2$-$C_6$alkanoyl interrupted by a -$NR_6C(O)$- group, or is $C_1$-$C_6$alkyl or $C_2$-$C_6$alkanoyl substituted by a -$SO_3H$ or by a -$COOR_6$ group.

Most preferably, $R_1$ is hydrogen, $C_1$-$C_4$alkyl, $C_2$-$C_5$alkanoyl, $C_2$-$C_4$alkyl or $C_2$-$C_5$alkanoyl interrupted by an oxygen, sulfur or -$N(R_6)$- group; $C_1$-$C_4$alkyl or $C_2$-$C_5$alkanoyl substituted by an hydroxy group or by a -$NHR_6$ group, $C_2$-$C_4$alkyl or $C_2$-$C_5$alkanoyl interrupted by a - $NR_6C(O)$- group, or is $C_1$-$C_4$alkyl or $C_2$-$C_5$alkanoyl substituted by a -$SO_3H$ or by a -$COOR_6$ group.

For instance, the present compounds are of the formula (2) or (3).

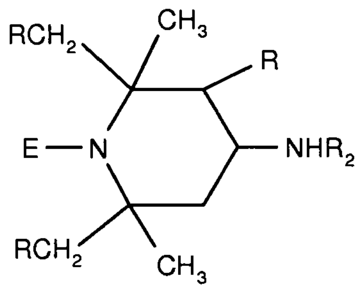

(2) or

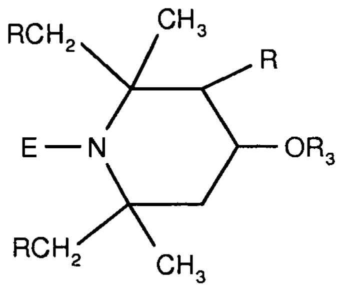

(3)

Preferably, $R_2$ and $R_3$ are $C_1$-$C_4$alkyl or $C_2$-$C_5$alkanoyl substituted by a -$COO^-Y^+$, -$N(R_6)(R_6')(R_6'')^+X^-$ or -$SO_3^-Y^+$ group.

For example, the present compounds are of the formula (4).

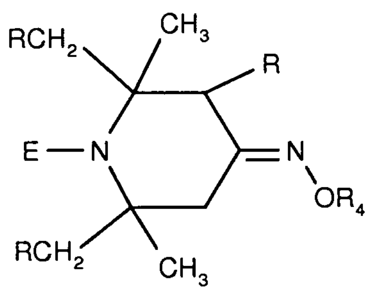

(4)

Preferably, $R_4$ is $C_1$-$C_4$alkyl or $C_2$-$C_5$alkanoyl substituted by a $-COO^-Y^+$, $-N(R_6)(R_6')(R_6'')^+X^-$ or $-SO_3^-Y^+$ group, and the other moities are as described previously.

For example, the present compounds are of the formula

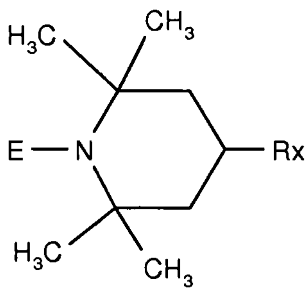

(11)

where

E is $-O-T(OH)_b$, and

where $R_x$ is selected from the group consisting of

$-NH_2^+CH_2CH_2OH\ Cl^-$, $-NH_3^+\ ^-OAc$, $-NHCH(CH_3)COO^-K^+$, $-NHCH_2CH_2NH(CH_3)_2^+\ ^-OAc$, $-NHCH_2CH_2SO_3^-K^+$, $-NHCH(COO^-\ K^+)CH_2CH_2SCH_3$, $-NHCH_2COO^-\ K^+$, $-OCH(CH_3)COO^-K^+$, $-OCH_2CH_2NH(CH_3)_2^+\ ^-OAc$, $-OCH_2CH_2SO_3^-K^+$, $-OCH(COO^-\ K^+)CH_2CH_2SCH_3$, $^-OCH_2COO^-\ K^+$.

For example, the present compounds are of the formula (1), (2), (3) or (5)

wherein E is $-O-T(OH)_b$;

R is hydrogen or methyl;

$R_1$ is hydrogen, $C_1$-$C_4$alkyl substituted by a hydroxy or a $COOR_6$ group; or $R_1$ is $C_2$-$C_4$alkyl interupted by a $-N(R_6)$- group; or $R_1$ is $C_2$-$C_{12}$alkyl interupted by 1 to 6 $-N(R_6)$- groups;

$R_1'$ is hydrogen;

$R_2$ is $C_1$-$C_4$alkyl substituted by a $-COO^-Y^+$, $-N(R_6)(R_6')(R_6'')^+X^-$, $-SO_3^-Y^+$, or $R_2$ is $C_2$-$C_{12}$alkyl interrupted by one to six sulfur groups further substituted by 1 to 3 $-COO^-Y^+$ groups; or $R_2$ is phenyl substituted by 1 to 2 $-SO_3^-Y^+$ groups; or $R_2$ is phenyl which is substituted by one or two $-COO^-Y^+$ groups;

$R_2'$ is hydrogen;

$R_3$ is $-SO_3^-Y^+$, $C_2$-$C_5$alkanoyl substituted by a $-COO^-Y^+$ group;

$R_4$ is hydrogen;

$R_6$ is hydrogen or $C_1$-$C_6$alkyl;

$R_6'$ is $C_1$-$C_6$alkyl;

$R_6''$ is hydrogen or $C_1$-$C_6$alkyl;

$R_7$ is $-N(R_2)(R_2')$ or

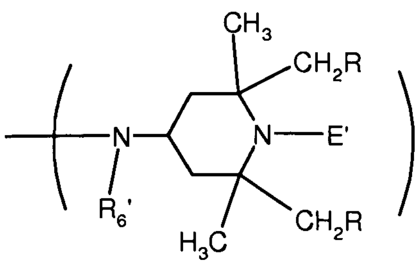

;

E' is hydrogen, $C_1$-$C_{18}$alkyl, $C_2$-$C_{18}$alkenyl, $C_7$-$C_{15}$phenylalkyl, $C_2$-$C_{18}$alkanoyl or phenyl, or E' is independently defined as for E,

$R_8$ is as defined for $R_7$, where one of $R_7$ and $R_8$ is $-N(R_2)(R_2')$;

$X^-$ is an inorganic or organic anion;

$Y^+$ is a mono-, di- or trivalent cation.

The solubility of the present sterically hindered amines in water at 20°C and standard pressure is for example greater than or equal to 1 g/L, for example ≥ 2 g/L, ≥ 5 g/L, ≥ 10 g/L, ≥ 20 g/L, ≥ 30 g/L, ≥ 40 g/L, ≥ 50 g/L, ≥ 60 g/L, ≥

70 g/L, ≥ 80 g/L, ≥ 90 g/L or ≥ 100 g/L.

Alkyl having up to 12 carbon atoms is branched or unbranched, and is for example for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl and dodecyl, 1,1,3,3,5,5-hexamethylhexyl.

Alkenyl is an unsaturated version of alkyl, and is branched or unbranched, for example isopropenyl, propenyl, hexenyl, heptenyl, and the like.

Unsubstituted or $C_1$-$C_4$alkyl-substituted $C_5$-$C_8$cycloalkyl is, for example, cyclopentyl, methylcyclopentyl, dimethylcyclopentyl, cyclohexyl, methylcyclohexyl, dimethylcyclohexyl, trimethylcyclohexyl, tert-butylcyclohexyl, cycloheptyl or cyclooctyl. For example cyclohexyl and tert-butylcyclohexyl.

$C_1$-$C_4$Alkyl-substituted phenyl, which contains for example 1 to 3, for instance 1 or 2, alkyl groups, is, for example, o-, m- or p-methylphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2-methyl-6-ethylphenyl, 4-tert-butylphenyl, 2-ethylphenyl or 2,6-diethylphenyl.

Phenylalkyl includes substituted phenylalkyl, for example phenylalkyl substituted on the phenyl ring by from 1 to 3 $C_1$-$C_4$alkyl groups or from 1 to 3 halogen or by a mixture thereof, and is for example, benzyl, 4-chlorobenzyl, $\alpha$-methylbenzyl, $\alpha,\alpha$-dimethylbenzyl, 2-phenylethyl, 2-methylbenzyl, 3-methylbenzyl, 4-methylbenzyl, 2,4-dimethylbenzyl, 2,6-dimethylbenzyl or 4-tert-butylbenzyl.

Phenyl includes unsubstituted phenyl and phenyl substituted by from 1 to 3 $C_1$-$C_4$ alkyl groups or from 1 to 3 halogen or by a mixture thereof.

Alkanoyl having up to 12 carbon atoms is branched or unbranched, and is for example formyl, acetyl, propionyl, butanoyl, pentanoyl, hexanoyl, heptanoyl, octanoyl, nonanoyl, decanoyl, undecanoyl or dodecanoyl.

$C_6$-$C_9$Cycloalkylcarbonyl is, for example, cyclopentylcarbonyl, cyclohexylcarbonyl, cycloheptylcarbonyl or cyclooctylcarbonyl.

Carbamoyl of up to 12 carbon atoms is branched or unbranched, and is for example the carbamoyl equivalent of the alkanoyl groups, for example butamoyl, pentamoyl, hexamoyl and the like.

Alkenoyl of up to 12 carbon atoms is branched or unbranched, and is an unsaturated version of alkanoyl.

Benzoyl substituted by one to three $C_1$-$C_4$alkyl, is for example o-, m- or p-methylbenzoyl, 2,3-dimethylbenzoyl, 2,4-dimethylbenzoyl, 2,5-dimethylbenzoyl, 2,6-dimethylbenzoyl, 3,4-dimethylbenzoyl, 3,5-dimethylbenzoyl, 2-methyl-6-ethylbenzoyl, 4-tert-butylbenzoyl, 2-ethylbenzoyl, 2,4,6-trimethylbenzoyl, 2,6-dimethyl-4-tert-butylbenzoyl or 3,5-di-tert-butylbenzoyl.

$C_2$-$C_{25}$Alkanoyl substituted by a di($C_1$-$C_6$alkyl) phosphonate group is, for example, $(CH_3CH_2O)_2POCH_2CO$-, $(CH_3O)_2POCH_2CO$-, $(CH_3CH_2CH_2CH_2O)_2POCH_2CO$-, $(CH_3CH_2O)_2POCH_2CH_2CO$-, $(CH_3O)_2POCH_2CH_2CO$-, $(CH_3CH_2CH_2CH_2O)_2POCH_2CH_2CO$-, $(CH_3CH_2O)_2PO(CH_2)_4CO$-, $(CH_3CH_2O)_2PO(CH_2)_8CO$- or $(CH_3CH_2O)_2PO(CH_2)_{17}CO$-.

$C_2$-$C_{12}$Alkyl interrupted by oxygen, sulfur or by -N($R_6$)- is, for example, $CH_3$-O-$CH_2$-, $CH_3$-S-$CH_2$-, $CH_3$-N($CH_3$)-$CH_2$-, $CH_3$-O-$CH_2CH_2$-O-$CH_2$-, $CH_3$-(O-$CH_2CH_2$-$)_2$O-$CH_2$-, $CH_3$-(O-$CH_2CH_2$-$)_3$O-$CH_2$- or $CH_3$-(O-$CH_2CH_2$-$)_4$O-$CH_2$-.

$C_3$-$C_{12}$Alkanoyl interrupted by oxygen, sulfur or by -N($R_6$)- is, for example, $CH_3$-O-$CH_2$CO-, $CH_3$-S-$CH_2$CO-, $CH_3$-N($CH_3$)-$CH_2$CO-, $CH_3$-O-$CH_2CH_2$-O-$CH_2$CO-, $CH_3$-(O-$CH_2CH_2$-$)_2$O-$CH_2$CO-, $CH_3$-(O-$CH_2CH_2$-$)_3$O-$CH_2$CO- or $CH_3$-(O-$CH_2CH_2$-$)_4$O-$CH_2$CO-.

Substitution by one to six also means of course by 1, 2, 3, 4, 5 or 6 groups, if the length of the chain so allows. Substitution by one to three means of course by 1, 2 or 3 groups, if the length of the chain allows.

Interruption by one to six groups means of course by 1, 2, 3, 4, 5 or 6 groups, if length of the chain allows.

$C_7$-$C_{18}$Phenylalkyl interrupted by oxygen, sulfur or by -N($R_6$)- and unsubstituted or substituted on the phenyl radical by from one to three $C_1$-$C_4$alkyl groups is branched or unbranched, and is for example phenoxymethyl, 2-methyl-phenoxymethyl, 3-methyl-phenoxymethyl, 4-methyl-phenoxymethyl, 2,4-dimethyl-phenoxymethyl, 2,3-dimethyl-phenoxymethyl, phenylthiomethyl, N-methyl-N-phenyl-aminomethyl, N-ethyl-N-phenyl-aminomethyl, 4-tert-butyl-phenoxymethyl, 4-tert-butyl-phenoxyethoxy-methyl, 2,4-di-tert-butyl-phenoxymethyl, 2,4-di-tert-butylphenoxyethoxymethyl, phenoxyethoxyethoxyethoxymethyl, benzyloxymethyl, benzyloxyethoxymethyl, N-benzyl-N-ethyl-aminomethyl or N-benzyl-N-isopropyl-aminomethyl.

The oligomers and co-oligomers of ethylene oxide, propylene oxide, ethylene glycol, propylene glycol, acrylic acid, methacrylic acid, ethylene imine, vinyl alcohol and vinyl acetate are of course oligomeric and co-oligomeric versions of poly(ethylene oxide), poly(propylene oxide), polyethylene glycol, polypropylene glycol, polyacrylic acid, polymethacrylic acid, poly(ethylene imine), polyacrylamide, polyvinylformamide, polyvinyl alcohol and polyvinyl acetate. Oligomers of the above, when mono-valent, may be capped with for example a methyl group or an acetate group, for example the oligomers of polyethylene glycol and polypropylene glycol. Homo- or co-oligomers between 2 and 24 monomer units are for example between 2 and 20 monomer units, between 2 and 17 monomer units, between 2 and 14 monomer units,

between 2 and 11 monomer units, between 2 and 9 monomer units, between 2 and 8 monomer units, between 2 and 6 monomer units, between 2 and 5 monomer units, or between 2 and 4 monomer units. The total number of carbon atoms in the oligomers and co-oligomers is for example less than 20.

$X^-$ is an inorganic or organic anion, such as phosphate, phosphonate, carbonate, bicarbonate, nitrate, chloride, bromide, bisulfite, sulfite, bisulfate, sulfate, borate, formate, acetate (OAc), benzoate, citrate, oxalate, tartrate, acrylate, polyacrylate, fumarate, maleate, itaconate, glycolate, gluconate, malate, mandelate, tiglate, ascorbate, polymethacrylate, a carboxylate of nitrilotriacetic acid, hydroxyethylethylenediaminetriacetic acid, ethylenediaminetetraacetic acid or of diethylenetriaminepentaacetic acid, a diethylenetriaminepentamethylenephosphonate, an alkylsulfonate or an arylsulfonate. Of course, where it is for example a di-, tri- or quaternary valent anion, it forms ion pairs with 2, 3 or 4 cations respectively.

$Y^+$ is a mono-, di- or tri-valent cation and is for example an alkali metal cation, alkaline earth metal cation or aluminum cation. For example, $Y^+$ is $Na^+$, $K^+$, $Mg^{++}$, $Ca^{++}$ or $Al^{+++}$. Of course, where it is a di- or tri-cation, it forms ion pairs with 2 or 3 anions respectively.

Also subject of the present invention are stabilized compositions comprising an organic material subject to the deleterious effects of light, heat and oxygen, and a water compatible or water soluble sterically hindered hydroxy substituted alkoxyamine compound of the present invention.

Such a composition may be a coating, ink jet ink, ink jet recording material, photographic recording material, multi-layer polymer structure, a coextruded film, a radiation cured film, ink or coating, an adhesive or a laminate.

Such a composition may additionally comprise at least one coadditive stabilizer selected from the group consisting of the phenolic antioxidants, metal stearates, metal oxides, organophosphorus compounds, benzofuranone antioxidants, hydroxylamines, ultraviolet light absorbers, and other hindered amine light stabilizers.

Such a composition may additionally comprises an ultraviolet light absorber selected from the group consisting of the benzophenones, 2H-benzotriazoles, aryl-s-triazines.

Such a composition may be a colored composition containing pigments or dyes.

The present invention also pertains to a process for stabilizing organic material subject to oxidative, thermal and/or actinic degradation which comprises adding to or incorporating into said material at least one compound as of the present invention.

Organic materials to be protected against the damaging effect of light, oxygen and/or heat are or contain in particular organic polymers, preferably synthetic organic polymers. The compounds of the present invention exhibit high thermal stability, compatibility and good persistence in the materials they are incorporated in or applied to.

The present water compatible or water soluble sterically hindered hydroxy substituted alkoxyamine compounds are particularly effective towards preventing color fading of compositions comprising pigments or dyes.

Accordingly, colored compositions comprising pigments or dyes are stabilized compositions according to this invention. That is, the stabilized organic material may be the pigment or dye.

The colored compositions are for example compositions comprising dyes, which compositions are selected from the group consisting of ink jet inks, ink jet recording media, coatings, body care products, household products, textiles and fabrics.

The amount of instant stabilizer compounds employed in the compositions of this invention are for example from 0.001% to 10% by weight, based on the weight of the composition and depending on the composition.

For example, the amount of the present stabilizers employed in body care products, household products, textiles and fabrics is from 0.001% to 10% by weight, or from 0.001% to 5% by weight, based on the weight of the composition.

The amount of instant stabilizer compounds employed in coatings is from 0.1 to 10% by weight, for example from 0.2 to 5% by weight, for example from 0.5 to 3% by weight based on the weight of the solvent-free binder. The binders can be dissolved or dispersed in customary organic solvents or in water or can be solvent-free.

If combinations of stabilizers are used, the sum of all light stabilizers is for example from 0.2 to 20% by weight, for instance from 0.5 to 5% by weight, based on the film-forming resin.

The ink jet ink according to this invention comprises 0.01 to 30% by weight, for example 0.1 to 20% by weight, of at least one present sterically hindered amine stabilizer, based on the weight of the ink jet ink.

The ink jet recording material according to this invention comprises 1 to 10000 $mg/m^2$, for example 50 to 2000 $mg/m^2$, of at least one present sterically hindered amine stabilizer.

The present sterically hindered amines can be dissolved either directly in the ink or coating composition or added thereto in the form of an emulsion or suspension. As already mentioned, the present sterically hindered amines can be also applied to the recording material in a separate operation, alone or together with other already described components, as a solution in water or in a suitable organic solvent. Application can be made by spraying, by sizing in a sizing press, by a separate coating operation or by immersion in a vat. After subjecting the recording material to such an after treatment, an additional drying step is necessary.

As mentioned, the stabilized compositions of the invention may optionally also contain other conventional stabilizers. For example, the compositions of this invention may contain from 0.01 to 5%, preferably from 0.025 to 2%,

and especially from 0.1 to 1% by weight of various conventional additives, such as the materials listed below, or mixtures thereof.

1. Antioxidants

1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-tert-butyl-4-methoxymethylphenol, nonylphenols which are linear or branched in the side chains, for example, 2,6-di-nonyl-4-methylphenol, 2,4-dimethyl-6-(1-methylundec-1-yl)phenol, 2,4-dimethy)-6-(1-methylheptadec-1-yl)phenol, 2,4-dimethy)-6-(1-methyltridec-1-yl)phenol and mixtures thereof.

1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-didodecylth iomethyl-4-nonylphenol.

1.3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis-(3,5-di-tert-butyl-4-hydroxyphenyl) adipate.

1.4. Tocopherols, for example α-tocopherol, β-tocopherol, γ-tocopherol, δ-tocopherol and mixtures thereof (Vitamin E).

1.5. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methylphenol), 4,4'-thiobis-(3,6-di-sec-amylphenol), 4,4'-bis(2,6-dimethyl-4-hydroxyphenyl)disulfide.

1.6. Alkylidenebisphenols, for example 2,2'-methylenebis(6-tert-butyl-4-methylphenol), 2,2'-methylenebis(6-tert-butyl-4-ethylphenol), 2,2'-methylenebis[4-methyl-6-(α-methylcyclohexyl)-phenol], 2,2'-methylenebis(4-methyl-6-cyclohexylphenol), 2,2'-methylenebis(6-nonyl-4-methylphenol), 2,2'-methylenebis(4,6-di-tert-butylphenol), 2,2'-ethylidenebis(4,6-di-tert-butylphenol), 2,2'-ethylidenebis(6-tert-butyl-4-isobutylphenol), 2,2'-methylenebis[6-(α-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methyl-phenyl)-3-n-dodecylmercaptobutane, ethylene glycol bis[3,3-bis (3-tert-butyl-4-hydroxyphenyl)butyrate], bis(3-tert-butyl-4-hydroxy-5-methyl-phenyl)dicyclopentadiene, bis[2-(3'tert-butyl-2-hydroxy-5-methylbenzyl)-6-tert-butyl-4-methylphenyl]terephthalate, 1,1-bis-(3,5-dimethyl-2-hydroxyphenyl) butane, 2,2-bis-(3,5-di-tert-butyl-4-hydroxyphenyl)propane, 2,2-bis-(5-tert-butyl-4-hydroxy2-methylphenyl)-4-n-dodecylmercaptobutane, 1,1,5,5-tetra-(5-tert-butyl-4-hydroxy-2-methylphenyl)pentane.

1.7. Benzyl compounds, for example 3,5,3',5'-tetra-tert-butyl-4,4'-dihydroxydibenzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine,

1,3,5-tri-(3,5-di-tert-butyl-4-hydroxybenzyl)-2,4,6-trimethylbenzene, di-(3,5-di-tert-butyl-4-hydroxybenzyl) sulfide, 3,5-di-tert-butyl-4-hydroxybenzyl-mercapto-acetic acid isooctyl ester, bis-(4-tert-butyl-3-hydroxy-2,6-dimethylbenzyl)dithiol terephthalate, 1,3,5-tris-(3,5-di-tert-butyl-4-hydroxybenzyl) isocyanurate, 1,3,5-tris-(4-tert-butyl-3-hydroxy-2,6-dimethylbenzyl) isocyanurate, 3,5-di-tert-butyl-4-hydroxybenzyl-phosphoric acid dioctadecyl ester and 3,5-di-tert-butyl-4-hydroxybenzyl-phosphoric acid monoethyl ester, calcium-salt.

1.8. Hydroxybenzylated malonates, for example dioctadecyl-2,2-bis-(3,5-di-tert-butyl-2-hydroxybenzyl)-malonate, di-octadecyl-2-(3-tert-butyl-4-hydroxy-5-methylbenzyl)-malonate, di-dodecylmercaptoethyl-2,2-bis-(3,5-di-tert-butyl-4-hydroxybenzyl)malonate, bis[4-(1,1,3,3-tetramethylbutyl)phenyl]-2,2-bis(3,5-di-tert-butyl-4-hydroxybenzyl) malonate.

1.9. Aromatic hydroxybenzyl compounds, for example 1,3,5-tris-(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene, 2,4,6-tris(3,5-di-tert-butyl-4-hydroxybenzyl)phenol.

1.10. Triazine compounds, for example 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris-(3,5-di-tert-butyl-4-hydroxyphenoxy)-1,2,3-triazine, 1,3,5-tris-(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris(3,5-di-tert-butyl-4-hydroxyphenylethyl)-1,3,5-triazine, 1,3,5-tris(3,5-di-tert-butyl-4-hydroxyphenylpropionyl)-hexahydro-1,3,5-triazine, 1,3,5-tris(3,5-dicyclohexyl-4-hydroxybenzyl)isocyanurate.

1.11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl3,5-di-tert-butyl-4-hydroxybenzylphosphonate, dioctadecyl-5-tert-butyl-4-hydroxy-3-methylbenzylphosphonate, the calcium salt of the monoethyl ester of 3,5-di-tert-butyl-4-hydroxybenzylphosphonic acid.

1.12. Acylaminophenols, for example 4-hydroxy-lauric acid anilide, 4-hydroxy-stearic acid anilide, 2,4-bis-octylmercapto-6-(3,5-tert-butyl-4-hydroxyanilino)-s-triazine and octyl-N-(3,5-di-tert-butyl-4-hydroxyphenyl)-carbamate.

1.13. Esters of β-(3,5-di-tert-butyl-4-hydroxyphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.

1.14. Esters of β-(5-tert-butyl-4-hydroxy-3-methylphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.

1.15. Esters of β-(3,5-dicyclohexyl-4-hydroxyphenyl)propionic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.

1.16. Esters of 3,5-di-tert-butyl-4-hydroxyphenyl acetic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis (hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.

1.17. Amides of β-(3,5-di-tert-butyl-4-hydroxyphenyl)propionic acid e.g. N,N'-bis(3,5-di-tert-butyl-4-hydroxyphenylpropionyl)hexamethylenediamide, N,N'-bis(3,5-di-tert-butyl-4-hydroxyphenylpropionyl)trimethylenediamide, N,N'-bis(3,5-di-tert-butyl-4-hydroxyphenylpropionyl)-hydrazide, N,N'-bis[2-(3-[3,5-di-tert-butyl-4-hydroxyphenyl]propionyloxy)ethyl]oxamide (Naugard®XL-1 supplied by Uniroyal).

1.18. Ascorbic acid (vitamin C)

1.19. Aminic antioxidants, for example N,N'-di-isopropyl-p-phenylenediamine, N,N'-di-sec-butyl-p-phenylenediamine, N,N'-bis(1,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-p-phenylenediamine, N-isopropyl-N'-phenyl-p-phenylenediamine, N-(1,3-dimethylbutyl)-N'-phenyl-p-phenylenediamine, N-(1-methylheptyl)-N'-phenyl-p-phenylenediamine, N-cyclohexyl-N'-phenyl-p-phenlenediamine, 4-(p-toluenesulfamoyl)diphenylamine, N,N'-dimethyl-N,N'-di-sec-butyl-p-phenylenediamine, diphenylamine, N-allyldiphenylamine, 4-isopropoxydiphenylamine, N-phenyl-1-naphthylamine, N-(4-tert-octylphenyl)-1-naphthylamine, N-phenyl-2-naphthylamine, octylated diphenylamine, for example p,p'-di-tert-octyldiphenylamine, 4-n-butylaminophenol,

4-butyrylaminophenol, 4-nonanoylaminophenol, 4-dodecanoylaminophenol, 4-octadecanoylaminophenol, bis(4-methoxyphenyl)amine, 2,6-di-tert-butyl-4-dimethylaminomethylphenol, 2,4'-diaminodiphenylmethane, 4,4'-diaminodiphenylmethane, N,N,N',N'-tetramethyl-4,4'-diaminodiphenylmethane, 1,2-bis[(2-methylphenyl)amino]ethane, 1,2-bis(phenylamino)propane, (o-tolyl)biguanide, bis[4-(1',3'-dimethylbutyl)phenyl]amine, tertoctylated N-phenyl-1-naphthylamine, a mixture of mono- and dialkylated tert-butyl/tert-octyldiphenylamines, a mixture of mono- and dialkylated nonyldiphenylamines, a mixture of mono- and dialkylated dodecyldiphenylamines, a mixture of mono- and dialkylated isopropyl/isohexyldiphenylamines, a mixture of mono- and dialkylated tertbutyldiphenylamines, 2,3-dihydro-3,3-dimethyl-4H-1,4-benzothiazine, phenothiazine, a mixture of mono- and dialkylated tert-butyl/tert-octylphenothiazines, a mixture of mono- and dialkylated tert-octyl-phenothiazines, N-allylphenothiazin, N,N,N',N'-tetraphenyl-1,4-diaminobut-2-ene, N,N-bis(2,2,6,6-tetramethyl-piperid-4-yl-hexamethylenediamine, bis(2,2,6,6-tetramethylpiperid-4-yl)sebacate, 2,2,6,6-tetramethylpiperidin-4-one, 2,2,6,6-tetramethylpiperidin-4-ol.

2. UV absorbers and light stabilizers

2.1. 2-(2-Hydroxyphenyl)-2H-benzotriazoles, for example known commercial hydroxyphenyl-2H-benzotriazoles and benzotriazoles as disclosed in, United States Patent Nos. 3,004,896; 3,055,896; 3,072,585; 3,074,910; 3,189,615; 3,218,332; 3,230,194; 4,127,586; 4,226,763; 4,275,004; 4,278,589; 4,315,848; 4,347,180; 4,383,863; 4,675,352; 4,681,905, 4,853,471; 5,268,450; 5,278,314; 5,280,124; 5,319,091; 5,410,071; 5,436,349; 5,516,914; 5,554,760; 5,563,242; 5,574,166; 5,607,987, 5,977,219 and 6,166,218 such as 2-(2-hydroxy-5-methylphenyl)-2H-benzotriazole, 2-(3,5-di-t-butyl-2-hydroxyphenyl)-2H-benzotriazole, 2-(2-hydroxy-5-t-butylphenyl)-2H-benzotriazole, 2-(2-hydroxy-5-t-octylphenyl)-2H-benzotriazole, 5-chloro-2-(3,5-di-t-butyl-2-hydroxyphenyl)-2H-benzotriazole, 5-chloro-2-(3-t-butyl-2-hydroxy-5-methylphenyl)-2H-benzotriazole, 2-(3-sec-butyl-5-t-butyl-2-hydroxyphenyl)-2H-benzotriazole, 2-(2-hydroxy-4-octyloxyphenyl)-2H-benzotriazole, 2-(3,5-di-t-amyl-2-hydroxyphenyl)-2H-benzotriazole, 2-(3,5-bis-α-cumyl-2-hydroxyphenyl)-2H-benzotriazole, 2-(3-t-butyl-2-hydroxy-5-(2-(ω-hydroxy-octa-(ethyleneoxy) carbonyl-ethyl)-, phenyl)-2H-benzotriazole, 2-(3-dodecyl-2-hydroxy-5-methylphenyl)-2H-benzotriazole, 2-(3-t-butyl-2-hydroxy-5-(2-octyloxycarbonyl)ethylphenyl)-2H-benzotriazole, dodecylated 2-(2-hydroxy-5-methylphenyl)-2H-benzotriazole, 2-(3-t-butyl-2-hydroxy-5-(2-octyloxycarbonylethyl)phenyl)-5-chloro-2H-benzotriazole, 2-(3-tert-butyl-5-(2-(2-ethylhexyloxy)-carbonylethyl)-2-hydroxyphenyl)-5-chloro-2H-benzotriazole, 2-(3-t-butyl-2-hydroxy-5-(2-methoxycarbonylethyl)phenyl)-5-chloro-2H-benzotriazole, 2-(3-t-butyl-2-hydroxy-5-(2-methoxycarbonylethyl)phenyl)-2H-benzotriazole, 2-(3-t-butyl-5-(2-(2-ethylhexyloxy)carbonylethyl)-2-hydroxyphenyl)-2H-benzotriazole, 2-(3-t-butyl-2-hydroxy-5-(2-isooctyloxycarbonylethyl)phenyl-2H-benzotriazole, 2,2'-methylene-bis(4-t-octyl-(6-2H-benzotriazol-2-yl)phenol), 2-(2-hydroxy-3-α-cumyl-5-t-octylphenyl)-2H-benzotriazole, 2-(2-hydroxy-3-t-octyl-5-α-cumylphenyl)-2H-benzotriazole, 5-fluoro-2-(2-hydroxy-3,5-di-α-cumylphenyl)-2H-benzotriazole, 5-chloro-2-(2-hydroxy-3,5-di-α-cumylphenyl)-2H-benzotriazole, 5-chloro-2-(2-hydroxy-3-α-cumyl-5-t-octylphenyl)-2H-benzotriazole, 2-(3-t-butyl-2-hydroxy-5-(2-isooctyloxycarbonylethyl)phenyl)-5-chloro-2H-benzotriazole, 5-trifluoromethyl-2-(2-hydroxy-3-α-cumyl-5-t-octylphenyl)-2H-benzotriazole, 5-trifluoromethyl-2-(2-hydroxy-5-t-octylphenyl)-2H-benzotriazole, 5-trifluoromethyl-2-(2-hydroxy-3,5-di-t-octylphenyl)-2H-benzotriazole, methyl 3-(5-trifluoromethyl-2H-benzotriazol-2-yl)-5-t-butyl-4-hydroxyhydrocinnamate, 5-butylsulfonyl-2-(2-hydroxy-3-α-cumyl-5-t-octylphenyl)-2H-benzotriazole, 5-trifluoromethyl-2-(2-hydroxy-3-α-cumyl-5-t-butylphenyl)-2H-benzotriazole, 5-trifluoromethyl-2-(2-hydroxy-3,5-di-t-butylphenyl)-2H-benzotriazole, 5-trifluoromethyl-2-(2-hydroxy-3,5-di-α-cumylphenyl)-2H-benzotriazole, 5-butylsulfonyl-2-(2-hydroxy-3,5-di-t-butylphenyl)-2H-benzotriazole and 5-phenylsulfonyl-2-(2-hydroxy-3,5-di-t-butylphenyl)-2H-benzotriazole.

2.2. 2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives.

2.3. Esters of substituted and unsubstituted benzoic acids, as for example 4-tert-butylphenyl salicylate, phenyl salicylate, octylphenyl salicylate, dibenzoyl resorcinol, bis(4-tert-butylbenzoyl) resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.

2.4. Acrylates and malonates, for example, α-cyano-β,β-diphenylacrylic acid ethyl ester or isooctyl ester, α-carbomethoxy-cinnamic acid methyl ester, α-cyano-β-methyl-p-methoxy-cinnamic acid methyl ester or butyl ester, α-carbomethoxy-p-methoxy-cinnamic acid methyl ester, N-(β-carbomethoxy-β-cyanovinyl)-2-methyl-indoline, Sanduvor® PR25, dimethyl p-methoxybenzylidenemalonate (CAS# 7443-25-6), and Sanduvor® PR31, di-(1,2,2,6,6-pentamethylpiperidin-4-yl) p-methoxybenzylidenemalonate (CAS #147783-69-5).

2.5. Nickel compounds, for example nickel complexes of 2,2'-thio-bis-[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the monoalkyl esters, e.g. the methyl or ethyl ester, of 4-hydroxy-3,5-di-tert-butylbenzylphosphonic acid, nickel complexes of ketoximes, e.g. of 2-hydroxy-4-methylphenyl undecylketoxime, nickel complexes of 1-phenyl-4-lauroyl-5-hydroxypyrazole, with or without additional ligands.

2.6. Sterically hindered amine stabilizers, for example 4-hydroxy-2,2,6,6-tetramethylpiperidine, 1-allyl-4-hydroxy-2,2,6,6-tetramethylpiperidine, 1-benzyl-4-hydroxy-2,2,6,6-tetramethylpiperidine, bis(2,2,6,6-tetramethyl-4-piperidyl) sebacate, bis(2,2,6,6-tetramethyl-4-piperidyl) succinate, bis(1,2,2,6,6-pentamethyl-4-piperidyl) sebacate, bis(1-octyloxy-2,2,6,6-tetramethyl-4-piperidyl) sebacate, bis(1,2,2,6,6-pentamethyl-4-piperidyl) n-butyl-3,5-di-tert-butyl-4-hydroxybenzylmalonate, the condensate of 1-(2-hydroxyethyl)-2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4-piperidyl)-1,2,3,4-butane-tetracarboxylate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidyl)-2-n-butyl-2-(2-hydroxy-3,5-di-tert-butylbenzyl) malonate, 3-n-octyl-7,7,9,9-tetramethyl-1,3,8-triazaspiro[4.5]decan-2,4-dione, bis(1-octyloxy-2,2,6,6-tetramethylpiperidyl) sebacate, bis(1-octyloxy-2,2,6,6-tetramethylpiperidyl) succinate, linear or cyclic condensates of N,N'-bis-(2,2,6,6-tetramethyl-4-piperidyl)-hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidin-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensation product of N,N'-bis(2,2,6,6-tetramethyl-4-piperidyl)hexamethylenediamine and 4-cyclohexylamino-2,6-dichloro-1,3,5-triazine, a condensation product of 1,2-bis(3-aminopropylamino) ethane and 2,4,6-trichloro-1,3,5-triazine as well as 4-butylamino-2,2,6,6-tetramethylpiperidine (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-undecyl-7,7,9,9-tetramethyl-1-oxa-3,8-diaza-4-oxo-spiro[4,5]decane, a reaction product of 7,7,9,9-tetramethyl-2-cycloundecyl-1-oxa-3,8-diaza-4-oxospiro [4,5]decane and epichlorohydrin, 1,1-bis(1,2,2,6,6-pentamethyl-4-piperidyloxycarbonyl)-2-(4-methoxyphenyl)ethene, N,N'-bis-formyl-N,N'-bis(2,2,6,6-tetramethyl-4-piperidyl)hexamethylenediamine, diester of 4-methoxy-methylene-malonic acid with 1,2,2,6,6-pentamethyl-4-hydroxypiperidine, poly[methylpropyl-3-oxy-4-(2,2,6,6-tetramethyl-4-piperidyl)]siloxane, reaction product of maleic acid anhydride-$\alpha$-olefin-copolymer with 2,2,6,6-tetramethyl-4-aminopiperidine or 1,2,2,6,6-pentamethyl-4-aminopiperidine.

The sterically hindered amine may also be one of the compounds described in U.S. Pat. No. 5,980,783, the relevant parts of which are hereby incorporated by reference, that is compounds of component I-a), I-b), I-c), I-d), I-e), I-f), I-g), I-h), I-i), I-j), I-k) or I-l), in particular the light stabilizer 1-a-1, 1-a-2, 1-b-1, 1-c-1, 1-c-2, 1-d-1, 1-d-2, 1-d-3, 1-e-1, 1-f-1, 1-g-1, 1-g-2 or 1-k-1 listed on columns 64-72 of said U.S. Pat. No. 5,980,783.

The sterically hindered amine may also be one of the compounds described in EP 782994, for example compounds as described in claims 10 or 38 or in Examples 1-12 or D-1 to D-5 therein.

2.7. Sterically hindered amines substituted on the N-atom by a hydroxy-substituted alkoxy group, for example compounds such as 1-(2-hydroxy-2-methylpropoxy)-4-octadecanoyloxy-2,2,6,6-tetramethylpiperidine, 1-(2-hydroxy-2-methylpropoxy)-4-hexadecanoyloxy-2,2,6,6-tetramethylpiperidine, the reaction product of 1-oxyl-4-hydroxy-2,2,6,6-tetramethylpiperidine with a carbon radical from t-amylalcohol, 1-(2-hydroxy-2-methylpropoxy)-4-hydroxy-2,2,6,6-tetramethylpiperidine, 1-(2-hydroxy-2-methylpropoxy)-4-oxo-2,2,6,6-tetramethylpiperidine, bis(1-(2-hydroxy-2-methylpropoxy)-2,2,6,6-tetramethylpiperidin-4-yl) sebacate, bis(1-(2-hydroxy-2-methylpropoxy)-2,2,6,6-tetramethylpiperidin-4-yl) adipate, bis(1-(2-hydroxy-2-methylpropoxy)-2,2,6,6-tetramethylpiperidin-4-yl) succinate, bis(1-(2-hydroxy-2-methylpropoxy)-2,2,6,6-tetramethylpiperidin-4-yl) glutarate and 2,4-bis{N-[1-(2-hydroxy-2-methylpropoxy)-2,2,6,6-tetramethylpiperidin-4-yl]-N-butylamino}-6-(2-hydroxyethylamino)-s-triazine.

2.8. Oxamides, for example 4,4'-dioctyloxyoxanilide, 2,2'-diethoxyoxanilide, 2,2'-dioctyloxy-5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxy-disubstituted oxanilides and mixtures of o- and p-ethoxy-disubstituted oxanilides.

2.9. Tris-aryl-o-hydroxyphenyl-s-triazines, for example known commercial tris-aryl-o-hydroxyphenyl-s-triazines and triazines as disclosed in, WO 96/28431 and United States Patent Nos. 3,843,371; 4,619,956; 4,740,542; 5,096,489;

5,106,891; 5,298,067; 5,300,414; 5,354,794; 5,461,151; 5,476,937; 5,489,503; 5,543,518; 5,556,973; 5,597,854; 5,681,955; 5,726,309; 5,736,597; 5,942,626; 5,959,008; 5,998,116; 6,013,704; 6,060,543; 6,187,919; 6,242,598 and 6,255,483, for example 4,6-bis-(2,4-dimethylphenyl)-2-(2-hydroxy-4-octyloxyphenyl)-s-triazine, Cyasorb® 1164, Cytec Corp, 4,6-bis-(2,4-dimethylphenyl)-2-(2,4-dihydroxyphenyl)-s-triazine, 2,4-bis(2,4-dihydroxyphenyl)-6-(4-chlorophenyl)-s-triazine, 2,4-bis[2-hydroxy-4-(2-hydroxyethoxy)phenyl]-6-(4-chlorophenyl)-s-triazine, 2,4-bis[2-hydroxy-4-(2-hydroxy-4-(2-hydroxyethoxy)phenyl]-6-(2,4-dimethylphenyl)-s-triazine, 2,4-bis[2-hydroxy-4-(2-hydroxyethoxy)phenyl]-6-(4-bromophenyl)-s-triazine, 2,4-bis[2-hydroxy-4-(2-acetoxyethoxy)phenyl]-6-(4-chlorophenyl)-s-triazine, 2,4-bis(2,4-dihydroxyphenyl)-6-(2,4-dimethylphenyl)-s-triazine, 2,4-bis(4-biphenylyl)-6-(2-hydroxy-4-octyloxycarbonylethylideneoxyphenyl)-s-triazine, 2-phenyl-4-[2-hydroxy-4-(3-secbutyloxy-2-hydroxypropyloxy)phenyl]-6-[2-hydroxy-4-(3-sec-amyloxy-2-hydroxypropyloxy)-phenyl]-s-triazine, 2,4-bis(2,4-dimethylphenyl)-6-[2-hydroxy-4-(3-benzyloxy-2-hydroxypropyloxy)phenyl]-s-triazine, 2,4-bis(2-hydroxy-4-n-butyloxyphenyl)-6-(2,4-di-n-butyloxyphenyl)-s-triazine, 2,4-bis(2,4-dimethylphenyl)-6-[2-hydroxy-4-(3-nonyloxy*-2-hydroxypropyloxy)-5-$\alpha$-cumylphenyl]-s-triazine (* denotes a mixture of octyloxy, nonyloxy and decyloxy groups), methylenebis-{2,4-bis(2,4-dimethylphenyl)-6-[2-hydroxy-4-(3-butyloxy-2-hydroxypropoxy)phenyl]-s-triazine}, methylene bridged dimer mixture bridged in the 3:5', 5:5' and 3:3' positions in a 5:4:1 ratio, 2,4,6-tris(2-hydroxy-4-isooctyloxycarbonylisopropylideneoxyphenyl)-s-triazine, 2,4-bis(2,4-dimethylphenyl)-6-(2-hydroxy-4-hexyloxy-5-$\alpha$-cumylphenyl)-s-triazine, 2-(2,4,6-trimethylphenyl)-4,6-bis[2-hydroxy-4-(3-butyloxy-2-hydroxypropyloxy)phenyl]-s-triazine, 2,4,6-tris[2-hydroxy-4-(3-sec-butyloxy-2-hydroxypropyloxy)-phenyl]-s-triazine, mixture of 4,6-bis-(2,4-dimethylphenyl)-2-(2-hydroxy-4-(3-dodecyloxy-2-hydroxypropoxy)-phenyl)-s-triazine and 4,6-bis-(2,4-dimethylphenyl)-2-(2-hydroxy-4-(3-tridecyloxy-2-hydroxypropoxy)-phenyl)-s-triazine, Tinuvin® 400, Ciba Specialty Chemicals Corp., 4,6-bis-(2,4-dimethylphenyl)-2-(2-hydroxy-4-(3-(2-ethylhexyloxy)-2-hydroxypropoxy)-phenyl)-s-triazine and 4,6-diphenyl-2-(4-hexyloxy-2-hydroxyphenyl)-s-triazine.

3. <u>Metal deactivators,</u> for example N,N'-diphenyloxamide, N-salicylal-N'-salicyloyl hydrazine, N,N'-bis(salicyloyl) hydrazine, N,N'-bis(3,5-di-tert-butyl-4-hydroxyphenylpropionyl) hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl dihydrazide, N,N'-bis(salicyloyl)thiopropionyl dihydrazide.

4. <u>Phosphites and phosphonites</u>, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, bis(2,6-di-tert-butyl-4-methylphenyl)-pentaerythritol diphosphite, diisodecyloxypentaerythritol diphosphite, bis(2,4-di-tert-butyl-6-methylphenyl)pentaerythritol diphosphite, bis(2,4,6-tris(tert-butylphenyl)pentaerythritol diphosphite, tristearyl sorbitol triphosphite, tetrakis(2,4-di-tert-butylphenyl) 4,4'-biphenylene diphosphonite, 6-isooctyloxy-2,4,8,1 0-tetra-tert-butyldibenzo[d,f][1,3,2]dioxaphosphepin, 6-fluoro-2,4,8,10-tetra-tert-butyl-12-methyldibenzo[d,g][1,3,2Jdioxaphosphocin, bis(2,4-di-tert-butyl-6-methylphenyl) methyl phosphite, bis(2,4-di-tert-butyl-6-methylphenyl) ethyl phosphite, 2,2',2''-nitrilo[triethyltris(3,3',5,5'-tetratert-butyl-1,1'-biphenyl-2,2'-diyl)phosphite], 2-ethylhexyl(3,3',5,5'-tetra-tert-butyl-1,1'-biphenyl-2,2'-diyl)phosphite.
Especially preferred are the following phosphites:
Tris(2,4-di-tert-butylphenyl) phosphite, tris(nonylphenyl) phosphite,

(A) 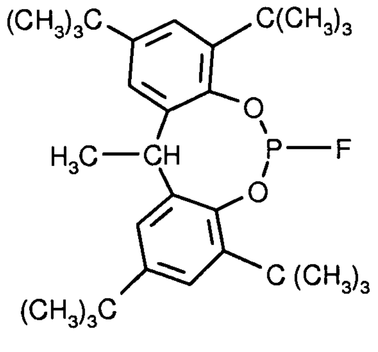

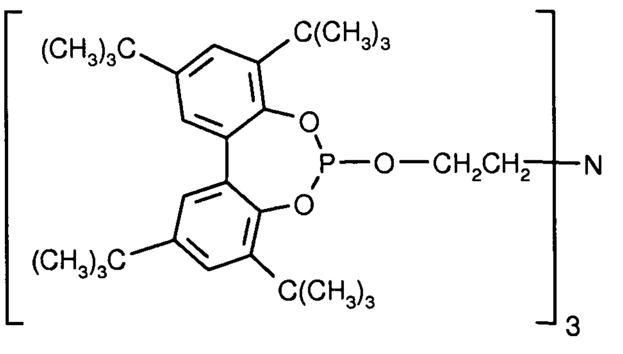 (B)

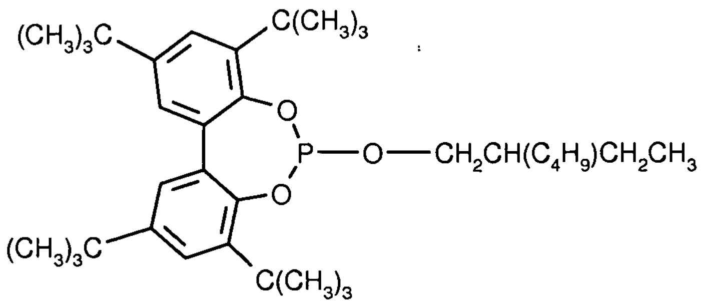 (C)

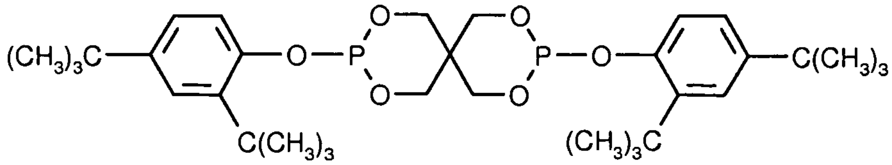 (D)

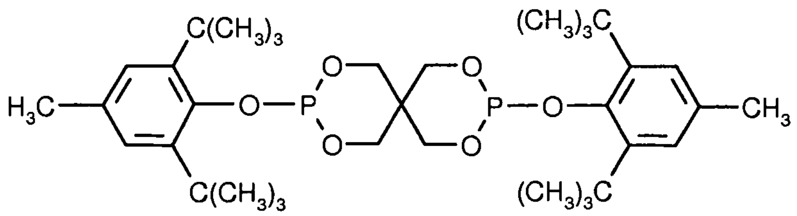 (E)

(F) 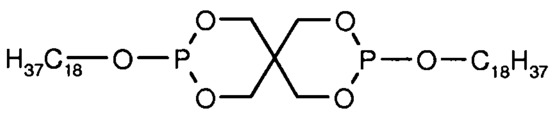 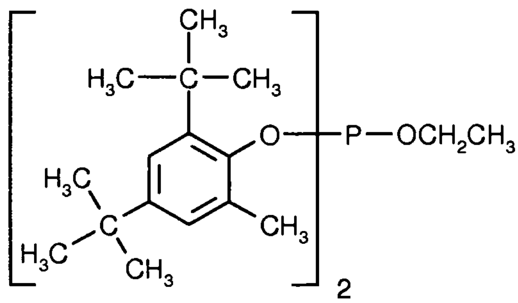 (G)

5. Hydroxylamines, for example N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N-methyl-N-octadecylhydroxylamine and the N,N-dialkylhydroxylamine derived from hydrogenated tallow amine.

6. Nitrones, for example N-benzyl-α-phenylnitrone, N-ethyl-α-methylnitrone, N-octyl-α-heptylnitrone, N-lauryl-α-undecylnitrone, N-tetradecyl-α-tridcylnitrone, N-hexadecyl-α-pentadecylnitrone, N-octadecyl-α-heptadecylnitrone, N-hexadecyl-α-heptadecylnitrone, N-ocatadecyl-α-pentadecylnitrone, N-heptadecyl-α-heptadecylnitrone, N-octadecyl-α-hexadecylnitrone, N-methyl-α-heptadecylnitrone and the nitrone derived from N,N-dialkylhydroxylamine derived from hydrogenated tallow amine.

7. Amine oxides, for example amine oxide derivatives as disclosed in U.S. Patent Nos. 5,844,029 and 5,880,191, didecyl methyl amine oxide, tridecyl amine oxide, tridodecyl amine oxide and trihexadecyl amine oxide.

8. Benzofuranones and indolinones, for example those disclosed in U.S. Pat. Nos. 4,325,863, 4,338,244, 5,175,312, 5,216,052, 5,252,643; DE-A-4316611;
DE-A-4316622; DE-A-4316876; EP-A-0589839 or EP-A-0591102 or 3-[4-(2-acetoxyethoxy)phenyl]-5,7-di-tert-butyl-benzofuran-2-one, 5,7-di-tert-butyl-3-[4-(2-stearoyloxyethoxy)phenyl]benzofuran-2-one, 3,3'-bis[5,7-di-tert-butyl-3-(4-[2-hydroxyethoxy]-phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butyl-benzofuran-2-one, 3-(3,5-dimethyl-4-pivaloyloxyphenyl)-5,7-di-tert-butyl-benzofuran-2-one, 3-(3,4-dimethylphenyl)-5,7-di-tert-butylbenzofuran-2-one, and 3-(2,3-dimethylphenyl)-5,7-di-tert-butyl-benzofuran-2-one.

9. Thiosynergists, for example dilauryl thiodipropionate or distearyl thiodipropionate.

10. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecylmercapto)propionate.

11. Polyamide stabilizers, for example copper salts in combination with iodides and/or phosphorus compounds and salts of divalent manganese.

12. Basic co-stabilizers, for example melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids, for example, calcium stearate, zinc stearate, magnesium behenate, magnesium stearate, sodium ricinoleate and potassium palmitate, antimony pyrocatecholate or zinc pyrocatecholate.

13. Nucleating agents, for example inorganic substances such as talcum, metal oxides such as titanium dioxide or magnesium oxide, phosphates, carbonates or sulfates of, preferably, alkaline earth metals; organic compounds such as mono- or polycarboxylic acids and the salts thereof, e.g. 4-tert-butylbenzoic acid, adipic acid, diphenylacetic acid, sodium succinate or sodium benzoate; polymeric compounds such as ionic copolymers (ionomers).

14. Fillers and reinforcing agents, for example calcium carbonate, silicates, glass fibres, glass bulbs, asbestos, talc, kaolin, mica, barium sulfate, metal oxides and hydroxides, carbon black, graphite, wood flour and flours or fibers of other natural products, synthetic fibers.

15. Dispersing Agents, such as polyethylene oxide waxes or mineral oil.

16. Other additives, for example plasticizers, lubricants, emulsifiers, pigments, dyes, optical brighteners, rheology additives, catalysts, flow-control agents, slip agents, crosslinking agents, crosslinking boosters, halogen scavengers, smoke inhibitors, flameproofing agents, antistatic agents, clarifiers such as substituted and unsubstituted bisbenzylidene sorbitols, benzoxazinone UV absorbers such as 2,2'-p-phenylene-bis(3,1-benzoxazin-4-one), Cyasorb® 3638 (CAS# 18600-59-4), and blowing agents.

The sterically hindered hydroxy substituted alkoxyamines of the present invention are prepared according to known methods, with the additional step of incorporating a water compatible or water soluble side chain.

The preparation of sterically hindered alkoxyamine stabilizers, also known as N-alkoxy hindered amines and NOR hindered amines or NOR hindered amine light stabilizers or NOR HALS, is disclosed for example in U.S. Pat. Nos. 5,004,770 and 5,096,950, the relevant disclosures of which are hereby incorporated by reference.

The preparation of sterically hindered hydroxy substituted alkoxyamine stabilizers, also known as hindered hydroxyalkoxyamine stabilizers, N-hydroxyalkoxy hindered amines, or NORol HALS, is disclosed for example in U.S. Pat. Nos. 6,271,377, 6,392,041 and 6,376,584, the relevant disclosures of which are hereby incorporated by reference.

The following non-limiting examples further illustrate the present invention.

Test compounds:

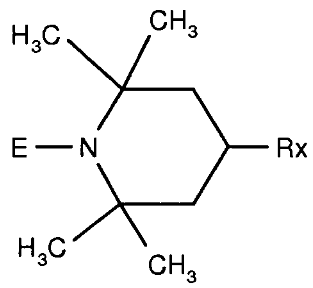

when E is 2-hydroxycyclohexyloxy or 2-hydroxy-2-methylpropoxy,
$R_x$ is selected from the group consisting of
$-NH_2^+CH_2CH_2OH\ Cl^-$, $-NH_3^+\ ^-OAc$, $-NHCH(CH_3)COO^-K^+$, $-NHCH_2CH_2NH(CH_3)_2^+\ ^-OAc$, $-NHCH_2CH_2SO_3^-K^+$, $-NHCH(COO^-K^+)CH_2CH_2SCH_3$, $-NHCH_2COO^-K^+$, $-OCH(CH_3)COO^-K^+$, $-OCH_2CH_2NH(CH_3)_2^+\ ^-OAc$, $-OCH_2CH_2SO_3^-K^+$, $-OCH(COO^-K^+)CH_2CH_2SCH_3$, $-OCH_2COO^-K^+$.

The following Examples more particularly point out the aspects of the present invention.

The Examples are as follows:

Compounds: Examples 1-18, 20, 30, 32, 34.
Ink Jet Media: Examples 21-29, 31.
Solubility: Examples 35-38.
Shampoo Formulations: Examples 39-40.
Mouthwash Formulations: Examples 41-42.
Coatings: Examples 57.
Inks: Examples 19.
Further Ink Jet Media: Examples 33.
Further Shampoo Formulations: Examples 43.
Body care products, household products, textile and fabrics: Examples 44-46. Polymer Formulations (compositions, fibers, plaques, combination with flame retardants, etc.): Examples 47-65.
Further Coatings: Examples 56.
Photographic Compositions: Examples 58-59.
Photocured Coatings: Examples 63.

**Example 1 (comparative)**

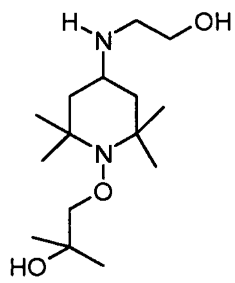

Example 32 (8.4 g, 0.035 mole), ethanolamine (3 g, 0.05 mole), and catalyst (10% Pd on C, 2 g, Engelhard Corp.) are added to 10 mL of isopropanol in 100 mL of ethanol. The reactor is pressured to 45 psig with hydrogen while heating to 60C. After a reaction time of two hours, the reactor is vented and catalyst is removed by filtration. The solvent is distilled off and the crude product is crystallized from 50 mL of ethyl acetate. The title compound is obtained (5.23 g, 52% yield) as clear white plates with a melting point of 130-132C whose structure is consistent with HNMR.

**Example 2 (intermediate)**

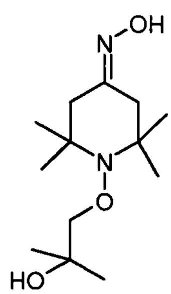

Example 32 (3.36 g, 0.14 mole) and hydroxylamine hydrochloride (1.39 g, 0.02 mole) are dissolved in a solution of 50 mL of water and 50 mL of ethanol. The solution is neutralized to pH = 10 with 2N aqueous sodium hydroxide solution. The solution is refluxed for 5 hours after which the solvent is distilled off and the residue is crystallized from ethyl acetate. After drying to constant weight, the title compound is obtained (3.11 g, 86% yield) as a white solid with a melting point of 131-134C whose structure is consistent with HNMR.

**Example 3 (intermediate)**

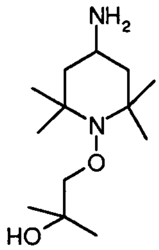

Example 2 (2.44 g, 0.0094 mole) is added to 50 mL of absolute ethanol. Sodium spheres (4 g, 0.17 mole) are added portionwise over thirty minutes after which the mixture is refluxed for one hour. The solution is poured into 200 mL of water and extracted thrice with 100 mL of methylene chloride. The combined organic layer is washed twice with 25 mL of water, dried over magnesium sulfate, and the solvent is distilled off. The title compound is obtained (2.3 g, 100% yield) as a white solid with a melting point of 110-114C whose structure is consistent with HNMR.

**Example 4**

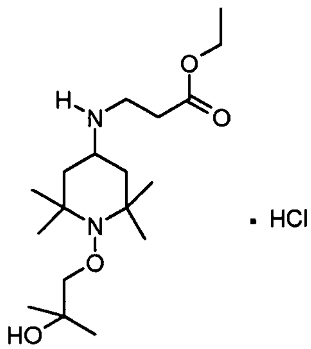

Example 32 (5.28 g, 0.022 mole), beta-alanine ethylester hydrochloride (3.84 g, 0.025mole), and catalyst ($PtO_2$, 1.0 g, Engelhard Corp.) are added to 75 mL of absolute ethanol. The reactor is pressured up to 45 psig with hydrogen while heating to 60C. After four hours, the reactor is vented and the catalyst is removed by filtration. Sixty-five mL of ethanol was distilled yielding a precipitate in the remaining ethanol. After filtration, the precipitate is recrystallized from methanol. The title compound is obtained as a white solid with a melting point of 215-220C whose structure is consistent with HNMR.

**Example 5**

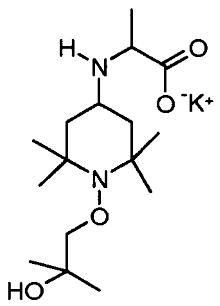

Example 32 (5.28 g, 0.022 mole), l-alanine (2.23 g, 0.025mole), potassium hydroxide (1.4 g, 0.025 mole), and catalyst ($PtO_2$, 0.5 g, Engelhard Corp.) are added to 50 mL of absolute methanol. The reactor is pressured up to 45 psig with hydrogen while heating to 60C. After four hours, the reactor is vented and the catalyst is removed by filtration. The solvent is removed by distillation and the residue is dried in vacuo, which crystallizes during drying. The title compound is obtained (7 g, 100% yield) as a white glassy solid with a melting point of 65-70C whose structure is consistent with HNMR.

**Example 6**

$CH_3COOH$ •

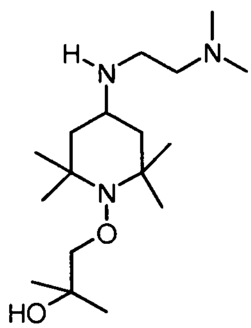

Example 32 (5.28 g, 0.022 mole), N,N-ethylenediamine (2.2 g, 0.025mole), and catalyst ($PtO_2$, 0.5 g, Engelhard Corp.) are added to 75 mL of absolute methanol. The reactor is pressured up to 45 psig with hydrogen while heating to 60C. After one hour, the reactor is vented and the catalyst is removed by filtration. The solvent is removed by distillation and the residue is dissolved in 10 mL of acetonitrile and titrated with 1.5 g of glacial acetic acid. The solvent is removed by distillation and the residue is dried overnight in a vacuum oven. The title compound is obtained (7.69 g) as a white solid with a melting point of 72-74C whose structure is consistent with HNMR.

**Example 7**

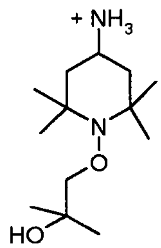

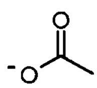

Example 3 (2.55 g, 0.01 mole) is dissolved in 100 mL of diethylether. To this solution is added 0.56 g of glacial acetic acid. A solid, which forms immediately, is filtered off, washed with diethylether, and dried to constant weight in a vacuum oven. The title compound is obtained (1.7 g, 56% yield) as a white solid with a melting point of 200-202C whose structure is consistent with HNMR.

**Example 8**

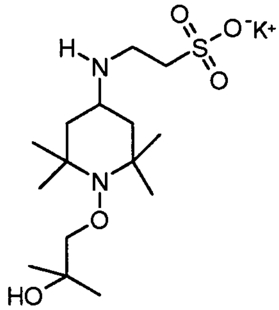

Example 32 (5.28 g, 0.022 mole), taurine (3.12 g, 0.025 mole), and catalyst ($PtO_2$, 0.5 g, Engelhard Corp.) are added to 50 mL of methanol and 25 mL of 1 M methanolic potassium hydroxide. The reactor is pressured to 45 psig with hydrogen while heating to 60C. After 4 hours, the reactor is vented and the catalyst is filtered off. The filtrate is subjected to vacuum distillation yielding a clear residue that solidifies upon standing. The title compound is obtained (7.7 g, 89.5% yield) as a glassy white solid with a melting point of 158-162C whose structure is consistent with HNMR.

**Example 9**

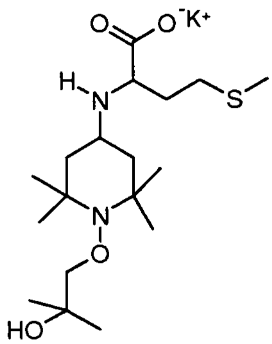

Example 32 (5.28 g, 0.022 mole), methionine (3.72 g, 0.025 mole), and catalyst ($PtO_2$, 0.5 g, Engelhard Corp.) are added to 50 mL of methanol and 25 mL of 1 M methanolic potassium hydroxide. The reactor is pressured to 45 psig with hydrogen while heating to 60C. After 3 hours, the reactor is vented and the catalyst is filtered off. The filtrate is subjected to vacuum distillation yielding a clear residue that solidifies upon standing. The title compound is obtained (9.45 g) as a white solid with a melting point of 108-112C whose structure is consistent with HNMR.

**Example 10**

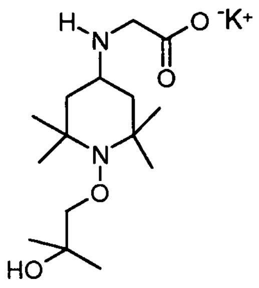

Example 32 (5.28 g, 0.022 mole), glycine (1.88 g, 0.025 mole), and catalyst ($PtO_2$ 0.5 g, Engelhard Corp.) are added to 50 mL of methanol and 25 mL of 1 M methanolic potassium hydroxide. The reactor is pressured to 45 psig with hydrogen while heating to 60C. After 2 hours, the reactor is vented and the catalyst is filtered off. The filtrate is subjected to vacuum distillation yielding a clear residue that solidifies upon standing. The title compound is obtained (7.87 g) as a white solid with a melting point of 94-98C whose structure is consistent with HNMR.

**Example 11 (comparative)**

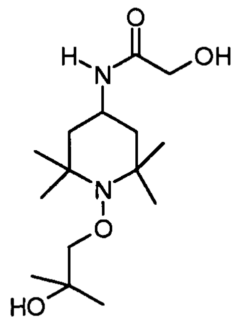

Example 3 (6.11 g, 0.025 mole) and methyl glycolate (4.5 g, 0.05 mole) are added to 75 mL of xylene. Under a nitrogen atmosphere, the solution is heated to 120C and held there for 18 hours. The solvent is removed by distillation and the residue is chromatographed on silica gel using a hexane:ethyl acetate gradient. The appropriate fractions are combined and the solvent is removed by distillation yielding a clear residue that solidifies upon standing. The title compound is obtained (1.51 g, 20% yield) as a white solid with a melting point of 153-154C whose structure is consistent with HNMR.

**Example 12 (comparative)**

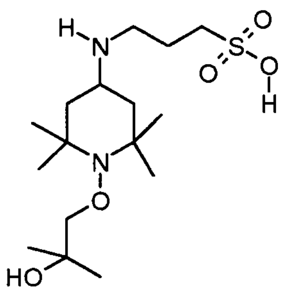

Synthetic Reference: I. Ismail, *J. Serb. Chem. Soc. 57(7),* 415-420 (**1992**)

Example 3 (4.88 g, 0.02 mole) and 1,1-propanesultone (2.44 g, 0.02 mole) are added to 60 mL of 2-butanol.

Under a nitrogen atmosphere, the solution is heated to reflux for 30 minutes. The precipitate is filtered at ambient temperature, washed with 2-butanol, and dried to constant weight in a vacuum oven. The title compound is obtained (4.45 g, 60.5% yield) as a white solid that decomposes upon melting at 290C whose structure is consistent with HNMR.

**Example 13 (comparative)**

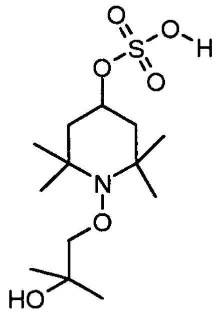

4-Hydroxy-1-(2-hydroxy-2-methylpropoxy)-2,2,6,6-tetramethylpiperidine (4.9 g, 0.02 mole) is dissolved in 200 mL of 1,2-dichloroethane and cooled to 5C. To this solution is added drop wise chlorosulfonic acid (1.32 mL, 0.02 mole) dissolved in 25 mL of 1,2-dichloroethane. The solution is left stirring overnight allowing the temperature to rise to ambient temperature. The solvent is removed by distillation and replaced with ethanol. The solution is clarified and the ethanol is removed by distillation. The title compound is obtained as a viscous clear resin whose structure is consistent with HNMR.

Analysis:

HNMR ($CD_3OD$): $\delta$ 1.29 (s, 6H), 1.56 (s, 6H), 1.57 (s, 6H), 1.82 (dd, 2H), 2.16 (ddd, 2H), 4.16 (tt, 1H), 4.17 (s, 2H)

**Example 14 (comparative)**

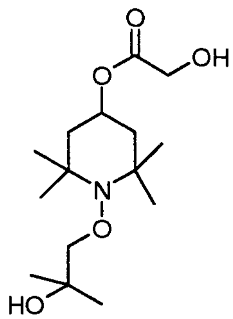

4-Hydroxy-1-(2-hydroxy-2-methylpropoxy)-2,2,6,6-tetramethylpiperidine (12.25 g, 0.05 mole), methyl glycolate (6.75 g, 0.075 mole), and Tyzor TBT (0.5 mL, 0.0013 mole) are dissolved in 250 mL of dry toluene. The solution is heated to reflux and allowed to reflux for 5 hours. The solution is cooled to 100C at which time 5 mL of water is added. The water is removed, the toluene layer is clarified, and the toluene is removed by distillation. The title compound is obtained (17.56 g) as a light orange oil with an assay of 87% as judged by gas chromatography whose structure is consistent with HNMR.

**Example 15 (comparative)**

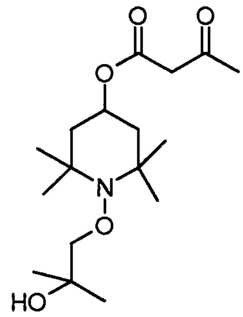

4-Hydroxy-1-(2-hydroxy-2-methylpropoxy)-2,2,6,6-tetramethylpiperidine (12.25 g, 0.05 mole), ethylacetoacetate (7.65 mL, 0.06 mole), and lithium t-butoxide (0.5 g, 0.006 mole) are dissolved in 250 mL of dry toluene. The solution is heated to reflux using a Dean Stark trap and allowed to reflux for 6 hours. The solution is cooled to 100C at which time 5 mL of water is added to destroy the catalyst. The water is removed, the toluene layer is clarified, and the toluene is removed by distillation. The title compound is obtained (16.7 g) as a light orange oil whose structure is consistent with HNMR.

**Example 16 (comparative)**

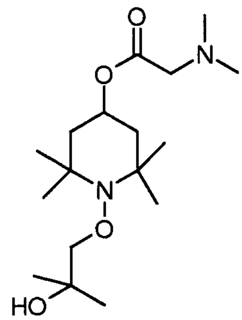

4-Hydroxy-1-(2-hydroxy-2-methylpropoxy)-2,2,6,6-tetramethylpiperidine (12.25 g, 0.05 mole), N,N-dimethylglycine ethyl ester (13 mL, 0.09 mole), and lithium t-butoxide (0.2 g, 0.0025 mole) are dissolved in 150 mL of dry toluene. The solution is heated to reflux using a Dean Stark trap and allowed to reflux for 5 hours. The solution is cooled to 100C at which time 5 mL of water is added to destroy the catalyst. The water is removed, the toluene layer is clarified, and the toluene is removed by distillation. The title compound is obtained (15.58 g, 94% yield) as a light yellow-orange oil with an assay of 93.2% as judged by gas chromatography whose structure is consistent with HNMR.

**Example 17**

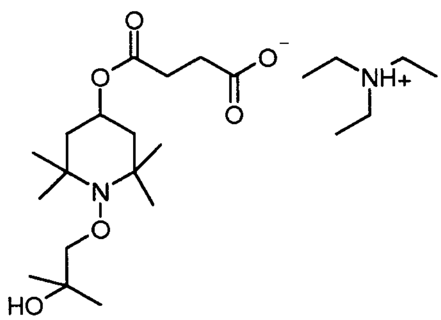

4-Hydroxy-1-(2-hydroxy-2-methylpropoxy)-2,2,6,6-tetramethylpiperidine (12.25 g, 0.05 mole), succinic acid monomethylester (6.6 g, 0.05 mole), triethylamine (5.05 g, 0.05 mole), and lithium t-butoxide (0.4 g, 0.005 mole) are dissolved in 200 mL of dry toluene. The solution is heated to reflux using a Dean Stark trap and allowed to reflux for 8 hours. The solution is cooled to 100C at which time 5 mL of water is added to destroy the catalyst. The water is removed, the toluene layer is clarified, and the toluene is removed by distillation. The title compound is obtained as a light yellow-orange oil (21.9 g) with an assay of 21% (remaining material is unreacted starting hindered amine) as judged by gas chromatography whose structure is consistent with HNMR.

**Example 18 (comparative)**

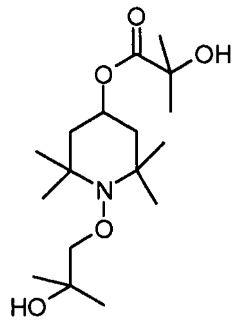

4-Hydroxy-1-(2-hydroxy-2-methylpropoxy)-2,2,6,6-tetramethylpiperidine (12.25 g, 0.05 mole), methyl 4-hydroxyisobutyrate (7.08 g, 0.06 mole), and titanium isopropoxide (0.75 mL, 0.0025 mole) are dissolved in 250 mL of dry toluene. The solution is heated to reflux using a Dean Stark trap and allowed to reflux for 18 hours. The solution is cooled to 100C at which time 5 mL of water is added to destroy the catalyst. The water is removed, the toluene layer is clarified, and the toluene is removed by distillation. The title compound is obtained (21.41 g) as a light-yellow oil with an assay of 79% as judged by gas chromatography whose structure is consistent with HNMR.

**Example 19**: Ink Jet Ink

Magenta and Yellow inks are extracted from an Hewlett-Packard three-color cartridge (HP C1823D). The stabilizer is weighed in an amount of 0.15 g into a test tube and dissolved in 2.85 g of either the magenta or yellow ink. The obtained ink is filtered and transferred into an emptied and carefully cleaned cartridge of a Deskjet 510 printer (Hewlett-Packard). A stepped image is then printed onto plain paper (sihl+eika) or, alternatively, onto Premium Photo paper from Hewlett-Packard (item code C6040A). The produced print is left to dry at 50°C under vacuum for two hours and thereafter irradiated behind a 5 mm thick window glass in an Atlas Ci-35 light fading device equipped with a Xenon lamp. The Atlas device is operated at 43°C, 50%RH without dark cycles and the light intensity is 461 W/m$^2$ (300-800 nm). The color density of each step is measured before and after exposure using a MacBeth TR 924 densitometer. The compounds according to this invention are able to improve the light fastness of the magenta and yellow prints.

**Example 20**

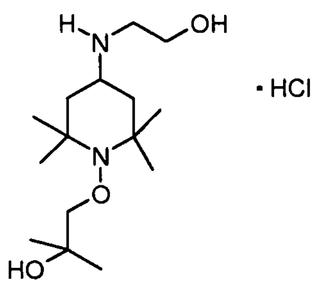

Example 1 (12.02 g as crude product, 0.042 mole) is dissolved in a mixture of diethylether and ethanol. With vigorous agitation, hydrochloric acid gas is introduced subsurface to the solution. A white insoluble solid is formed which is filtered off and dried in a vacuum oven until constant weight is reached. The title compound is obtained (2.16 g, 16% yield) as a white solid with a melting point of 217-233C whose structure is consistent with HNMR.

**Example 21: Ink Jet Media**

A resin-coated paper impregnated with inorganic adsorbent particles (Konica QP Photoglossy ink jet paper, Konica Corp.) is purchased. On the ink-receiving layer, a 0.8 wt % methanol solution, unless stated otherwise, of the instant compounds is applied in an amount to achieve 650-700 mg/m$^2$. The paper is allowed to dry under ambient temperature and pressure for 24 hours. Separately, test patterns (cyan) are printed on the treated sheets using a Hewlett Packard DeskJet 970 Cxi printer at 100% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting. The change in color is given by Delta E (DE) which is calculated by:

$$DE = [(DL^*)^2 + (Da^*)^2 + (Db^*)^2]^{1/2}$$

| Stabilizer | DE for cyan after 15 weeks |
|---|---|
| None | 4.29 |
| Example 11/DABCO●HCl | 4.08 (comparative) |
| Example 6/DABCO | 3.09 |

DABCO●HCl is 1,4-diazabicyclo[2.2.2]octane hydrochloride salt.
DABCO is 1,4-diazabicyclo[2.2.2]octane.
Mixtures of stabilizers are in a 1:1 by weight ratio with the total stabilizer concentration added of 650-700 mg/m2.
As the above data show, compounds according to this invention improve the light fastness of ink jet prints.

**Example 22**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/m$^2$. The paper is allowed to dry under ambient temperature and pressure for 24 hours. Separately, test patterns (magenta) are printed on the treated sheets using a Hewlett Packard DeskJet 970 Cxi printer at 100% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for magenta after 15 weeks |
|---|---|
| None | 5.49 |
| Example 11/DABCO●HCl | 4.79 (comparative) |
| Example 6/DABCO | 2.23 |

DABCO●HCl is 1,4-diazabicyclo[2.2.2]octane hydrochloride salt.
DABCO is 1,4-diazabicyclo[2.2.2]octane.
Mixtures of stabilizers are in a 1:1 by weight ratio with the total stabilizer concentration added of 650-700 mg/m2.
As the above data show, compounds according to this invention improve the light fastness of ink jet prints.

**Example 23**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/m$^2$. The paper is allowed to dry under ambient temperature and pressure for 24

hours. Separately, test patterns (yellow) are printed on the treated sheets using a Hewlett Packard DeskJet 970 Cxi printer at 100% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for yellow after 15 weeks |
|---|---|
| None | 2.48 |
| Example 11/DABCO●HCl | 2.35 (comparative) |
| Example 6/DABCO | 1.73 |

DABCO●HCl is 1,4-diazabicyclo[2.2.2]octane hydrochloride salt.
DABCO is 1,4-diazabicyclo[2.2.2]octane.
Mixtures of stabilizers are in a 1:1 by weight ratio with the total stabilizer concentration added of 650-700 mg/m2.
As the above data show, compounds according to this invention improve the light fastness of ink jet prints.

**Example 24**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$, unless otherwise stated. The paper is allowed to dry under ambient temperature and pressure for 24 hours. Separately, test patterns (cyan) are printed on the treated sheets using a Hewlett Packard DeskJet 970 Cxi printer at 100% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabillizer | DE for cyan after 4 weeks |
|---|---|
| None | 6.19 |
| Example 20 | 5.26 |
| Example 2 | 4.69 (comparative) |
| Example 20 (2x) | 4.62 |
| Example 7 | 4.27 |
| Example 1 | 4.21 (comparative) |

Example 20 (2x) is done at a concentration of 1300 to 1400 mg/m2.
As the above data show, compounds according to this invention improve the light fastness of ink jet prints.

**Example 25**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$, unless otherwise stated. The paper is allowed to dry under ambient temperature and pressure for 24 hours. Separately, test patterns (magenta) are printed on the treated sheets using a Hewlett Packard DeskJet 970 Cxi printer at 100% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for magenta after 4 weeks |
|---|---|
| None | 25.73 |
| Example 20 | 25.20 |
| Example 20 (2x) | 21.96 |
| Example 7 | 20.78 |
| Example 1 | 20.23 (comparative) |
| Example 7 (2x) | 19.52 |
| Example 7 (3x) | 12.99 |

Example 20 (2x) and Example 7 (2x) are done at a concentration of 1300 to 1400 mg/m2 while Example 7 (3x) is done at a concentration of 1950 to 2100 mg/m2.
As the above data show, compounds according to this invention improve the light fastness of ink jet prints.

**Example 26**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$, unless stated otherwise. The paper is allowed to dry under ambient temperature and pressure for 24 hours. Separately, test patterns (yellow) are printed on the treated sheets using a Hewlett Packard DeskJet 970 Cxi printer at 50% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for yellow after 4 weeks |
|---|---|
| None | 5.80 |
| Example 20 | 5.53 |
| Example 2 | 3.87 (comparative) |

As the above data show, compounds according to this invention improve the light fastness of ink jet prints.

**Example 27**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$, unless otherwise stated. The paper is allowed to dry under ambient temperature and pressure for 24 hours. Separately, test patterns (cyan) are printed on the treated sheets using a Hewlett Packard DeskJet 970 Cxi printer at 50% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for cyan after 4 weeks |
|---|---|
| None | 7.29 |
| Example 20 | 5.98 |
| Example 7 | 5.85 |
| Example 2 | 5.53 (comparative) |
| Example 1 | 5.29 (comparative) |
| Example 20 (2x) | 5.11 |
| Example 7 (2x) | 4.95 |
| Example 7 (3x) | 4.34 |

Example 20 (2x) and Example 7 (2x) are done at a concentration of 1300 to 1400 mg/m2 while Example 7 (3x) is done at a concentration of 1950 to 2100 mg/m2.
As the above data show, compounds according to this invention improve the light fastness of ink jet prints.

**Example 28**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$, unless otherwise stated. The paper is allowed to dry under ambient temperature and pressure for 24 hours. Separately, test patterns (magenta) are printed on the treated sheets using a Hewlett Packard DeskJet 970 Cxi printer at 50% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for magenta after 4 weeks |
|---|---|
| None | 20.16 |
| Example 7 | 16.62 |
| Example 7 (2x) | 14.14 |
| Example 7 (3x) | 8.60 |

Example 7 (2x) is done at a concentration of 1300 to 1400 mg/m2 while Example 7 (3x) is done at a concentration of 1950 to 2100 mg/m2.
As the above data show, compounds according to this invention improve the light fastness of ink jet prints.

**Example 29**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$, unless stated otherwise. The paper is allowed to dry under ambient temperature and pressure for 24 hours. Separately, test patterns (yellow) are printed on the treated sheets using a Hewlett Packard DeskJet 970 Cxi printer at 50% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for yellow after 4 weeks |
|---|---|
| None | 4.68 |
| Example 20 | 3.15 |
| Example 2 | 1.36 (comparative) |

As the above data show, compounds according to this invention improve the light fastness of ink jet prints.

**Example 30 (comparative)**

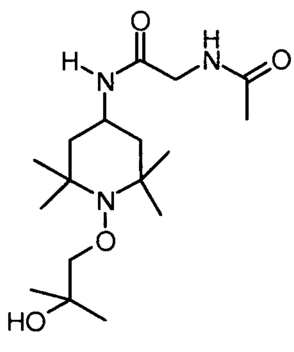

Example 3 (6.1 g, 0.025 mole) and ethyl acetamidoacetate (3.62 g, 0.025 mole) are added to 75 mL of xylene and heated to reflux. The solution is refluxed for 18 hours and then the xylene is removed by distillation. The remaining orange residue is recrystallized from methylene chloride and dried to constant weight in a vacuum oven. The title compound is obtained (2.41 g, 28% yield) as a white crystalline solid with a melting point of 77-80C whose structure is consistent with HNMR.

**Example 31 (comparative)**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds is applied in an amount to achieve 650-700 mg/$m^2$. The paper is allowed to dry under ambient temperature and pressure for 24

hours. Separately, test patterns (cyan and yellow) are printed on the treated sheets using a Hewlett Packard DeskJet 970 Cxi printer at 100% print density. The obtained prints are left to dry at ambient temperature and pressure for 24 hours. Color densities and CIEL*a*b coordinates before and after exposure are measured using X-Rite 938 Spectrodensitometer. Exposures are carried out using normal office fluorescent lighting.

| Stabilizer | DE for cyan after 3 months |
|---|---|
| None | 12.40 |
| Compound A | 11.96 |
| Example 30 | 11.39 |
| Compound A/Example 30 | 10.96 |

Compound A is N,N-dibenzylhydroxylamine hydrochloride

Mixtures of stabilizers are in a 1:1 by weight ratio with the total stabilizer concentration added of 650-700 mg/m2.

| Stabilizer | DE for yellow after 3 months |
|---|---|
| None | 6.85 |
| Compound A | 5.31 |
| Example 30 | 2.96 |
| Example 30/Compound A | 1.37 |

Compound A is N,N-dibenzylhydroxylamine hydrochloride

Mixtures of stabilizers are in a 1:1 by weight ratio with the total stabilizer concentration added of 650-700 mg/m2.

**Example 32 (intermediate)**

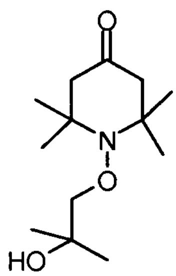

4-Hydroxy-1-(2-hydroxy-2-methylpropoxy)-2,2,6,6-tetramethylpiperidine (42.5 g, 0.17 mole), calcium hypochlorite (100g, 0.7 mole), and resin (25 g, IRA 900 resin, ACROS) are added to 600 mL of carbon tetrachloride. After heating to 40C, the slurry is stirred for seven hours and then is stirred overnight at ambient temperature. The slurry is filtered to remove resin and salts. The resulting filtrate is washed twice with water, dried over magnesium sulfate and the solvent removed by distillation. The title compound is obtained (37.1 g, 88% yield) as a white solid with a melting point of 58-61 C whose structure is consistent with HNMR.

**Example 33 Ink Jet Media**

A resin-coated paper impregnated with inorganic adsorbent particles (Konica QP Photoglossy ink jet paper, Konica Corp.) is purchased. On the ink-receiving layer, a 0.8 wt % methanol solution of the instant compounds and 2-(2-hydroxy-3-tert-butyl-5-methylphenyl)-2H-benzotriazole-5-sulfonic acid, sodium salt, a benzotriazole based UV absorber, is applied in an amount to achieve 650-700 mg/m$^2$. The UV absorber and the instant compounds are in a 2:1 ratio by weight. The paper is allowed to dry under ambient temperature and pressure for 24 hours. Separately, test patterns (cyan, magenta and yellow) are printed on the treated sheets using an Epson printer at 100% print density. The obtained prints are left to dry at ambient temperature and pressure for 24 hours. Color densities and CIEL*a*b coordinates before

and after exposure are measured using X-Rite 938 Spectrodensitometer. Exposures are carried out using normal office fluorescent lighting. The compounds according to this invention -improve the light fastness of ink jet prints.

**Example 34**

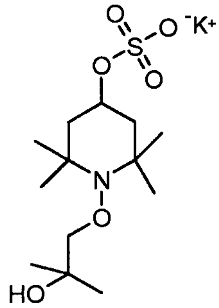

Example 13 (0.304 g, 0.001 mole) is dissolved in 2 mL of absolute ethanol. Potassium hydroxide (0.05 g, 0.001 mole) is added to the solution at which time a precipitate is formed. The precipitate is filtered and dried to constant weight in a vacuum oven. The title compound is obtained as a waxy white solid with a melting point of 115-120C whose structure is consistent with HNMR.

**Example 35**

The solubility of the instant compounds is evaluated in polar solvent systems. A solution of 50% butyl carbitol by weight and 50% water by weight is prepared. The instant compounds are added to this solution, stirred for 30 minutes, and sampled for analysis. Samples containing bis(1-octyloxy-2,2,6,6-tetramethylpiperidin-4-yl) sebacate and 4-hydroxy-1-(2-hydroxy-2-methylpropoxy)-2,2,6,6-tetramethylpiperidine are filtered to remove any undissolved residue and all samples are analyzed by high pressure liquid chromatography or gas chromatography.

| Compound | Solubility (% by weight) |
|---|---|
| HALS A | 0.2 |
| HALS B | 13.4 |
| Example 5 | ≥15 |
| Example 10 | ≥20 |

HALS A is bis(1-octyloxy-2,2,6,6-tetramethylpiperidin-4-yl) sebacate; HALS B is 4-hydroxy-1-(2-hydroxy-2-methylpropoxy)-2,2,6,6-tetramethylpiperidine.
This demonstrates that the instant compounds are soluble in highly polar solvents.

**Example 36**

The solubility of the instant compounds is evaluated in polar solvent systems. A solution of 50% butyl cellusolve by weight and 50% water by weight is prepared. The instant compounds are added to this solution, stirred for 30 minutes, and sampled for analysis. Samples containing bis(1-octyloxy-2,2,6,6-tetramethylpiperidin-4-yl) sebacate and 4-hydroxy-1-(2-hydroxy-2-methylpropoxy)-2,2,6,6-tetramethylpiperidine are filtered to remove any undissolved residue and all samples are analyzed by high pressure liquid chromatography or gas chromatography.

| Compound | Solubility (% by weight) |
|---|---|
| HALS A | 0.7 |
| HALS B | 15.5 |
| Example 5 | ≥18 |
| Example 10 | ≥20 |

HALS A is bis(1-octyloxy-2,2,6,6-tetramethylpiperidin-4-yl) sebacate; HALS B is 4-hydroxy-1-(2-hydroxy-2-methylpropoxy)-2,2,6,6-tetramethylpiperidine.
This demonstrates that the instant compounds are soluble in highly polar solvents.

**Example 37**

The solubility of the instant compounds is evaluated in polar solvent systems. A solution of 25% butyl cellusolve by weight and 75% water by weight is prepared. The instant compounds are added to this solution, stirred for 30 minutes, and sampled for analysis. Samples containing bis(1-octyloxy-2,2,6,6-tetramethylpiperidin-4-yl) sebacate and 4-hydroxy-1-(2-hydroxy-2-methylpropoxy)-2,2,6,6-tetramethylpiperidine are filtered to remove any undissolved residue and all samples are analyzed by high pressure liquid chromatography or gas chromatography.

| Compound | Solubility (% by weight) |
|---|---|
| HALS A | <0.1 |
| HALS B | 13.6 |
| Example 10 | ≥18 |

HALS A is bis(1-octyloxy-2,2,6,6-tetramethylpiperidin-4-yl) sebacate. HALS B is 4-hydroxy-1-(2-hydroxy-2-methylpropoxy)-2,2,6,6-tetramethylpiperidine.
This demonstrates that the instant compounds are soluble in highly polar solvents.

**Example 38**

The solubility of the instant compounds is evaluated in polar solvent systems. The instant compounds are added to water, stirred for 30 minutes, and sampled for analysis. Samples containing bis(1-octyloxy-2,2,6,6-tetramethylpiperidin-4-yl) sebacate and 4-hydroxy-1-(2-hydroxy-2-methylpropoxy)-2,2,6,6-tetramethylpiperidine are filtered to remove any undissolved residue and all samples are analyzed by high pressure liquid chromatography or gas chromatography.

| Compound | Solubility (% by weight) |
|---|---|
| HALS A | <0.1 |
| HALS B | 1.4 |
| Example 10 | ≥8 |

HALS A is bis(1-octyloxy-2,2,6,6-tetramethylpiperidin-4-yl) sebacate; HALS B is 4-hydroxy-1-(2-hydroxy-2-methylpropoxy)-2,2,6,6-tetramethylpiperidine.
This demonstrates that the instant compounds are soluble in highly polar solvents.

**Example 39**

The instant compounds are added to a commercial shampoo formulation and are evaluated for their ability to reduce the amount of dye fading when the samples are exposed to fluorescent lighting. The instant compounds (0.36 g) are dissolved in 5 mL of methanol which is then added to 120 g of shampoo (Suave$^R$ Natural$^R$, Fresh Mountain Strawberry) with agitation. A benzotriazole UV absorber, 2-(2-hydroxy-3-tert-butyl-5-methylphenyl)-2H-benzotriazole-5-sulfonic acid, sodium salt (UVA), is optionally added to the shampoo formulation at an equivalent concentration. The stabilized shampoo formulation is agitated for 15 minutes and put into 20 mL glass scintillation vials. These formulations are weathered under fluorescent light aging at ambient temperature. The CIEL*a*b coordinates before and after exposure are measured using X-Rite 938 Spectrodensitometer. Color change is expressed as Delta E (DE).

| Stabilizer/(Loading) | DE after 2 weeks |
|---|---|
| None | 11.74 |
| Example 17/(0.30 wt%) | 8.91 |
| Example 6/(0.30 wt%) | 7.53 |
| Example 6/UVA (0.30 wt%/0.30 wt%) | 2.33 |

The compounds according to this invention improve the light fastness of shampoo formulations.

**Example 40**

The instant compounds are added to a commercial shampoo formulation and are evaluated for their ability to reduce the amount of dye fading when the samples are exposed to fluorescent lighting. The instant compounds (0.36 g) are dissolved in 5 mL of methanol which is then added to 120 g of shampoo (Clairol$^R$ Herbal Essences Shampoo) with agitation. A benzotriazole UV absorber, 2-(2-hydroxy-3-tert-butyl-5-methylphenyl)-2H-benzotriazole-5-sulfonic acid, sodium salt (UVA), is optionally added to the shampoo formulation. The stabilized shampoo formulation is agitated for 15 minutes and put into 20 mL glass scintillation vials. These formulations are weathered under fluorescent light aging at ambient temperature. The CIEL*a*b coordinates before and after exposure are measured using X-Rite 938 Spectrodensitometer. Color change is expressed as Delta E (DE).

| Stabilizer/(Loading) | DE after 2 weeks |
|---|---|
| None | 8.87 |
| Example 7/(0.30 wt%) | 3.96 |
| Example 5/(0.30 wt%) | 1.55 |
| Example 7/UVA (0.15 wt%/0.15 wt%) | 0.86 |

The compounds according to this invention improve the light fastness of shampoo formulations.

**Example 41**

The instant compounds are added to a commercial mouthwash formulation and are evaluated for their ability to reduce the amount of dye fading when the samples are exposed to fluorescent lighting. The instant compounds (0.36 g) are dissolved in 5 mL of methanol which is then added to 120 g of mouthwash (Scope$^R$ Original Mint) with agitation. A benzotriazole UV absorber, 2-(2-hydroxy-3-tert-butyl-5-methylphenyl)-2H-benzotriazole-5-sulfonic acid, sodium salt (UVA), is optionally added to the mouthwash formulation. The stabilized mouthwash formulation is agitated for 15 minutes and put into 20 mL glass scintillation vials. These formulations are weathered under fluorescent light aging at ambient temperature. The CIEL*a*b coordinates before and after exposure are measured using X-Rite 938 Spectrodensitometer. Color change is expressed as Delta E (DE).

| Stabilizer/(Loading) | DE after 2 weeks |
|---|---|
| None | 6.15 |
| Example 18/(0.30 wt%) | 5.11 (comparative) |
| Example 8/UVA (0.15 wt%/0.15 wt%) | 4.28 |

The compounds according to this invention improve the light fastness of mouthwash formulations.

**Example 42**

The instant compounds are added to a commercial mouthwash formulation and are evaluated for their ability to reduce the amount of dye fading when the samples are exposed to fluorescent lighting. The instant compounds (0.36 g) are dissolved in 5 mL of methanol which is then added to 120 g of mouthwash (Listerine$^R$ Cool Mint) with agitation. A benzotriazole UV absorber, 2-(2-hydroxy-3-tert-butyl-5-methylphenyl)-2H-benzotriazole-5-sulfonic acid, sodium salt (UVA), is optionally added to the mouthwash formulation. The stabilized mouthwash formulation is agitated for 15 minutes and put into 20 mL glass scintillation vials. These formulations are weathered under fluorescent light aging at ambient temperature. The CIEL*a*b coordinates before and after exposure are measured using X-Rite 938 Spectrodensitometer. Color change is expressed as Delta E (DE).

| Stabilizer/(Loading) | DE after 35 days |
|---|---|
| None | 1.83 |
| Example 10/UVA (0.15 wt%/0.15 wt%) | 1.21 |

The compounds according to this invention improve the light fastness of mouthwash formulations.

**Example 43**

An aqueous based test formulation is prepared as follows:

| | |
|---|---|
| sodium laureth sulfate (30%, TEXAPON NSO, Cognis) | 30% |
| cocamidopropylbetaine (30%, DEHYTON K, Cognis) | 10% |
| colorant* | 0.001% |
| instant stabilizer | 0.05% |
| citric acid (10% aqueous solution) | to pH 6 |
| deionized water | to 100% |

*Colorant is PURICOLOR BLUE ABL9 (FD&C Blue No.1)

About 20 mL of each of the aqueous test formulations are placed in a borosilicate glass bottle. The glass bottles are exposed in an Atlas Ci-65 Xenon arc WeatherOmeter, AATCC Test Method 16. Color measurements are performed on a Hunter Ultrascan XE spectrophotometer. Delta L, a and b values are the difference between the initial values and the values at each interval. It is seen that the stabilizers of the present invention provide excellent color stability in personal care products.

**Example 44**

The components below are thoroughly mixed in the cited sequence at 50°C, a clear homogeneous solution being obtained. The UV absorber is, for example, 3-(2H-benzotriazol-2-yl)-4-hydroxy-5-(1-methylpropyl)-benzenesulfonic acid monosodium salt.

| Ingredients | (w/w) % |
|---|---|
| ethanol, 96% | 60 |
| d-limonene | 5 |
| cedrene | 1.5 |
| citronellol | 0.5 |
| savin | 0.5 |
| instant stabilizer | 0.08 |
| UV absorber | 0.1 |
| S,S-EDDS | 0.005 |
| colorant (D&C Yellow No.5) | 0.02 |
| water | ad. 100 |

Excellent results are achieved for this example of a toilet water formulation.

**Example 45**

The hydroxypropyl cellulose is first predissolved in half of the alcohol (Vortex mixer) and is charged with the aminomethylpropanol. The other components - with the exception of the acrylate resin - are dissolved in alcohol and this solution is added, with stirring, to the hydroxypropyl cellulose. Subsequently, the acrylate resin is added and stirred until completely dissolved. The UV absorber used is, for example, benzophenone-4 is 5-benzoyl-4-hydroxy-2-methoxybenzenesulfonic acid, sodium salt.

| Ingredients | (w/w) % |
|---|---|
| alcohol, anhydrous | 96.21 |
| octylacrylamide/acrylate/butylaminoethylmethacrylate copolymer | 2.52 |
| hydroxypropyl cellulose | 0.51 |
| aminomethylpropanol (95%) | 0.46 |
| instant stabilizer | 0.05 |

(continued)

| Ingredients | (w/w) % |
|---|---|
| UV absorber | 0.05 |
| perfume oil | 0.20 |

Excellent results are achieved for this example of a hair styling spray formulation.

**Example 46**

The instant stabilizers are each deposited (from water) on a dyed cotton fabric at 0.05, 0.1, 0.2, 0.5 and 1.0 percent by weight, based on the weight of the cotton. The dyed fabrics contain the following dyes at 0.05, 0.1, 0.2 and 0.5 percent by weight based on cotton. This results in 60 separate formulations for each dye listed:

Scarlet HE-3G, Crimson HE-XL, Yellow HE-6G, Red HE-XL, Blue HE-XL, Turquoise H-A, Navy HE-XL, Remazol, Red RB, Brilliant Red RBS, Orange FR, Navy CG, Turquoise G, Black B

The cotton fabrics are subjected to light exposure in an Atlas Ci-65 Xenon arc WetherOmeter or to accelerated fluorescent lighting. The present stabilizers provide outstanding color protection to the dyed fabrics. This experiment simulates dye protection achievable through deposition of the present stabilizers via treatment with, for example, stabilizer-containing laundry detergent or fabric conditioner.

**Example 47**

Molded test specimens are prepared by injection molding thermoplastic olefin (TPO) pellets containing pigments, a phosphite, a phenolic antioxidant or hydroxylamine, a metal stearate, ultraviolet light absorbers or a hindered amine stabilizer or a mixture of UV absorber and hindered amine stabilizer.
Pigmented TPO pellets are prepared from pure pigment or pigment concentrate, coadditives and commercially available TPO by mixing the components in a Superior/MPM 1" single screw extruder with a general all-purpose screw (24:1 L/D) at 400°F (200°C), cooled in a water bath and pelletized. The resulting pellets are molded into 60 mil (0.006 inch), 2"x2" plaques at about 375°F (190°C) on a BOY 30M Injection Molding Machine.
Pigmented TPO formulation composed of polypropylene blended with a rubber modifier where the rubber modifier is an in-situ reacted copolymer or blended product containing copolymers of propylene and ethylene with or without a ternary component such as ethylidene norbornene are stabilized with a base stabilization system consisting of an N,N-dialkylhydroxylamine or a hindered phenolic antioxidant with or without an organophosphorus compound.
All additive and pigment concentrations in the final formulation are expressed as weight percent based on the resin.
Formulation contained thermoplastic olefin pellets and one or more of the following components:

0.0 to 2.0% pigment,
0.0 to 50.0% talc,
0.0 to 0.1 % phosphite,
0.0 to 1.25% phenolic antioxidant,
0.0 to 0.1% hydroxylamine
0.05 to 0.10 calcium stearate,
0.0 to 1.25% UV absorber
0.0 to 1.25% hindered amine stabilizer.
The components are dry-blended in a tumble dryer prior to extrusion and molding.
Test plaques are mounted in metal frames and exposed in an Atlas Ci65 Xenon Arc Weather-Ometer at 70°C black panel temperature, 0.55 $W/m^2$ at 340 nanometers and 50% relative humidity with intermittent light/dark cycles and water spray (Society of Automotive Engineers - SAE J 1960 Test Procedure). Specimens are tested at approximately 625 kilojoule intervals by performing color measurements on an Applied Color Systems spectrophotometer by reflectance mode according to ASTM D 2244-79. Data collected include delta E, L*, a* and b* values. Gloss measurements are conducted on a BYK-Gardner Haze/Gloss Meter at 60° according to ASTM D 523.

UV Exposure Testing

Test specimens exposed to UV radiation exhibit exceptional resistance to photodegradation when stabilized

with light stabilizer systems comprising a combination of 2-(2-hydroxy-3,5-di-tert-amylphenyl)-2H-benzotriazole, an instant stabilizer and N,N',N",N"'-tetrakis(4,6-bis(butyl-(1,2,2,6,6-pentamethylpiperidin-4-yl)amino)-s-triazin-2-yl]-1,10-diamino-4,7-diazadecane. The control sample consists of a stabilizer formulation commonly used in the industry to impart UV stability. All of the samples contain a pigment, Pigment Red 177, and talc.
The test plaques described earlier contain the following (all concentrations are weight percent based on resin):

Polymer substrate is commercially available polyolefin blend POLYTROPE® TPP 518-01 supplied by A. Schulman Inc. Akron, Ohio)
Color package is 0.025% Red 3B -Pigment Red 177, C.I. #65300.
Each plaque contains:

0.2% 2-(3,5-di-t-amyl-2-hydroxyphenyl)-2H-benzotriazole;
0.1 % calcium stearate; and
15% talc.

The Control plaques additionally contain

0.1 % 50:50 blend of neopentanetetrayl tetrakis(4-hydroxy-3,5-di-tert-butylhydrocinnamate) and [tris-(2,4-di-tert-butylphenyl) phosphite;
0.2% [bis(2,2,6,6-tetramethylpiperidin-4-yl) sebacate];
0.2% [polycondensation product of 4,4'-hexamethylene-bis(amino-2,2,6,6-tetramethylpiperidine) and 2,4-dichloro-6-tert-octylamino-s-triazine].

The test plaques containing the instant stabilizers additionally each contain 0.05% N,N,-dialkylhydroxylamine;
The instant stabilizers demonstrate greatly improved gloss retention compared to the less effective control system. Resistance to color change upon UV exposure is also enhanced. Polymer blends containing an unsaturated ternary component, such as EPDM blends, are especially benefited with the more efficient instant light stabilizer systems described above.

**Example 48**

Molded test specimens are prepared by injection molding thermoplastic olefin (TPO) pellets containing the instant compounds, pigments and other coadditives as described in Example 47.
The light stable formulations are painted with one-pack paint systems and tested for TPO/paint interactions. Before painting, the test specimens are first washed in accordance with GM998-4801 and dried for 15 minutes at 200°F (94°C). Adhesion promoter is applied to the dry film thickness of 0.2-0.4 mils. The samples are dried for five minutes before a 1 K basecoat is applied to a film thickness of 1.2-1.4 mils. The painted panels are dried for three minutes, a clearcoat is then applied to a dry film thickness of 1.2-1.5 mils followed by ten minutes flash drying and a 30 minute oven bake at 250°F (121°C).
Paint adhesion is measured by Aggressive Adhesion Testing (proprietary test procedure conducted at Technical Finishing, Inc.) and Taber Scuff. Painted panels which retain greater than 80% of the paint finish are considered acceptable. After Aggressive Adhesion Testing, samples with less than 5% paint loss are deemed acceptable.
The instant compounds provide very low levels of paint loss when analyzed by the testing protocols listed above.

**Example 49**

Molded test specimens are prepared by injection molding polypropylene pellets containing pigments, a phosphite, a phenolic antioxidant or hydroxylamine, a metal stearate, ultraviolet light absorbers or a hindered amine stabilizers or a mixture of UV absorbers and hindered amine stabilizers.
Pigmented polypropylene pellets are prepared from pure pigment or pigment concentrates, stabilizers, co-additives and commercially available polypropylene by mixing the components in a Superior/MPM 1" single screw extruder with a general all-purpose screw (24:1 UD) at 475°F (250°C), cooled in a water bath and pelletized. The resulting pellets are molded into 60 mil (0.06 inch thick) 2"x2" plaques at about 475°F (250°C) on a BOY 30M Injection Molding Machine.
Pigmented polypropylene formulations composed of polypropylene homopolymer or polypropylene copolymer are stabilized with a base stabilization system consisting of an N,N-dialkylhydroxylamine or a hindered phenolic antioxidant with or without an organophosphorous compound.
All additive and pigment concentrations in the final formulations are expressed as weight percent based on the resin. Formulations contained polypropylene pellets and one or more of the following components; 0.0% - 2.0% pigment,

0.0% - 50.0% talc,
0.0% - 50.0% calcium carbonate,
0.0% - 0.1% phosphite,
0.0% - 1.25% phenolic antioxidant,
0.0% - 0.1% hydroxylamine,
0.05% - 0.10% calcium stearate,
0.0% - 1.25% UV absorber,
0.0% - 1.25% hindered amine stabilizer.
The components are dry blended in a tumble dryer prior to extrusion and molding.

Test plaques are mounted in metal frames and exposed in an Atlas Ci65 Xenon Arc Weather-o-meter at 70°C black panel temperature, 0.55 $W/m^2$ at 340 nanometers and 50% relative humidity with intermittent light/dark cycles and water spray (Society of Automotive Engineers - SAE J 1960 Test Procedure). Specimens are tested at approximately 625 kilojoule intervals by performing color measurements on an Applied Color Systems spectrophotometer by reflectance mode according to ASTM D 2244-79. Data collected included delta E, L*, a* and b* values. Gloss measurements are conducted on a BYK-GARDNER Haze/Gloss Meter at 60° according to ASTM D523.

UV Exposure Testing

Test specimens exposed to UV radiation exhibit exceptional resistance to photodegradation when stabilized with light stabilizer systems comprised of a combination of 2-(3,5-di-t-amyl-2-hydroxyphenyl)-2H-benzotriazole, an instant stabilizer, and an oligomeric hindered amine. The oligomeric hindered amine is oligomer of N-{[2-(N-2,2,6,6-tetramethylpiperidin-4-yl)butylamino]-s-triazin-4-yl}-N,N'-bis(2,2,6,6-tetramethylpiperidin-4-yl)-1,6-hexanediamine terminated with 2,4-bis(dibutylamino)-s-triazin-6-yl. The Control sample consists of a stabilizer formulation commonly used in the industry to impart UV stability. All of the samples contain Pigment Red 177.

- All formulations are base stabilized with 0.05% dialklyhydroxylamine in the final resin formulation.
- Polymer substrate is a commercially available polypropylene homopolymer - Profax 6501 (commercial supplier Montell Polyolefins).
- Color package is 0.25% Red 3B - Pigment Red 177, C.I. # 65300 in the final resin formulation.
- Each formulation contains a hydroxyphenyl benzotriazole UV absorber - 2-(2-hydroxy-3,5-di-tert-amylphenyl)-2H-benzotriazole.
- Each formulation contains 0.1 % calcium stearate.
- Samples are 60 mil thick 2" x 2" injection molded plaques.
- UV exposures conducted under SAE J 1960 - Exterior Automotive conditions.

All additive and pigment concentrations in the final formulations are expressed as weight percent on the resin.
The light stabilized formulations show much greater resistance to photodegradation than unstabilized specimens which fail quickly under the UV exposure conditions outlined above.

**Example 50**

Film grade polyethylene is dry blended with approximately 10.0% by weight of the test additives and then melt compounded at 200°C into "Masterbatch" pellets. The fully formulated "Masterbatch" pellets are dry blended with polyethylene resin to get the desired final stabilizer concentrations. Typical formulations contain the instant compounds at levels from 0.05% to 2.0%, a metal stearate such as calcium stearate at 0.05% to 0.5%, a phosphite at 0% to 0.1%, a phenolic antioxidant at 0% to 1.25%, an N,N-dialkylhydroxylamine at 0% to 0.1% and optionally a hindered amine at 0% to 2.0%. This stabilized fully formulated resin is then blown at 200°C into a 150 micron thick film on a DOLCI film line. The blown films are exposed in an Atlas Xenon-Arc WeatherOmeter according to ASTM G26 at 63°C bpt, 0.35 $W/m^2$ at 340 nm with no spray cycle. Films are tested periodically for any change in elongation using an Instron 112 tensile tester. Failure in this test is determined by observation of the loss of % elongation in the film. The longer it takes for this loss to occur, the more effective is the stabilizer system.
The films containing the instant compound mixture show good light stabilizing efficacy.

**Example 51**

Film grade polyethylene is dry blended with 10% loading of the test additives, as described in Example 50, and then melt compounded at 200°C into fully formulated master batch pellets. The master batch pellets are dry blended with the polyethylene resin to get the final stabilizer concentration. The fully formulated resin is then blown at 200°C into

a 150 micron thick film using a DOLCI film line.
The resulting films are exposed on a greenhouse on galvanized iron backing. Treatment includes applications of pesticides on a regular basis (i.e. sodium N-methyldithiocarbamate, VAPAM' every six months and SESMETRIN/ every month). Performance is measured by monitoring the percent residual elongation. Failure is defined as the time to a 50% loss of original elongation.
The films containing the instant compounds show good resistance to pesticides.

**Example 52**

Greenhouse film samples are prepared as described in Example 50, but in addition to the instant compounds also contain a metal stearate or a metal oxide. Typical formulations contain from 0.05 to 2% by weight of the instant hindered amines, 0.05 to 0.5% of a metal stearate such as calcium oxide, and 0.05 to 0.5% of a metal oxide such as zinc oxide or magnesium oxide.
Effectiveness is monitored as described in Example 51. The films containing the instant compounds exhibit good light stability.

**Example 53**

Molding grade polypropylene is dry blended with test additives and then melt compounded into pellets. In addition to the instant compounds, selected flame retardants are also included. The flame retardants are tris(3-bromo-2,2-bis(bromomethyl)propyl)phosphate, decabromodiphenyl oxide, ethylene bis-(tetrabromophthalimide), or ethylene bis-(dibromonorbornanedicarboximide). The pelletized fully formulated resin is then injection molded into test specimens using a Boy 50M laboratory model injection molder.
Test plaques are mounted in metal frames and exposed in an Atlas Ci65 Xenon Arc Weather-Ometer with intermittent light/dark cycles and water spray following the ASTM G26 test procedure. Specimens are tested at periodic intervals for changes in tensile properties. Failure in this test is determined by the observation of the loss of tensile properties. The longer it takes for the loss in properties to occur, the more effective is the stabilizer system. The test samples containing the instant compounds exhibit good light stabilization properties.

**Example 54**

Resin materials of the general class known as thermoplastic elastomers, examples of which include, copolymers of styrene with butadiene or isoprene and/or ethylene-cobutylene such as SBS, SEBS and SIS, are dry blended with the instant compounds and melt compounded into pellets. Typical formulations contain the instant compounds at levels from 0.05% to 2.0%, a metal stearate such as calcium stearate at 0.05% to 0.5%, pigments from 0% to 5%, UV absorbers at levels of 0.05% to 2.0%, phosphites at 0.0% - 0.1 %, phenolic antioxidants at 0.0% - 1.25%, N,N-dialkylhydroxylamine at 0.0% - 0.1%, and optionally other hindered amine stabilizers at levels of 0.0% to 2.0%.
The pelletized fully formulated resin is then processed into a useful article such as blown or cast extrusion into film; injection molded into a molded article; thermoformed into molded articles; extruded into wire and cable housing; or rotational molded into hollow articles.
The materials containing the instant compounds exhibit stability against deleterious effects of UV light and thermal exposure.

**Example 55**

Articles prepared according to Example 54 which additionally contain an organophosphorus stabilizer selected from the group consisting of tris(2,4-di-tert-butylphenyl) phosphite, bis(2,4-di-tert-butyl-6-methylphenyl) ethyl phosphite, 2,2',2''-nitrilo[triethyl-tris-(3,3',5,5'-tetra-tert-butyl-1,1'-biphenyl-2,2'-diyl) phosphite], tetrakis(2,4-di-butylphenyl) 4,4'-biphenylenediphosphonite, tris(nonylphenyl) phosphite, bis(2,4-di-tert-butylphenyl) pentaerythrityl diphosphite, 2,2'-ethylidenebis(2,4-di-tert-butylphenyl) fluorophosphite and 2-butyl-2-ethylpropan-1,3-diyl 2,4,6-tri-tert-butylphenyl phosphite as well as the instant compounds also exhibit stability against the deleterious effects of actinic light and thermal exposure.

Example 56 Polyester Urethane Coating

The hindered amine test stabilizers are incorporated into a two-component polyester urethane coating based on a commercially available polyester polyol (DESMOPHEN® 670-80) and commercially available isocyanurate (DESMODUR® N-3390) at a level of 2% by weight based on total resin solids. The coating system is catalyzed with 0.015% dibutyl tin dilaurate based on total resin solids.

Each coating formulation is applied by draw down onto transparent glass slides approximately 4" x 6" to a film thickness of about 2 mils (0.002").
The instant compounds provide excellent solubility and compatibility in a polyester urethane coating.

**Example 57**

Formulation Reference: NeoResins, Inc., Formulation WB-201 0, Technical Brochure, August, 2000.
The following components are mixed together under good agitation:

| | |
|---|---|
| Butyl cellusolve | 5.68 parts |
| Carbitol | 4.26 parts |
| Triton X-1 00 | 0.25 parts |
| Water | 1.66 parts |
| Instant compound | 0.58 parts |

This solution is added to a 4 ounce clear glass jar containing 72.85 parts of NeoPac R-9699 under vigorous agitation and agitated for 10 minutes. Optionally, a defoamer, like Dehydran 1620 (Henkel), and a flash rusting agent, like Heiscore XAB (Cas Chem), are added. The lid is placed securely on the jar. The solidified coatings are visually observed for clarity after solidification. The development of opacity or haziness is indicative of an incompatibility between the hindered amine stabilizer and the formulated coating.

Solidified coating in jar

| Sample* | 0 days | 1 day | 19 days |
|---|---|---|---|
| A | clear | clear | clear |
| B | hazy | hazy | hazy |
| C | clear | clear | clear |
| D | clear | clear | clear |
| E | clear | clear | clear |
| F | clear | clear | clear |

*A is unstabilized.
B contains 2% by weight of bis(1-octyloxy-2,2,6,6-tetramethylpiperidin-4-yl) sebacate.
C contains 2% by weight of Instant Example 17.
D contains 2% by weight of Instant Example 7.
E contains 2% by weight of Instant Example12.
F contains 2% by weight of Instant Example 8.

These data show that the instant compounds provide excellent solubility and compatibility in a high solids water borne urethane/acrylic copolymer coating system.

**Example 58** Photographic Layers

Chromogenic photographic layers are prepared by hand-coating a gelatine emulsion containing silver bromide, yellow coupler and an additive on a polyethylene-coated paper. The composition of the layer is as given in following table, amounts are in mg/m$^2$:

| Component | Amount in the layer |
| --- | --- |
| Gelatine | 5150 |
| AgBr | 520 |
| Yellow coupler | 1.07 $mmol/m^2$ |
| Coupler solvent solv1 | 33% of the coupler weight/$m^2$ |
| Additive | 30% of the coupler weight/$m^2$ |
| Hardener ha1 | 300 |
| Surfactant su1 | 340 |

The layers are dried for 7 days in a ventilated cabinet.

The dried samples are exposed to white light through a stepwedge of 0.3 logE exposure steps. They are developed with the P94 process for negative color paper from Agfa-Gevaert, following the manufacturers' recommendations.

After exposure and processing, the remission density of the yellow dye is measured in the blue channel. The samples are then exposed in an Atlas WeatherOmeter behind a separate UV filter so as to receive 60kJ/$cm^2$ light energy. The UV filter consists of an emulsion coated onto a polyester transparent support, such that the layer contains 1g/$m^2$ of a benzotriazole UV absorber. The temperature is 43°C and the relative humidity 50%. The density loss starting from a blue-density of 1 is determined. Low $\Delta$D number are desired. The additives of the present invention improve the light stability of yellow photographic layers.

**Example 59** Photographic Layers

Chromogenic photographic layers are prepared by hand-coating a gelatine emulsion containing silver bromide, yellow coupler and an additive on a polyethylene-coated paper.
The composition of the layer is as given in following table, amounts are in mg/$m^2$:

| Component | Amount in the layer |
| --- | --- |
| Gelatine | 5150 |
| AgBr | 520 |
| Yellow coupler | 1.07 $mmol/m^2$ |
| Coupler solvent solv1 | 33% of the coupler weight/$m^2$ |
| Additive | 30% of the coupler weight/$m^2$ |
| Hardener ha1 | 300 |
| Surfactant su1 | 340 |

The layers are dried for 7 days in a ventilated cabinet.
The dried samples are exposed to white light through a stepwedge of 0.3 logE exposure steps. They are developed with the P94 process for negative colour paper from Agfa-Gevaert, following the manufacturers recommendations.
After exposure and processing, the remission density of the yellow dye is measured in the blue channel. The samples are then subjected to storage in a Weiss climatic cabinet for 28 days. The density loss starting from a blue-density of 1 is determined. Low DD numbers are desired.
The additives of the present invention improve the dark stability of yellow photographic layers.

**Example 60** Preformed Films for Lamination to Plastic Parts

The instant invention also pertains to protective and decorative films which are preformed, then applied to a substrate via a dry paint transfer process. These films consist of a single decorative layer which is applied to a carrier sheet, then laminated to a self-supporting, thermoformable backing sheet. The carrier sheet is then removed from the opposite side of the film, exposing the decorative layer. The composite film/backing sheet then is thermoformed to a three-dimensional shape. Additionally, these films may also consist of multiple layers, where, for example, a thermoplastic,

thermoformable clearcoat is applied to the carrier sheet, then hardened to form an optically clear film. A color coat is then applied to the exposed face of the clearcoat, and hardened, resulting in a clear coat/color coat paint film supported by the carrier. This composite is then laminated to a thermoformable backing sheet. The carrier sheet is removed, as above, and the composite clearcoat/colorcoat/backing is then thermoformed.

The polymeric resins for the above application must be thermoplastic, and may be fluoropolymer/acrylic blends.

**Example 61** Coextrusion over Polycarbonate

A sheet composition suitable for use in weatherable glazing is prepared by coextrusion of a 0.010" thick light stabilized PMMA layer ("cap layer") over a 0.100" thick polycarbonate bulk substrate ("bulk layer"). Composition of the layers is given in the table below.

| | |
|---|---|
| Cap Layer | 100.00 phr PMMA<br>0.10 phr process stabilizer<br>0.25 phr Instant Compound<br>3.50 phr HPT UV absorber |
| Bulk Layer | 100.00 phr Polycarbonate (e.g. LEXAN® 141 from GE)<br>0.08 phr phosphite process stabilizer<br>0.10 phr BZT UV absorber |

The weatherability of the sheets of the present invention, containing the instant hindered amines is superior to a control sheet prepared without the use of NOR HALS in the cap layer. Coextruded sheets are also prepared, replacing HPT UV absorber in the cap layer with each of 2,2'-methylene-bis(4-t-octyl-(6-2H-benzotriazol-2-yl)phenol) and 2-(2-hydroxy-3,5-di-alphacumylphenyl)-2H-benzotriazole. Excellent results are achieved for these coextruded sheets containing the instant hindered amines.

BZT UV absorber is 2-(2-hydroxy-3,5-di-alpha-cumylphenyl)-2H-benzotriazole, HPT UV absorber is 4,6-diphenyl-2-(4-hexyloxy-2-hydroxyphenyl)-s-triazine. Phosphite stabilizer is tris(2,4-di-tert-butylphenyl) phosphite. Process stabilizer is a 80:20 blend of tris(2,4-di-tert-butylphenyl) phosphite and β-(3,5-di-tert-butyl-4-hydroxyphenyl)propionic acid octadecanoic ester.

**Example 62** Multilayer Polymer Structures

The instant compounds have utility as light stabilizers to protect light sensitive polymers present in multilayer polymer structures. Examples of such polymer structures include but are not limited to:

1.) Sheets and signs as seen in WO97/42261; and United States Patent No. 5,387,458 which are incorporated herein by reference;
2.) Solar Control Films of Various Construction as seen in United States Patent Nos. 3,290,203, 3,681,179, 3,776,805 and 4,095,013, incorporated herein by reference; and
3.) Base stock or cap stock for coextrusion structures such as window profiles, laminates over automotive bumpers or auto exterior panels.

Window profiles include photosensitive polymers such as ABS, ASA, SAN or vinylogous polymers such as PVC. Automotive polymeric materials which are photosensitive include for example ABS, SAN, ASA and polycarbonate as well as blends such as PC/ABS, which include Pulse® from Dow, Cycoloy® from GE, Bayblend® from Bayer, PC/PBT known as Xenoy® from GE, PC/ASA such as Geloy® from GE, and the "W-4" polymer as disclosed by General Electric Company (Modern Plastics May 2000 pages 90-91).

The instant hindered amines of this disclosure act to protect against photolytic degradation of a polymer component, or an incorporated pigment, dye colorant, or protect adhesive or "tie-layers" in such constructions.

A multilayer polymer composite is prepared by different routes, such as co-extrusion of one or more polymer compositions to form the multilayer composite. Alternatively, compression molding or thermoforming of one or polymer compositions produces the desired polymer composite. In particular, these techniques are used in the manufacture of signage, typically composed of one or more layers of polymeric materials formed on top of a base material (metal sheet, plastic, etc).

Examples of potential polymeric materials that may comprise one or more sections of the laminate, sign, sheet or composite structure may include:

polycarbonate
polyesters such as PET, PBT, PEN, PTT
acrylics such as PMMA and acrylate copolymer or terpolymers polyolefins
vinylogous polymers and copolymers composed of vinyl chloride, vinyl acetate, vinylidene chloride, vinylidene fluoride.

The present hindered amines each provide excellent stabilization to such constructions.

**Example 63** Photo-Cured White Pigmented Coating

A model white UV-curable coating for wood is prepared based on polyester acrylate chemistry with a titanium dioxide level of 25 % by weight. A base formulation is prepared which is complete excluding a photoinitiator hindered amine of component (b):

Rutile $TiO_2$, 100.0 g
Ebercryl® 830, 240.0 g
HDODA, 42 g
TMPTA, 18.0 g

The $TiO_2$ is added as a 63 % dispersion in a portion of the Ebercryl® 830. Ebercryl® 830 is a hexafunctional polyester acrylate oligomer. HDODA is 1,6-hexanediol diacrylate. TMPTA is trimethylolpropane triacrylate. The Ebecryl® products as well as the acrylate monomers are available from UCB Chemicals Corp., Smyrna, GA.
To a portion of the base formulation is added a photoinitiator mixture of bis(2,4,6-trimethylbenzoyl)phenylphosphine oxide /1-hydroxycyclohexylphenylketone in a 1:2 ratio.
The photoinitiator mixture is 3.0 weight percent of the total formulation.
The formulations further contain 1 % each of the instant hindered amines of the present disclosure.
Eight replicate prints of each formulation are prepared. Films are prepared with a draw-down bar over a white Scotchcal® vinyl film from 3M. Samples are cured with a moving belt at 58 feet/min. under two medium pressure mercury lamps perpendicular to the belts @ 300 watts/in. each. The prints received one pass under the lamps. Irradiance received is 618 $mJ/cm^2$. Final cured thickness is 2.1 mils (53 microns).
The present hindered amines provide excellent stabilization to the photo-cured coatings.

## Claims

1. A water compatible or water soluble sterically hindered hydroxy substituted alkoxyamine compound selected from the group consisting of compounds of formulae (1) to (5)

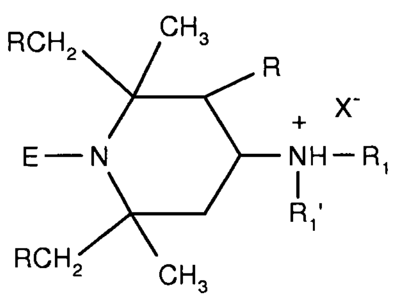

(1)

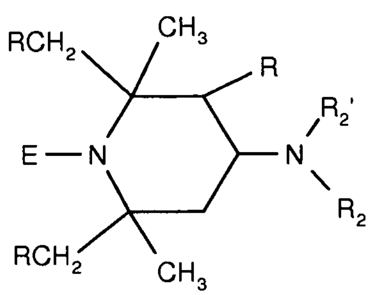

(2)

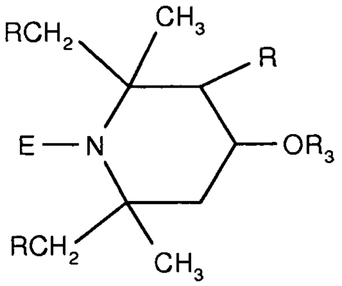

(3)

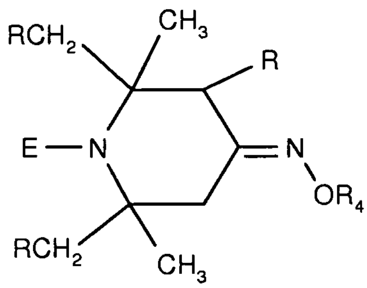

(4)

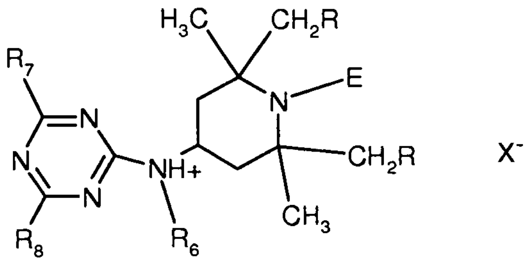

(5)

where

E is $-O-T-(OH)_b$,

T is a straight or branched chain alkylene of 1 to 18 carbon atoms, cycloalkylene of 5 to 18 carbon atoms, cycloalkenylene of 5 to 18 carbon atoms, a straight or branched chain alkylene of 1 to 4 carbon atoms substituted by phenyl or by phenyl substituted by one or two alkyl groups of 1 to 4 carbon atoms;

b is 1, 2 or 3 with the proviso that b cannot exceed the number of carbon atoms in T, and when b is 2 or 3, each hydroxyl group is attached to a different carbon atoms of T;

R is hydrogen or methyl,

$R_1$ is hydrogen, $C_1$-$C_{12}$alkyl, $C_5$-$C_8$cycloalkyl, $C_5$-$C_8$cycloalkyl substituted by one to three $C_1$-$C_4$alkyl, $C_2$-$C_{12}$alkenyl, phenyl, $C_7$-$C_9$phenylalkyl, glycidyl, $C_2$-$C_{12}$alkanoyl, $C_6$-$C_9$cycloalkylcarbonyl, $C_2$-$C_{12}$carbamoyl, $C_2$-$C_{12}$alkenoyl, benzoyl, benzoyl substituted by one to three $C_1$-$C_4$alkyl, $C_2$-$C_{12}$alkanoyl substituted by a di ($C_1$-$C_6$alkyl) phosphonate,

or $R_1$ is $C_2$-$C_{12}$alkyl, $C_2$-$C_{12}$alkanoyl or $C_7$-$C_{18}$phenylalkyl, each interrupted by one to six oxygen, sulfur or -N ($R_6$)- groups; $C_1$-$C_{12}$alkyl, $C_2$-$C_{12}$alkanoyl, phenyl or $C_7$-$C_{18}$phenylalkyl, each substituted by one to six hydroxy groups or by one to six $-NHR_6$ groups; $C_2$-$C_{12}$alkyl, $C_2$-$C_{12}$alkanoyl or $C_7$-$C_{18}$phenylalkyl, each interrupted by one to three $-NR_6C(O)$-groups; or $C_1$-$C_{12}$alkyl, $C_2$-$C_{12}$alkanoyl, phenyl or $C_7$-$C_{18}$-phenylalkyl, each substituted by one to three $-SO_3H$ groups or by one to three $-COOR_6$ groups; or

$R_1$ is said alkyl substituted by a piperazine or by a morpholine group; or

$R_1$ is said interrupted group further substituted by one to six hydroxy groups or by one to six $-NHR_6$ groups; or $R_1$ is said interrupted group further substituted by one to three $-SO_3H$ groups or by one to three $-COOR_6$ groups; or $R_1$ is a mono-valent homo- or co-oligomer consisting of monomer units derived from monomers selected from the group consisting of ethylene oxide, propylene oxide, ethylene glycol, propylene glycol, acrylic acid, methacrylic acid, ethylene imine, acrylamide, vinyl formamide, vinyl alcohol and vinyl acetate; which homo- or co-oligomer consists of between 2 and 24 monomer units;

$R_1'$ is independently defined as for $R_1$,

$R_6$ is hydrogen or $C_1$-$C_6$alkyl,

$R_6'$, $R_6''$ are independently defined as for $R_6$,

$R_7$ is $-N(R_2)(R_2')$ or is chlorine, alkoxy of 1 to 12 carbon atoms, 2-hydroxyethylamino or $-N(R_6)(R_6')$;

or $R_7$ is

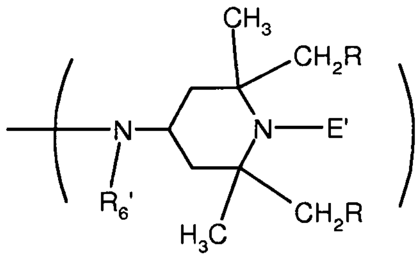

or

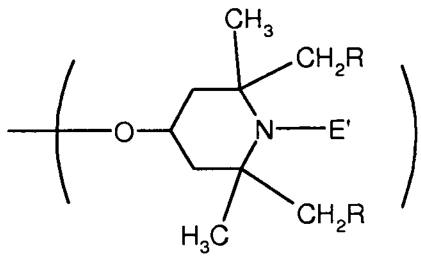

;

wherein E' is hydrogen, $C_1$-$C_{18}$alkyl, $C_2$-$C_{18}$alkenyl, $C_7$-$C_{18}$phenylalkyl, $C_2$-$C_{18}$alkanoyl or phenyl, or E' is independently defined as for E;

$R_8$ is defined as for $R_7$, where one of $R_7$ and $R_8$ is $-N(R_2)(R_2')$;

X is an inorganic or organic anion,

$Y^+$ is a mono-, di- or tri-valent cation, and

$R_2$ is $C_2$-$C_{12}$alkyl, $C_2$-$C_{12}$alkanoyl or $C_7$-$C_{18}$phenylalkyl, each interrupted by one to six oxygen, sulfur or $-N(R_6)-$ groups, each further substituted by 1 to 3 $-COO^-Y^+$ groups; or

$R_2$ is $C_1$-$C_{12}$alkyl, $C_2$-$C_{12}$alkanoyl, phenyl or $C_7$-$C_{18}$phenylalkyl, each substituted by one or two $-COO^-Y^+$, $-N(R_6)(R_6')(R_6'')^+X^-$ or $-SO_3^-Y^+$ groups; or

$R_2$ is $C_1$-$C_{12}$alkyl, $C_2$-$C_{12}$alkanoyl, phenyl or $C_7$-$C_{18}$phenylalkyl, each of which is substituted by one or two $-COO^-Y^+$, $-N(R_6)(R_6')(R_6'')^+X^-$ or $-SO_3^-Y^+$ groups, each further substituted by one or two -OH, $-COOR_6$ or $-NHR_6$ groups;

$R_2'$ is $C_2$-$C_{12}$alkyl, $C_2$-$C_{12}$alkanoyl or $C_7$-$C_{18}$phenylalkyl, each interrupted by one to six oxygen, sulfur or $-N(R_6)-$ groups; $C_1$-$C_{12}$alkyl, $C_2$-$C_{12}$alkanoyl, phenyl or $C_7$-$C_{18}$phenylalkyl, each substituted by one to six hydroxy groups or by one to six $-NHR_6$ groups; $C_2$-$C_{12}$alkyl, $C_2$-$C_{12}$alkanoyl or $C_7$-$C_{18}$phenylalkyl, each interrupted by one to three $-NR_6C(O)-$ groups; or $R_2$ is $C_1$-$C_{12}$alkyl, $C_2$-$C_{12}$alkanoyl, phenyl or $C_7$-$C_{18}$phenylalkyl, each substituted by one to three $-SO_3H$ groups or by one to three $-COOR_6$ groups; or

$R_2'$ is alkyl substituted by a piperazine or by a morpholine group; or

$R_2'$ is one of said interrupted groups further substituted by one to six hydroxy groups or by one to six $-NHR_6$ groups; or

$R_2'$ is one of said interrupted groups further substituted by one to three $-SO_3H$ groups or by one to three $-COOR_6$ groups; or

$R_2'$ is $C_2$-$C_{12}$alkyl, $C_2$-$C_{12}$alkanoyl or $C_7$-$C_{18}$phenylalkyl, each interrupted by one to six oxygen, sulfur or $-N(R_6)-$ groups, each further substituted by 1 to 3 $-COO^-Y^+$ groups; or

$R_2'$ is $C_1$-$C_{12}$alkyl, $C_2$-$C_{12}$alkanoyl, phenyl or $C_7$-$C_{18}$phenylalkyl, each substituted by one or two $-COO^-Y^+$, $-N(R_6)(R_6')(R_6'')^+X^-$ or $-SO_3^-Y^+$ groups; or

$R_2'$ is $C_1$-$C_{12}$alkyl, $C_2$-$C_{12}$alkanoyl, phenyl or $C_7$-$C_{18}$phenylalkyl, each of which is substituted by one or two $-COO^-Y^+$, $-N(R_6)(R_6')(R_6'')^+X^-$ or $-SO_3^-Y^+$ groups, each further substituted by one or two -OH, $-COOR_6$ or $-NHR_6$ groups; or

$R_2'$ is a mono-valent homo- or co-oligomer consisting of monomer units derived from monomers selected from the group consisting of ethylene oxide, propylene oxide, ethylene glycol, propylene glycol, acrylic acid, methacrylic acid, ethylene imine, acrylamide, vinyl formamide, vinyl alcohol and vinyl acetate; which homo- or co-oligomer consists of between 2 and 24 monomer units, or

$R_2'$ is hydrogen,

$R_3$ is $C_2$-$C_{12}$alkyl, $C_2$-$C_{12}$alkanoyl or $C_7$-$C_{18}$phenylalkyl, each interrupted by one to six oxygen, sulfur or $-N(R_6)-$ groups, each further substituted by 1 to 3 $-COO^-Y^+$ groups; or

$R_3$ is $C_1$-$C_{12}$alkyl, $C_2$-$C_{12}$alkanoyl, phenyl or $C_7$-$C_{18}$phenylalkyl, each substituted by one or two $-COO^-Y^+$, $-N(R_6)(R_6')(R_6'')^+X^-$ or $-SO_3^-Y^+$ groups; or

$R_3$ is $C_1$-$C_{12}$alkyl, $C_2$-$C_{12}$alkanoyl, phenyl or $C_7$-$C_{18}$phenylalkyl, each of which is substituted by one or two $-COO^-Y^+$, $-N(R_6)(R_6')(R_6'')^+X^-$ or $-SO_3^-Y^+$ groups, each further substituted by one or two -OH, $-COOR_6$ or $-NHR_6$ groups; or

$R_3$ is $-SO_3^-Y^+$ or $-PO_3H^-Y^+$, and

$R_4$ is $C_2$-$C_{12}$alkyl, $C_2$-$C_{12}$alkanoyl or $C_7$-$C_{18}$phenylalkyl, each interrupted by one to six oxygen, sulfur or $-N(R_6)-$ groups, each further substituted by 1 to 3 $-COO^-Y^+$ groups; or

$R_4$ is $C_1$-$C_{12}$alkyl, $C_2$-$C_{12}$alkanoyl, phenyl or $C_7$-$C_{18}$phenylalkyl, each substituted by one or two $-COO^-Y^+$, $-N(R_6)(R_6')(R_6'')^+X^-$ or $-SO_3^-Y^+$ groups; or

$R_4$ is $C_1$-$C_{12}$alkyl, $C_2$-$C_{12}$alkanoyl, phenyl or $C_7$-$C_{18}$phenylalkyl, each of which is substituted by one or two

$-COO^-Y^+$, $-N(R_6)(R_6')(R_6'')^+X^-$ or $-SO_3^-Y^+$ groups, each further substituted by one or two -OH, $-COOR_6$ or $-NHR_6$ groups.

**2.** A compound according to claim 1 of the formula (1) wherein $R_1$' is hydrogen.

**3.** A compound according to claim 2 where $R_1$ is hydrogen, $C_1$-$C_4$alkyl, $C_2$-$C_5$alkanoyl, $C_2$-$C_4$alkyl or $C_2$-$C_5$alkanoyl interrupted by an oxygen, sulfur or $-N(R_6)$- group; $C_1$-$C_4$alkyl or $C_2$-$C_5$alkanoyl substituted by an hydroxy group or by a $-NHR_6$ group, $C_2$-$C_4$alkyl or $C_2$-$C_5$alkanoyl interrupted by a $-NR_6C(O)$- group, or is $C_1$-$C_4$alkyl or $C_2$-$C_5$alkanoyl substituted by a $-SO_3H$ or by a $-COOR_6$ group.

**4.** A compound according to claim 1 of the formula (2), wherein $R_2$' is hydrogen , or of the formula (3)

**5.** A compound according to claim 4 where $R_2$ and $R_3$ are $C_1$-$C_4$alkyl or $C_2$-$C_5$alkanoyl substituted by a $-COO^-Y^+$, $-N(R_6)(R_6')(R_6'')^+X^-$ or $-SO_3^-Y^+$ group

**6.** A compound according to claim 1 of the formula (4)

**7.** A compound according to claim 6 where
$R_4$ is $C_1$-$C_4$alkyl or $C_2$-$C_5$alkanoyl substituted by a $-COO^-Y^+$, $-N(R_6)(R_6')(R_6'')^+X^-$ or $-SO_3^-Y^+$ group

**8.** A compound according to claim 1 of the formula

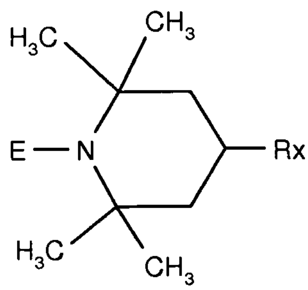

where $R_x$ is selected from the group consisting of $-NH_2^+CH_2CH_2OH\ Cl^-$, $-NH_3^+\ ^-OAc$, $-NHCH(CH_3)COO^-K^+$, $-NHCH_2CH_2NH(CH_3)_2^+\ ^-OAc$,- $NHCH_2CH_2SO_3^-K^+$, $-NHCH(COO^-\ K^+)CH_2CH_2SCH_3$, $-NHCH_2COO^-\ K^+$, $-OCH(CH_3)COO^-K^+$, $-OCH_2CH_2NH(CH_3)_2^+$ OAc, $-OCH_2CH_2SO_3^-K^+$, $-OCH(COO^-\ K^+)CH_2CH_2SCH_3$, $-OCH_2COO^-\ K^+$.

**9.** A compound according to claim 1 of formula (1), (2), (3) or (5) wherein
$R_1$ is hydrogen, $C_1$-$C_4$alkyl substituted by a hydroxy or a $COOR_6$ group; or $R_1$ is $C_2$-$C_4$alkyl interupted by a $-N(R_6)$- group; or $R_1$ is $C_2$-$C_{12}$alkyl interupted by 1 to 6 $-N(R_6)$-groups;
$R_1$' is hydrogen;
$R_2$ is $C_1$-$C_4$alkyl substituted by a $-COO^-Y^+$, $-N(R_6)(R_6')(R_6'')^+X^-$, $-SO_3^-Y^+$; or $R_2$ is $C_2$-$C_{12}$alkyl interrupted by one to six sulfur groups further substituted by 1 to 3 $-COO^-Y^+$ groups; or $R_2$ is phenyl substituted by 1 to 2 $-SO_3^-Y^+$ groups; or $R_2$ is phenyl which is substituted by one or two $-COO^-Y^+$ groups;
$R_2$' is hydrogen;
$R_3$ is $-SO_3^-Y^+$, $C_2$-$C_5$alkanoyl substituted by a $-COO^-Y^+$ group;
$R_6$' is $C_1$-$C_6$alkyl;
$R_7$ is $-N(R_2)(R_2')$ or

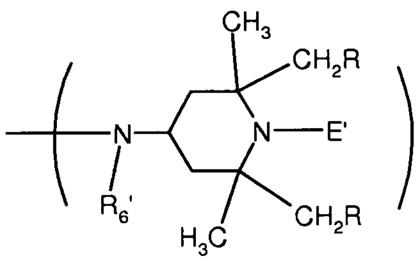 ;

$R_8$ is as defined for $R_7$, where one of $R_7$ and $R_8$ is $-N(R_2)(R_2')$;

**10.** A stabilized composition comprising
an organic material subject to the deleterious effects of light, heat and oxygen, and a water compatible or water soluble sterically hindered hydroxy substituted alkoxyamine compound according to claim **1**.

**11.** A composition according to claim **10** which is a coating, ink jet ink, ink jet recording material, photographic recording material, multi-layer polymer structure, a coextruded film, a radiation cured film, ink or coating, an adhesive or a laminate.

**12.** A composition according to claim **10** which additionally comprises at least one coadditive stabilizer selected from the group consisting of the phenolic antioxidants, metal stearates, metal oxides, organophosphorus compounds, benzofuranone antioxidants, hydroxylamines, ultraviolet light absorbers, and other hindered amine light stabilizers.

**13.** A composition according to claim **10** which additionally comprises an ultraviolet light absorber selected from the group consisting of the benzophenones, 2H-benzotriazoles, aryl-s-triazines.

**14.** A composition according to claim **10** which is a colored composition containing pigments or dyes.

**15.** A process for stabilizing organic material subject to oxidative, thermal and/or actinic degradation which comprises adding to or incorporating into said material at least one compound according to claim **1**.

## Patentansprüche

**1.** Wasserverträgliche oder wasserlösliche sterisch gehinderte hydroxysubstituierte Alkoxyaminverbindung, ausgewählt aus der Gruppe bestehend aus Verbindungen der Formeln (1) bis (5)

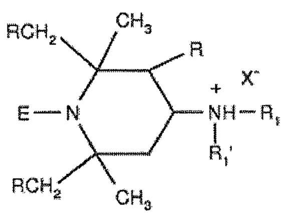

(1)

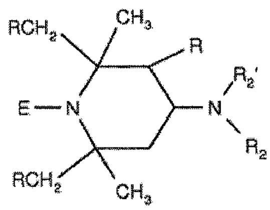

(2)

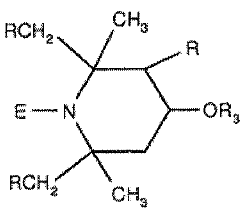

(3)

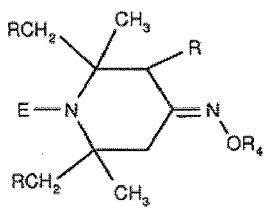

(4)

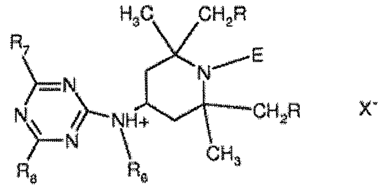

(5)

wobei
E für -O-T-$(OH)_b$ steht,
T für ein gerad- oder verzweigtkettiges Alkylen mit 1 bis 18 Kohlenstoffatomen, Cycloalkylen mit 5 bis 18 Kohlenstoffatomen, Cycloalkenylen mit 5 bis 18 Kohlenstoffatomen oder ein gerad- oder verzweigtkettiges Alkylen mit 1 bis 4 Kohlenstoffatomen, das durch Phenyl oder durch Phenyl, das durch eine oder zwei Alkylgruppen mit 1 bis 4 Kohlenstoffatomen substituiert ist, substituiert ist, steht;
b für 1, 2 oder 3 steht, mit der Maßgabe, dass b nicht größer als die Zahl der Kohlenstoffatome in T sein kann und dann, wenn b für 2 oder 3 steht,
jede Hydroxylgruppe an ein anderes Kohlenstoffatom von T gebunden ist;
R für Wasserstoff oder Methyl steht;
$R_1$ für Wasserstoff, $C_1$-$C_{12}$-Alkyl, $C_5$-$C_8$-Cycloalkyl, $C_5$-$C_8$-Cycloalkyl, das durch ein bis drei $C_1$-$C_4$-Alkyl substituiert ist, $C_2$-$C_{12}$-Alkenyl, Phenyl, $C_7$-$C_9$-Phenylalkyl, Glycidyl, $C_2$-$C_{12}$-Alkanoyl, $C_6$-$C_9$-Cycloalkylcarbonyl, $C_2$-$C_{12}$-Carbamoyl, $C_2$-$C_{12}$-Alkenoyl, Benzoyl, Benzoyl, das durch ein bis drei $C_1$-$C_4$-Alkyl substituiert ist, oder $C_2$-$C_{12}$-Alkanoyl, das durch ein Di($C_1$-$C_6$-alkyl)phosphonat substituiert ist, steht,
oder $R_1$ für $C_2$-$C_{12}$-Alkyl, $C_2$-$C_{12}$-Alkanoyl oder $C_7$-$C_{18}$-Phenylalkyl, jeweils durch eine bis sechs Sauerstoff-, Schwefel- oder -$N(R_6)$-Gruppen unterbrochen; $C_1$-$C_{12}$-Alkyl, $C_2$-$C_{12}$-Alkanoyl, Phenyl oder $C_7$-$C_{18}$-Phenylalkyl, jeweils durch eine bis sechs Hydroxygruppen oder durch eine bis sechs -$NHR_6$-Gruppen substituiert; $C_2$-$C_{12}$-Alkyl, $C_2$-$C_{12}$-Alkanoyl oder $C_7$-$C_{18}$-Phenylalkyl, jeweils durch eine bis drei -$NR_6C(O)$-Gruppen unterbrochen; oder $C_1$-$C_{12}$-Alkyl, $C_2$-$C_{12}$-Alkanoyl, Phenyl oder $C_7$-$C_{18}$-Phenylalkyl, jeweils durch eine bis drei -$SO_3H$-Gruppen oder eine bis drei -$COOR_6$-Gruppen substituiert; steht oder
$R_1$ für das Alkyl, das durch eine Piperazin- oder eine Morpholingruppe substituiert ist, steht oder $R_1$ für die unterbrochene Gruppe, die ferner durch eine bis sechs Hydroxygruppen oder eine bis sechs -$NHR_6$-Gruppen substituiert ist; steht oder
$R_1$ für die unterbrochene Gruppe, die ferner durch eine bis drei -$SO_3H$-Gruppen oder eine bis drei -$COOR_6$-Gruppen substituiert ist, steht oder $R_1$ für ein einwertiges Homo- oder Cooligomer aus Monomereinheiten, die sich von Monomeren aus der Gruppe bestehend aus Ethylenoxid, Propylenoxid, Ethylenglykol, Propylenglykol, Acrylsäure, Methacrylsäure, Ethylenimin, Acrylamid, Vinylformamid, Vinylalkohol und Vinylacetat ableiten, steht, wobei das Homo- oder Cooligomer aus zwischen 2 und 24 Monomereinheiten besteht;
$R_1$' unabhängig wie für $R_1$ definiert ist;
$R_6$ für Wasserstoff oder $C_1$-$C_6$-Alkyl steht;
$R_6$' und $R_6$'' unabhängig wie für $R_6$ definiert sind;
$R_7$ für -$N(R_2)(R_2')$ oder Chlor, Alkoxy mit 1 bis 12 Kohlenstoffatomen, 2-Hydroxyethylamino oder -$N(R_6)(R_6')$ steht oder $R_7$ für

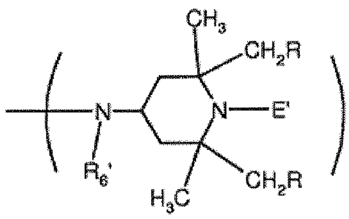

oder

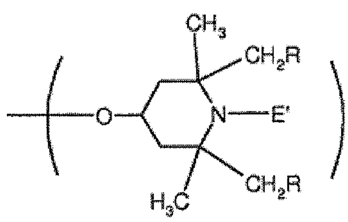

steht; wobei E' für Wasserstoff, $C_1$-$C_{18}$-Alkyl, $C_2$-$C_{18}$-Alkenyl, $C_7$-$C_{15}$-Phenylalkyl, $C_2$-$C_{18}$-Alkanoyl oder Phenyl steht oder E' unabhängig wie für E definiert ist;
$R_8$ wie für $R_7$ definiert ist, wobei eine der Gruppen $R_7$ und $R_8$ für -$N(R_2)(R_2')$ steht;
$X^-$ für ein anorganisches oder organisches Anion steht;
$Y^+$ für ein ein-, zwei- oder dreiwertiges Kation steht und
$R_2$ für $C_2$-$C_{12}$-Alkyl, $C_2$-$C_{12}$-Alkanoyl oder $C_7$-$C_{18}$-Phenylalkyl, jeweils durch eine bis sechs Sauerstoff-, Schwefel- oder -$N(R_6)$-Gruppen unterbrochen und jeweils weiter durch 1 bis 3 -$COO^-Y^+$-Gruppen substituiert, steht oder
$R_2$ für $C_1$-$C_{12}$-Alkyl, $C_2$-$C_{12}$-Alkanoyl, Phenyl oder $C_7$-$C_{18}$-Phenylalkyl, jeweils durch eine oder zwei -$COO^-Y^+$-, -$N(R_6)(R_6')(R_6'')^+X^-$- oder -$SO_3^-Y^+$-Gruppen substituiert, steht oder
$R_2$ für $C_1$-$C_{12}$-Alkyl, $C_2$-$C_{12}$-Alkanoyl, Phenyl oder $C_7$-$C_{18}$-Phenylalkyl, wobei jede dieser Gruppen durch eine oder zwei -$COO^-Y^+$-, -$N(R_6)(R_6')(R_6'')^+X^-$- oder - $SO_3^-Y^+$-Gruppen substituiert ist und jeweils weiter durch eine oder zwei -OH-, -$COOR_6$- oder $NHR_6$-Gruppen substituiert ist, steht;
$R_2$' für $C_2$-$C_{12}$-Alkyl, $C_2$-$C_{12}$-Alkanoyl oder $C_7$-$C_{18}$-Phenylalkyl, jeweils durch eine bis sechs Sauerstoff-, Schwefel- oder -$N(R_6)$-Gruppen unterbrochen; $C_1$-$C_{12}$-Alkyl, $C_2$-$C_{12}$-Alkanoyl, Phenyl oder
$C_7$-$C_{18}$-Phenylalkyl, jeweils durch eine bis sechs Hydroxygruppen oder durch eine bis sechs -$NHR_6$-Gruppen sub-

stituiert; $C_2$-$C_{12}$-Alkyl, $C_2$-$C_{12}$-Alkanoyl oder $C_7$-$C_{18}$-Phenylalkyl, jeweils durch eine bis drei -$NR_6C(O)$-Gruppen unterbrochen; oder für $C_1$-$C_{12}$-Alkyl, $C_2$-$C_{12}$-Alkanoyl, Phenyl oder $C_7$-$C_{18}$-Phenylalkyl, jeweils durch eine bis drei -$SO_3H$-Gruppen oder eine bis drei -$COOR_6$-Gruppen substituiert; steht oder $R_2$' für Alkyl, das durch eine Piperazin- oder eine Morpholingruppe substituiert ist, steht oder

$R_2$' für eine der unterbrochenen Gruppen, die ferner durch eine bis sechs Hydroxygruppen oder eine bis sechs -$NHR_6$-Gruppen substituiert sind, steht oder

$R_2$' für eine der unterbrochenen Gruppen, die ferner durch eine bis drei -$SO_3H$-Gruppen oder eine bis drei -$COOR_6$-Gruppen substituiert sind, steht oder

$R_2$' für $C_2$-$C_{12}$-Alkyl, $C_2$-$C_{12}$-Alkanoyl oder $C_7$-$C_{18}$-Phenylalkyl, jeweils durch eine bis sechs Sauerstoff-, Schwefel- oder -$N(R_6)$-Gruppen unterbrochen und jeweils weiter durch 1 bis 3 -$COO^-Y^+$-Gruppen substituiert, steht oder

$R_2$' für $C_1$-$C_{12}$-Alkyl, $C_2$-$C_{12}$-Alkanoyl, Phenyl oder $C_7$-$C_{18}$-Phenylalkyl, jeweils durch eine oder zwei -$COO^-Y^+$-, -N $(R_6)(R_6')(R_6'')^+X^-$- oder -$SO_3^-Y^+$-Gruppen substituiert, steht oder

$R_2$' für $C_1$-$C_{12}$-Alkyl, $C_2$-$C_{12}$-Alkanoyl, Phenyl oder $C_7$-$C_{18}$-Phenylalkyl, wobei jede dieser Gruppen durch eine oder zwei -$COO^-Y^+$-, -$N(R_6)(R_6')(R_6'')^+X^-$- oder - $SO_3^-Y^+$-Gruppen substituiert ist und jeweils weiter durch eine oder zwei -OH-, -$COOR_6$- oder $NHR_6$-Gruppen substituiert ist, steht oder

$R_2$' für ein einwertiges Homo- oder Cooligomer aus Monomereinheiten, die sich von Monomeren aus der Gruppe bestehend aus Ethylenoxid, Propylenoxid, Ethylenglykol, Propylenglykol, Acrylsäure, Methacrylsäure, Ethylenimin, Acrylamid, Vinylformamid, Vinylalkohol und Vinylacetat ableiten, steht, wobei das Homo- oder Cooligomer aus zwischen 2 und 24 Monomereinheiten besteht; oder

$R_2$' für Wasserstoff steht;

$R_3$ für $C_2$-$C_{12}$-Alkyl, $C_2$-$C_{12}$-Alkanoyl oder $C_7$-$C_{18}$-Phenylalkyl, jeweils durch eine bis sechs Sauerstoff-, Schwefel- oder -$N(R_6)$-Gruppen unterbrochen und jeweils weiter durch 1 bis 3 -$COO^-Y^+$-Gruppen substituiert, steht oder

$R_3$ für $C_1$-$C_{12}$-Alkyl, $C_2$-$C_{12}$-Alkanoyl, Phenyl oder $C_7$-$C_{18}$-Phenylalkyl, jeweils durch eine oder zwei -$COO^-Y^+$-, -N $(R_6)(R_6')(R_6'')^+X^-$- oder -$SO_3^-Y^+$-Gruppen substituiert, steht oder

$R_3$ für $C_1$-$C_{12}$-Alkyl, $C_2$-$C_{12}$-Alkanoyl, Phenyl oder $C_7$-$C_{18}$-Phenylalkyl, wobei jede dieser Gruppen durch eine oder zwei -$COO^-Y^+$-, -$N(R_6)(R_6')(R_6'')^+X^-$- oder -$SO_3^-Y^+$-Gruppen substituiert ist und jeweils weiter durch eine oder zwei -OH-, -$COOR_6$- oder $NHR_6$-Gruppen substituiert ist, steht oder

$R_3$ für -$SO_3^-Y^+$ oder -$PO_3H^-Y^+$ steht und

$R_4$ für $C_2$-$C_{12}$-Alkyl, $C_2$-$C_{12}$-Alkanoyl oder $C_7$-$C_{18}$-Phenylalkyl, jeweils durch eine bis sechs Sauerstoff-, Schwefel- oder -$N(R_6)$-Gruppen unterbrochen und jeweils weiter durch 1 bis 3 -$COO^-Y^+$-Gruppen substituiert, steht oder

$R_4$ für $C_1$-$C_{12}$-Alkyl, $C_2$-$C_{12}$-Alkanoyl, Phenyl oder $C_7$-$C_{18}$-Phenylalkyl, jeweils durch eine oder zwei -$COO^-Y^+$-, -N $(R_6)(R_6')(R_6'')^+X^-$- oder -$SO_3^-Y^+$-Gruppen substituiert, steht oder

$R_4$ für $C_1$-$C_{12}$-Alkyl, $C_2$-$C_{12}$-Alkanoyl, Phenyl oder $C_7$-$C_{18}$-Phenylalkyl, wobei jede dieser Gruppen durch eine oder zwei -$COO^-Y^+$-, -$N(R_6)(R_6')(R_6'')^+X^-$- oder -$SO_3^-Y^+$-Gruppen substituiert ist und jeweils weiter durch eine oder zwei -OH-, -$COOR_6$- oder $NHR_6$-Gruppen substituiert ist, steht.

**2.** Verbindung nach Anspruch 1 der Formel (1), worin $R_1$' für Wasserstoff steht.

**3.** Verbindung nach Anspruch 2, wobei $R_1$ für Wasserstoff, $C_1$-$C_4$-Alkyl, $C_2$-$C_5$-Alkanoyl, durch eine Sauerstoff-, Schwefel- oder -$N(R_6)$-Gruppe unterbrochenes $C_2$-$C_4$-Alkyl oder $C_2$-$C_5$-Alkanoyl; durch eine Hydroxygruppe oder eine -$NHR_6$-Gruppe substituiertes $C_1$-$C_4$-Alkyl oder $C_2$-$C_5$-Alkanoyl; durch eine -$NR_6C(O)$-Gruppe unterbrochenes $C_2$-$C_4$-Alkyl oder $C_2$-$C_5$-Alkanoyl oder durch eine -$SO_3H$- oder -$COOR_6$-Gruppe substituiertes $C_1$-$C_4$-Alkyl oder $C_2$-$C_5$-Alkanoyl steht.

**4.** Verbindung nach Anspruch 1 der Formel (2), worin $R_2$' für Wasserstoff steht, oder der Formel (3).

**5.** Verbindung nach Anspruch 4, wobei $R_2$ und $R_3$ für durch eine -$COO^-Y^+$-, -$N(R_6)(R_6')(R_6'')^+Y^-$- oder -$SO_3^-Y^+$-Gruppe substituiertes $C_1$-$C_4$-Alkyl oder $C_2$-$C_5$-Alkanoyl stehen.

**6.** Verbindung nach Anspruch 1 der Formel (4).

**7.** Verbindung nach Anspruch 6, wobei
$R_4$ für durch eine -$COO^-Y^+$-, -$N(R_6)(R_6')(R_6'')^+X^-$- oder -$S0_3^-Y^+$-Gruppe substituiertes $C_1$-$C_4$-Alkyl oder $C_2$-$C_5$-Alkanoyl steht.

**8.** Verbindung nach Anspruch 1 der Formel

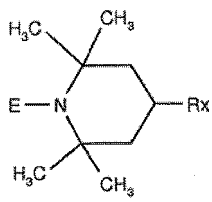

wobei $R_x$ aus der Gruppe bestehend aus $-NH_2^+CH_2CH_2OH\ Cl^-$, $-NH_3^+ + {}^-OAc$, $-NHCH(CH_3)COO^-K^+$, $-NHCH_2CH_2NH(CH_3)_2^+\ {}^-OAc$, $-NHCH_2CH_2SO_3^-K^+$, $-NHCH(COO^-K^+)CH_2CH_2SCH_3$, $-NHCH_2COO^-K^+$, $-OCH(CH_3)COO^-K^+$, $-OCH_2CH_2NH(CH_3)_2^+\ {}^-OAc$, $-OCH_2CH_2SO_3^-K^+$, $-OCH(COO^-K^+)CH_2CH_2SCH_3$ und $-OCH_2COO^-K^+$ ausgewählt ist.

**9.** Verbindung nach Anspruch 1 der Formel (1), (2), (3) oder (5), worin
$R_1$ für Wasserstoff, durch eine Hydroxy- oder $COOR_6$-Gruppe substituiertes $C_1$-$C_4$-Alkyl steht oder $R_1$ für durch eine $-N(R_6)$-Gruppe unterbrochenes $C_2$-$C_4$-Alkyl steht oder $R_1$ für durch 1 bis 6 $-N(R_6)$-Gruppen unterbrochenes $C_2$-$C_{12}$-Alkyl steht;
$R_1$' für Wasserstoff steht;
$R_2$ für durch ein $-COO^-Y^+$, $-N(R_6)(R_6')(R_6'')^+X^-$ oder $-SO_3^-Y^+$ substituiertes $C_1$-$C_4$-Alkyl steht oder $R_2$ für $C_2$-$C_{12}$-Alkyl, das durch eine bis sechs Schwefelgruppen unterbrochen und weiter durch 1 bis 3 $-COO^-Y^+$-Gruppen substituiert ist; oder $R_2$ für Phenyl, das durch 1 bis 2 $-SO_3^-Y^+$-Gruppen substituiert ist, steht oder $R_2$ für Phenyl, das durch eine oder zwei $-COO^-Y^+$-Gruppen substituiert ist, steht;
$R_2$' für Wasserstoff steht;
$R_3$ für $-SO_3^-Y^+$ oder durch eine $-COO^-Y^+$-Gruppe substituiertes $C_2$-$C_5$-Alkanoyl steht;
$R_6$' für $C_1$-$C_6$-Alkyl steht;
$R_7$ für $-N(R_2)(R_2')$ oder

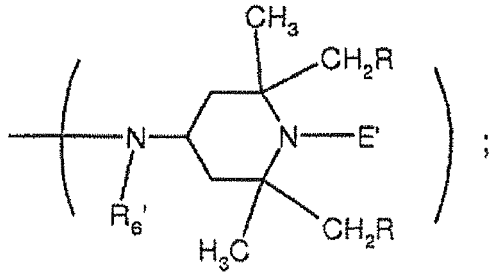

steht;
$R_8$ wie für $R_7$ definiert ist, wobei eine der Gruppen $R_7$ und $R_8$ für $-N(R_2)(R_2')$ steht.

**10.** Stabilisierte Zusammensetzung, umfassend ein organisches Material, das den nachteiligen Wirkungen von Licht, Wärme und Sauerstoff unterliegt, und eine wasserverträgliche oder wasserlösliche sterisch gehinderte hydroxysubstituierte Alkoxyaminverbindung nach Anspruch 1.

**11.** Zusammensetzung nach Anspruch 10, bei der es sich um einen Überzug, eine Tintenstrahltinte, ein Tintentrahlaufzeichnungsmaterial, ein photographisches Aufzeichnungsmaterial, eine mehrschichtige Polymerstruktur, eine coextrudierte Folie, eine strahlungsgehärtete Folie, eine strahlungsgehärtete Tinte, einen strahlungsgehärteten Überzug, einen Klebstoff oder ein Laminat handelt.

**12.** Zusammensetzung nach Anspruch 10, die zusätzlich mindestens einen Coadditiv-Stabilisator aus der Gruppe bestehend aus phenolischen Antioxidantien, Metallstearaten, Metalloxiden, Organophosphorverbindungen, Benzofuran-Antioxidantien, Hydroxylaminen, Ultraviolettlichtabsorbern und anderen Lichtschutzmitteln vom HALS-Typ umfasst.

**13.** Zusammensetzung nach Anspruch 10, die zusätzlich einen Ultraviolettlichtabsorber aus der Gruppe bestehend aus den Benzophenonen, 2N-benzotriazolen und Aryl-s-triazinen umfasst.

**14.** Zusammensetzung nach Anspruch 10, bei der es sich um eine Pigmente oder Farbstoff enthaltende gefärbte Zusammensetzung handelt.

**15.** Verfahren zur Stabilisierung von organischem Material, das oxidativem, thermischem und/oder aktinischem Abbau unterliegt, bei dem man mindestens eine Verbindung nach Anspruch 1 zu dem Material gibt oder in das Material einarbeitet.

## Revendications

**1.** Composé d'alcoxyamine hydroxysubstitué stériquement encombré hydrocompatible ou hydrosoluble choisi dans le groupe constitué par les composés des formules (1) à (5) :

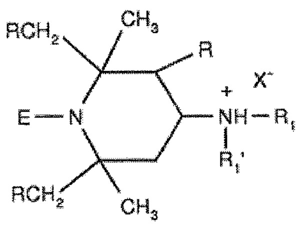

(1)

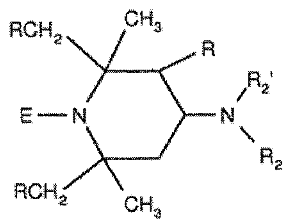

(2)

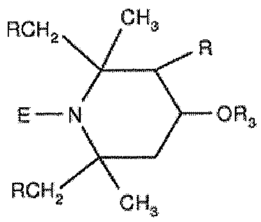

(3)

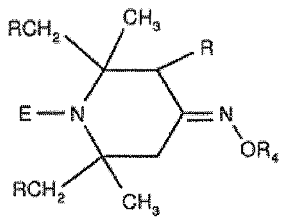

(4)

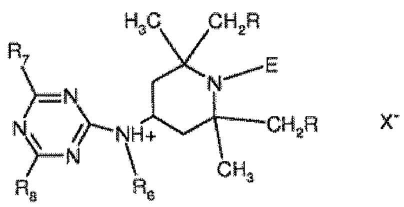

(5)

où
E représente -O-T-$(OH)_b$,
T représente un alkylène à chaîne linéaire ou ramifiée de 1 à 18 atomes de carbone, un cycloalkylène de 5 à 18 atomes de carbone, un cycloalcénylène de 5 à 18 atomes de carbone, ou un alkylène à chaîne linéaire ou ramifiée de 1 à 4 atomes de carbone substitué par un phényle ou par un phényle substitué par un ou deux groupes alkyle de 1 à 4 atomes de carbone ;
b vaut 1, 2 ou 3, à condition que b ne puisse pas dépasser le nombre d'atomes de carbone dans T, et lorsque b vaut 2 ou 3, chaque groupe hydroxyle soit attaché à un atome de carbone différent de T ;
R représente un hydrogène ou un méthyle ;
$R_1$ représente un hydrogène, un alkyle en $C_1$-$C_{12}$, un cycloalkyle en $C_5$-$C_8$, un cycloalkyle en $C_5$-$C_8$ substitué par un à trois alkyles en $C_1$-$C_4$, un alcényle en $C_2$-$C_{12}$, un phényle, un phénylalkyle en $C_7$-$C_9$, un glycidyle, un alcanoyle en $C_2$-$C_{12}$, un cycloalkylcarbonyle en $C_6$-$C_9$, un carbamoyle en $C_2$-$C_{12}$, un alcénoyle en $C_2$-$C_{12}$, un benzoyle, un

benzoyle substitué par un à trois alkyles en $C_1$-$C_4$, ou un alcanoyle en $C_2$-$C_{12}$ substitué par un phosphonate de di (alkyle en $C_1$-$C_6$), ou bien

$R_1$ représente un alkyle en $C_2$-$C_{12}$, un alcanoyle en $C_2$-$C_{12}$ ou un phénylalkyle en $C_7$-$C_{18}$, chacun interrompu par un à six oxygènes, soufres ou groupes -N($R_6$)- ; un alkyle en $C_1$-$C_{12}$, un alcanoyle en $C_2$-$C_{12}$, un phényle ou un phénylalkyle en $C_7$-$C_{18}$, chacun substitué par un à six groupes hydroxyle ou par un à six groupes -NH$R_6$ ; un alkyle en $C_2$-$C_{12}$, un alcanoyle en $C_2$-$C_{12}$ ou un phénylalkyle en $C_7$-$C_{18}$, chacun interrompu par un à trois groupes -N$R_6$C (O)- ; ou un alkyle en $C_1$-$C_{12}$, un alcanoyle en $C_2$-$C_{12}$, un phényle ou un phénylalkyle en $C_7$-$C_{18}$, chacun substitué par un à trois groupes -$SO_3$H ou par un à trois groupes -COO$R_6$, ou bien

$R_1$ représente ledit alkyle substitué par un groupe pipérazine ou morpholine, ou bien

$R_1$ représente ledit groupe interrompu substitué en outre par un à six groupes hydroxyle ou par un à six groupes -NH$R_6$, ou bien

$R_1$ représente ledit groupe interrompu substitué en outre par un à trois groupes -$SO_3$H ou par un à trois groupes -COO$R_6$, ou bien

$R_1$ représente un homo- ou co-oligomère monovalent constitué de motifs monomères dérivés de monomères choisis dans le groupe constitué par l'oxyde d'éthylène, l'oxyde de propylène, l'éthylène glycol, le propylène glycol, l'acide acrylique, l'acide méthacrylique, l'éthylène-imine, l'acrylamide, le vinylformamide, l'alcool vinylique et l'acétate de vinyle, lequel homo- ou co-oligomère se compose de 2 à 24 motifs monomères ;

$R_1$' est indépendamment défini comme $R_1$ ;

$R_6$ représente un hydrogène ou un alkyle en $C_1$-$C_6$ ; $R_6$' et $R_6$" sont indépendamment définis comme $R_6$ ;

$R_7$ représente -N($R_2$)($R_2$'), ou représente un chlore, un alcoxy de 1 à 12 atomes de carbone, un groupe 2-hydroxyéthylamino ou -N($R_6$)($R_6$'), ou bien

$R_7$ représente

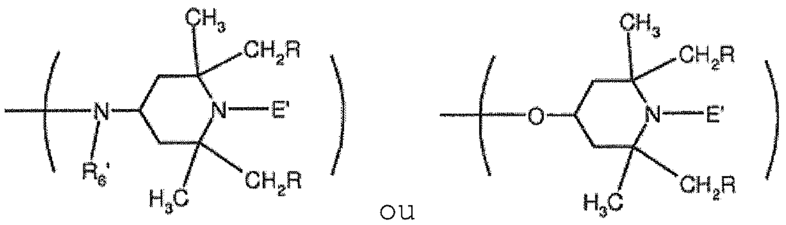

dans lesquels E' représente un hydrogène, un alkyle en $C_1$-$C_{18}$, un alcényle en $C_2$-$C_{18}$, un phénylalkyle en $C_7$-$C_{15}$, un alcanoyle en $C_2$-$C_{18}$ ou un phényle, ou bien E' est indépendamment défini comme E ;

$R_8$ est défini comme $R_7$, l'un de $R_7$ et $R_8$ représentant -N($R_2$)($R_2$') ;

$X^-$ représente un anion inorganique ou organique ;

$Y^+$ représente un cation mono-, di- ou trivalent ;

$R_2$ représente un alkyle en $C_2$-$C_{12}$, un alcanoyle en $C_2$-$C_{12}$ ou un phénylalkyle en $C_7$-$C_{18}$, chacun interrompu par un à six oxygènes, soufres ou groupes -N($R_6$)-, chacun substitué en outre par un à trois groupes -$COO^-Y^+$, ou bien

$R_2$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes -$COO^-Y^+$, -N($R_6$)($R_6$')($R_6$")$^+X^-$ ou -$SO_3^-Y^+$, ou bien

$R_2$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes -$COO^-Y^+$, -N($R_6$)($R_6$')($R_6$")$^+X^-$ ou -$SO_3^-Y^+$, chacun substitué en outre par un ou deux groupes -OH, -COO$R_6$ ou -NH$R_6$ ;

$R_2$' représente un alkyle en $C_2$-$C_{12}$, un alcanoyle en $C_2$-$C_{12}$ ou un phénylalkyle en $C_7$-$C_{18}$, chacun interrompu par un à six oxygènes, soufres ou groupes -N($R_6$)- ; un alkyle en $C_1$-$C_{12}$, un alcanoyle en $C_2$-$C_{12}$, un phényle ou un phénylalkyle en $C_7$-$C_{18}$, chacun substitué par un à six groupes hydroxyle ou par un à six groupes -NH$R_6$ ; un alkyle en $C_2$-$C_{12}$, un alcanoyle en $C_2$-$C_{12}$ ou un phénylalkyle en $C_7$-$C_{18}$, chacun interrompu par un à trois groupes -N$R_6$C (O)- ; ou un alkyle en $C_1$-$C_{12}$, un alcanoyle en $C_2$-$C_{12}$, un phényle ou un phénylalkyle en $C_7$-$C_{18}$, chacun substitué par un à trois groupes -$SO_3$H ou par un à trois groupes -COO$R_6$, ou bien

$R_2$' représente ledit alkyle substitué par un groupe pipérazine ou morpholine, ou bien

$R_2$' représente l'un desdits groupes interrompus, substitué en outre par un à six groupes hydroxyle ou par un à six groupes -NH$R_6$, ou bien

$R_2$' représente l'un desdits groupes interrompus, substitué en outre par un à trois groupes -$SO_3$H ou par un à trois groupes -COO$R_6$, ou bien

$R_2$' représente un alkyle en $C_2$-$C_{12}$, un alcanoyle en $C_2$-$C_{12}$ ou un phénylalkyle en $C_7$-$C_{18}$, chacun interrompu par un à six oxygènes, soufres ou groupes -N($R_6$)-, chacun substitué en outre par un à trois groupes -$COO^-Y^+$, ou bien

$R_2$' représente un alkyle en $C_1$-$C_{12}$, un alcanoyle en $C_2$-$C_{12}$, un phényle ou un phénylalkyle en $C_7$-$C_{18}$, chacun

substitué par un ou deux groupes $-COO^-Y^+$, $-N(R_6)(R_6')(R_6'')^+X^-$ ou $-SO_3^-Y^+$, ou bien
$R_2'$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes $-COO^-Y^+$, $-N(R_6)(R_6')(R_6'')^+X^-$ ou $-SO_3^-Y^+$, chacun substitué en outre par un ou deux groupes -OH, $-COOR_6$ ou $-NHR_6$, ou bien
$R_2'$ représente un homo- ou co-oligomère monovalent constitué de motifs monomères dérivés de monomères choisis dans le groupe constitué par l'oxyde d'éthylène, l'oxyde de propylène, l'éthylène glycol, le propylène glycol, l'acide acrylique, l'acide méthacrylique, l'éthylène-imine, l'acrylamide, le vinylformamide, l'alcool vinylique et l'acétate de vinyle, lequel homo- ou co-oligomère se compose de 2 à 24 motifs monomères, ou bien
$R_2'$ représente un hydrogène ;
$R_3$ représente un alkyle en $C_2$-$C_{12}$, un alcanoyle en $C_2$-$C_{12}$ ou un phénylalkyle en $C_7$-$C_{18}$, chacun interrompu par un à six oxygènes, soufres ou groupes $-N(R_6)$-, chacun substitué en outre par un à trois groupes $-COO^-Y^+$, ou bien
$R_3$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes $-COO^-Y^+$, $-N(R_6)(R_6')(R_6'')^+X^-$ ou $-SO_3^-Y^+$, ou bien
$R_3$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes $-COO^-Y^+$, $-N(R_6)(R_6')(R_6'')^+X^-$ ou $-SO_3^-Y^+$, chacun substitué en outre par un ou deux groupes -OH, $-COOR_6$ ou $-NHR_6$, ou bien
$R_3$ représente $-SO_3^-Y^+$ ou $-PO_3H^-Y^+$ ; et
$R_4$ représente un alkyle en $C_2$-$C_{12}$, un alcanoyle en $C_2$-$C_{12}$ ou un phénylalkyle en $C_7$-$C_{18}$, chacun interrompu par un à six oxygènes, soufres ou groupes $-N(R_6)$-, chacun substitué en outre par un à trois groupes $-COO^-Y^+$, ou bien
$R_4$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes $-COO^-Y^+$, $-N(R_6)(R_6')(R_6'')^+X^-$ ou $-SO_3^-Y^+$, ou bien
$R_4$ représente un alkyle en $C_1$-$C_{12}$, un alcanoyle en $C_2$-$C_{12}$, un phényle ou un phénylalkyle en $C_7$-$C_{18}$, chacun substitué par un ou deux groupes $-COO^-Y^+$, $-N(R_6)(R_6')(R_6'')^+X^-$ ou $-SO_3^-Y^+$, chacun substitué en outre par un ou deux groupes -OH, $-COOR_6$ ou $-NHR_6$.

**2.** Composé selon la revendication 1 de formule (1) dans laquelle $R_1'$ représente un hydrogène.

**3.** Composé selon la revendication 2 dans lequel $R_1$ représente un hydrogène, un alkyle en $C_1$-$C_4$, un alcanoyle en $C_2$-$C_5$, un alkyle en $C_2$-$C_4$ ou un alcanoyle en $C_2$-$C_5$ interrompu par un oxygène, un soufre ou un groupe $-N(R_6)$-, un alkyle en $C_1$-$C_4$ ou un alcanoyle en $C_2$-$C_5$ substitué par un groupe hydroxyle par un groupe $-NHR_6$, un alkyle en $C_2$-$C_4$ ou un alcanoyle en $C_2$-$C_5$ interrompu par un groupe $-NR_6C(O)$-, ou un alkyle en $C_1$-$C_4$ ou un alcanoyle en $C_2$-$C_5$ substitué par un groupe $-SO_3H$ ou $-COOR_6$.

**4.** Composé selon la revendication 1 de formule (2) dans laquelle $R_2'$ représente un hydrogène, ou de formule (3).

**5.** Composé selon la revendication 4 dans lequel $R_2$ et $R_3$ représentent un alkyle en $C_1$-$C_4$ ou un alcanoyle en $C_2$-$C_5$ substitué par un groupe $-COO^-Y^+$, $-N(R_6)(R_6')(R_6'')^+X^-$ ou $-SO_3^-Y^+$.

**6.** Composé selon la revendication 1 de formule (4).

**7.** Composé selon la revendication 6 dans lequel $R_4$ représente un alkyle en $C_1$-$C_4$ ou un alcanoyle en $C_2$-$C_5$ substitué par un groupe $-COO^-Y^+$, $-N(R_6)(R_6')(R_6'')^+X^-$ ou $-SO_3^-Y^+$.

**8.** Composé selon la revendication 1 de formule

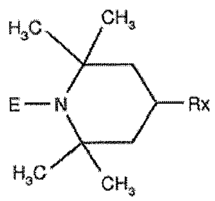

où $R_x$ est choisi dans le groupe constitué par $-NH_2^+CH_2CH_2OH\ Cl^-$, $-NH_3^+\ {}^-OAc$, $-NHCH(CH_3)COO^-K^+$,$-NHCH_2CH_2NH(CH_3)_2^+\ {}^-OAC$, $-NHCH_2CH_2SO_3^-K^+$, $-NHCH(COO^-K^+)CH_2CH_2SCH_3$, $-NHCH_2COO^-K^+$, $-OCH(CH_3)COO^-K^+$, $-OCH_2CH_2NH(CH_3)_2^+\ {}^-OAc$, $-OCH_2CH_2SO_3^-K^+$, $-OCH(COO^-K^+)CH_2CH_2SCH_3$, et $-OCH_2COO^-K^+$.

**9.** Composé selon la revendication 1 de formule (1), (2), (3) ou (5) dans lesquelles

$R_1$ représente un hydrogène, un alkyle en $C_1$-$C_4$ substitué par un groupe hydroxyle ou -$COOR_6$ ; ou bien $R_1$ représente un alkyle en $C_2$-$C_4$ interrompu par un groupe -N($R_6$)- ; ou bien $R_1$ représente un alkyle en $C_2$-$C_{12}$ interrompu par un à six groupes -N($R_6$)- ;
$R_1$' représente un hydrogène ;
$R_2$ représente un alkyle en $C_1$-$C_4$ substitué par un groupe -$COO^-Y^+$, -N($R_6$)($R_6$')($R_6$")$^+X^-$ ou -$SO_3^-Y^+$ ; ou bien $R_2$ représente un alkyle en $C_2$-$C_{12}$ interrompu par un à six soufres, substitué en outre par un à trois groupes -$COO^-Y^+$ ; ou bien $R_2$ représente un phényle substitué par un à deux groupes -$SO_3^-Y^+$ ; ou bien $R_2$ représente un phényle substitué par un ou deux groupes -$COO^-Y^+$ ;
$R_2$' représente un hydrogène ;
$R_3$ représente -$SO_3^-Y^+$ ou un alcanoyle en $C_2$-$C_5$ substitué par un groupe -$COO^-Y^+$ ;
$R_6$' représente un alkyle en $C_2$-$C_6$ ;
$R_7$ représente -N($R_2$)($R_2$') ou

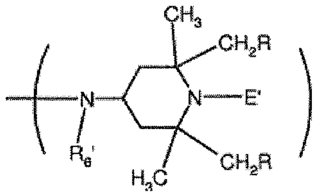

;

et
$R_8$ est défini comme $R_7$, l'un de $R_7$ et $R_8$ représentant -N($R_2$)($R_2$').

**10.** Composition stabilisée comprenant
un matériau organique soumis aux effets nocifs de la lumière, de la chaleur et de l'oxygène, et
un composé d'alcoxyamine hydroxysubstitué stériquement encombré hydrocompatible ou hydrosoluble selon la revendication 1.

**11.** Composition selon la revendication 10 qui est un revêtement, une encre pour jet d'encre, un matériau d'enregistrement par jet d'encre, un matériau d'enregistrement photographique, une structure polymère multicouche, un film coextrudé, un film, une encre ou un revêtement durci par rayonnement, un adhésif ou un stratifié.

**12.** Composition selon la revendication 10 qui comprend en outre au moins un co-additif stabilisant choisi dans le groupe constitué par les antioxydants phénoliques, les stéarates métalliques, les oxydes métalliques, les composés organophosphorés, les antioxydants à base de benzofuranone, les hydroxylamines, les absorbeurs d'ultraviolets, et les autres photostabilisants à base d'amine stériquement encombrée.

**13.** Composition selon la revendication 10 qui comprend en outre un absorbeur d'ultraviolets choisi dans le groupe constitué par les benzophénones, les 2H-benzotriazoles, et les aryl-s-triazines.

**14.** Composition selon la revendication 10 qui est une composition colorée contenant des pigments ou des colorants.

**15.** Procédé de stabilisation d'un matériau organique soumis à une dégradation oxydative, thermique et/ou actinique qui comprend l'addition à ou l'incorporation dans ledit matériau d'au moins un composé selon la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5004770 A
- US 5096950 A
- US 6271377 B
- US 6392041 B
- US 6376584 B
- US 6254724 B
- US 6465645 B
- US 5286865 A
- US 5216156 A
- US 20020050226 A
- EP 1174476 A
- US 6102997 A
- JP 2000044851 B
- JP 99170686 B
- US 3004896 A
- US 3055896 A
- US 3072585 A
- US 3074910 A
- US 3189615 A
- US 3218332 A
- US 3230194 A
- US 4127586 A
- US 4226763 A
- US 4275004 A
- US 4278589 A
- US 4315848 A
- US 4347180 A
- US 4383863 A
- US 4675352 A
- US 4681905 A
- US 4853471 A
- US 5268450 A
- US 5278314 A
- US 5280124 A
- US 5319091 A
- US 5410071 A
- US 5436349 A
- US 5516914 A
- US 5554760 A
- US 5563242 A
- US 5574166 A
- US 5607987 A
- US 5977219 A
- US 6166218 A
- US 5980783 A
- EP 782994 A
- WO 9628431 A
- US 3843371 A
- US 4619956 A
- US 4740542 A
- US 5096489 A
- US 5106891 A
- US 5298067 A
- US 5300414 A
- US 5354794 A
- US 5461151 A
- US 5476937 A
- US 5489503 A
- US 5543518 A
- US 5556973 A
- US 5597854 A
- US 5681955 A
- US 5726309 A
- US 5736597 A
- US 5942626 A
- US 5959008 A
- US 5998116 A
- US 6013704 A
- US 6060543 A
- US 6187919 B
- US 6242598 B
- US 6255483 B
- US 5844029 A
- US 5880191 A
- US 4325863 A
- US 4338244 A
- US 5175312 A
- US 5216052 A
- US 5252643 A
- DE 4316611 A
- DE 4316622 A
- DE 4316876 A
- EP 0589839 A
- EP 0591102 A
- WO 9742261 A
- US 5387458 A
- US 3290203 A
- US 36811793 B
- US 776805 A
- US 4095013 A

**Non-patent literature cited in the description**


- **SAE J.** Test Procedure. Society of Automotive Engineers, 1960
- *Modern Plastics,* May 2000, 90-91